# EUROPEAN PATENT APPLICATION

(11) **EP 4 272 812 A1**
(43) Date of publication of application: **08.11.2023**
(21) Application number: 20926369.8
(22) Date of filing: 30.12.2020
(51) Int. Cl.: A61N 5/10, A61B 5/00, A61B 6/00, A61B 6/12, G16H 30/40, G16H 20/40

(54) **MEDICAL IMAGE ANALYSIS METHOD CONSIDERING FEATURE INFORMATION, MEDICAL IMAGE ANALYSIS DEVICE, AND MEDICAL IMAGE ANALYSIS SYSTEM**

(71) Applicant: Neurophet Inc., Seoul 06247 (KR)
(72) Inventor: KIM, Dong Hyeon, Seoul 05350 (KR); LEE, Min Ho, Goyang-si, Gyeonggi-do 10206 (KR)
(74) Representative: Dantz, Jan Henning
(86) International application number: PCT/KR2020/019416
(87) International publication number: WO 2022/145538

(57) **Abstract**

Provided is a medical image analysis method including: obtaining a target medical image; obtaining treatment plan information for determining a target area to which radiation is to be emitted, the treatment plan information including first feature information or second feature information; selecting a target parameter set from among a first parameter set corresponding to the first feature information and a second parameter set corresponding to the second feature information on the basis of the treatment plan information; determining, as the target parameter set, parameters of a feature node set including at least one of a plurality of nodes of an artificial neural network trained to obtain area information related to the target area on the basis of the target medical image; and providing treatment assistance information related to the target area corresponding to the treatment plan information on the basis of the artificial neural network to which the target parameter set is applied and the target medical image.

## Description

### [Technical Field]

The present application relates to a medical image analysis method, device and system for analyzing a medical image.

### [Background Art]

In the field of tumor treatment, it is necessary to define an area to which radiation is to be emitted before a treatment to completely remove a tumor while protecting organs around the tumor. In this case, an area to which radiation is to be emitted may be defined differently according to a treatment history or style of an operator who will conduct a treatment, an age or health condition of a patient who will have the treatment, and the like.

Methods of defining an area to which radiation is to be emitted to treat a tumor according to the related art include a manual method of manually defining an area to which radiation is to be emitted by an operator who will conduct a treatment and an automatic method of automatically defining an area to which radiation is to be emitted by software.

However, treatment assistance information based on an area, to which radiation is to be emitted, defined by the manual method is calculated with an operator's naked eye and thus reproducibility and accuracy of a treatment are relatively low, whereas in the case of the automatic method, an area to which radiation is to be emitted may be defined differently according to an operator, a patient, a type of tumor, etc. (hereinafter referred to as an operator and the like) and thus cannot be automatically calculated while reflecting characteristics of the operator and the like.

Therefore, it is necessary to conduct research into a medical image analysis device and method for dividing an area to which radiation is to be emitted while reflecting characteristics of an operator and the like and automatically calculating treatment assistance information.

### [Disclosure]

### [Technical Problem]

An aspect of the present disclosure provides a medical image analysis method, device, and system for providing information related to a medical image.

Aspects of the present disclosure are not limited thereto and other aspects which are not mentioned herein will be clearly understood by those of ordinary skill in the art from the present specification and the accompanying drawings.

### [Technical Solution]

According to a method for analyzing a medical image disclosed in the present application, the method comprises: obtaining a target medical image; obtaining a treatment plan information for determining a target area to be radiated, wherein the treatment plan information includes a first feature information or a second feature information; selecting a target parameter set, based on the treatment plan information, among a first parameter set corresponding to the first feature information and a second parameter set corresponding to the second feature information; determining parameter values of a feature node set including at least one of a plurality of nodes of an artificial neural network learned to obtain an area information related to the target area as the target parameter set, based on the target medical image; and providing the treatment auxiliary information related to the target area corresponding to the treatment plan information, based on the artificial neural network to which the target parameter set is applied and the target medical image.

According to a method for analyzing a medical image disclosed in the present application, the method comprises: obtaining a target medical image; obtaining a treatment plan information including a first feature information and second feature information related to parameters which are a basis for determining a target area to be irradiated; obtaining a first area related to a target tumor and a second area adjacent to the first area and related to the target area, by performing a segmentation the target medical image into a plurality of areas bas based on the treatment plan information, using an artificial neural network including a node set having a target parameter set determined based on the treatment plan information; determining a boundary of the second area based on the target parameter set of the node set, wherein when the treatment plan information includes the first feature information, the second area has a first boundary, and when the treatment plan information includes the second feature information, the second area has a second boundary different from the first boundary; and providing the determined boundary of the second area and a boundary of the first area on the medical image.

According to a device for analyzing a medical image disclosed in the present application, the device comprises: an image acquisition unit for obtaining a target medical image; and a controller for providing a treatment auxiliary information based on the target medical image, and wherein the controller configured to: obtain a target medical image; obtain a treatment plan information for determining a target area to be radiated, wherein the treatment plan information includes a first feature information or a second feature information; select a target parameter set, based on the treatment plan information, among a first parameter set corresponding to the first feature information and a second parameter set corresponding to the second feature information; determine parameter values of a feature node set including at least one of a plurality of nodes of an artificial neural network learned to obtain an area information related to the target area as the target parameter set, based on the target medical image; and provide the treatment auxiliary information related to the target area corresponding to the treatment plan information, based on the artificial neural network to which the target parameter set is applied and the target medical image.

According to a device for analyzing a medical image disclosed in the present application, the device comprises: an image acquisition unit for obtaining a target medical image; and a controller for providing a treatment auxiliary information based on the target medical image, and wherein the controller configured to: obtain a target medical image; obtain a treatment plan information including a first feature information and second feature information related to parameters which are a basis for determining a target area to be irradiated; obtain a first area related to a target tumor and a second area adjacent to the first area and related to the target area, by performing a segmentation the target medical image into a plurality of areas bas based on the treatment plan information, using an artificial neural network including a node set having a target parameter set determined based on the treatment plan information; determine a boundary of the second area based on the target parameter set of the node set, wherein when the treatment plan information includes the first feature information, the second area has a first boundary, and when the treatment plan information includes the second feature information, the second area has a second boundary different from the first boundary; and provide the determined boundary of the second area and a boundary of the first area on the medical image;

The objects of the present invention are not limited to the aforementioned object, and other objects which are not described herein should be clearly understood by those skilled in the art from the following description and the accompanying drawings.

### [Advantageous Effects]

According to an embodiment of the present application, a neural network model may be trained on the basis of feature information related to a medical image, and a target medical image may be analyzed on the basis of the trained neural network model to provide treatment assistance information reflecting the feature information.

However, effects of the present disclosure are not limited thereto and other effects which are not mentioned herein will be clearly understood by those of ordinary skill in the art from the present specification and the accompanying drawings.

### [Description of Drawings]

FIG. 1 illustrates an example of treatment assistance information that may be provided by analyzing a medical image according to an embodiment of the present application.
FIG. 2 is a schematic diagram of a medical image analysis system according to an embodiment of the present application.
FIG. 3 is a block diagram of a medical image analysis device according to an embodiment of the present application.
FIG. 4 is a flowchart of a process for segmenting a medical image according to an embodiment of the present application.
FIG. 5 is a flowchart of a method of training a neural network model by a learning device according to an embodiment of the present application.
FIG. 6 is a diagram of an example of a structure of a training data set related to a medical image according to an embodiment of the present application.
FIG. 7 illustrates an example of an artificial neural network model that may be provided to a learning device according to an embodiment of the present application.
FIG. 8 is a flowchart of a method of training an artificial neural network model according to an embodiment of the present application.
FIG. 9 is a schematic diagram illustrating a method of training an artificial neural network model according to an embodiment of the present application.
FIG. 10 is a flowchart of a method of training an artificial neural network model according to an embodiment of the present application.
FIGS. 11 and 12 are diagrams of examples of a structure of a training data set related to a medical image according to an embodiment of the present application.
FIGS. 13 and 14 are schematic diagrams illustrating methods of renewing a parameter set of a feature layer according to an embodiment of the present application.
FIG. 15 is a flowchart of an image segmentation method using a neural network model, which is performed by a medical image analysis device 2000, according to an embodiment of the present application.
FIG. 16 is a diagram illustrating an example of a structure of a target medical image according to an embodiment of the present application.
FIG. 17 is a schematic diagram illustrating segmenting a target medical image by a medical image analysis device (2000) according to an embodiment of the present application.
FIG. 18 is a flowchart of a segmentation method of a target medical image according to an embodiment of the present application.
FIG. 19 illustrates an example of a user interface related to a result of analyzing a target medical image according to an embodiment of the present application.
FIG. 20 is a schematic diagram illustrating segmenting a target medical image according to an embodiment of the present application.
FIG. 21 illustrates an example of a user interface related to a result of analyzing a target medical image analysis according to an embodiment of the present application.
FIGS. 22 to 24 illustrate examples of a user interface related to a result of analyzing a target medical image according to an embodiment of the present application.

### [Best Mode]

A method for analyzing a medical image according to an one embodiment, the method comprises: obtaining a target medical image; obtaining a treatment plan information for determining a target area to be radiated, wherein the treatment plan information includes a first feature information or a second feature information; selecting a target parameter set, based on the treatment plan information, among a first parameter set corresponding to the first feature information and a second parameter set corresponding to the second feature information; determining parameter values of a feature node set including at least one of a plurality of nodes of an artificial neural network learned to obtain an area information related to the target area as the target parameter set, based on the target medical image; and providing the treatment auxiliary information related to the target area corresponding to the treatment plan information, based on the artificial neural network to which the target parameter set is applied and the target medical image.

According to a method for analyzing a medical image according to an one embodiment, wherein the artificial neural network is configured to obtain a plurality of areas including the target area and a tumor area by performing a segmentation to the target medical image, based on one or more labels related to a radiation irradiation.

According to a method for analyzing a medical image according to an one embodiment, wherein one or more labels include a label related to at least one of an area corresponding to an organ in which a tumor is located, an area related to a margin considering a movement of a patient, an area related to a margin considering a movement of organ, an area that should not be irradiated with the radiation, and the tumor area, wherein the artificial neural network is learned to assign the one or more labels to a cell of the target medical image and to obtain an area information related to the target area, an area information related to the area that should not be irradiated with the radiation, and the area information related to the tumor area, wherein the treatment auxiliary information related to the target area is obtained based on the label assigned to the cell.

According to a method for analyzing a medical image according to an one embodiment, wherein when the treatment plan information includes the first feature information, the target parameter set is determined as the first parameter set, and the providing the treatment auxiliary information comprises: obtaining a first target area information obtained based on area information obtained via the artificial neural network which is applied the first parameter set, and wherein when the treatment plan information includes the second feature information, the target parameter set is determined as the second parameter set, and the providing the treatment auxiliary information comprises: obtaining a second target area information obtained based on area information obtained via the artificial neural network which is applied the second parameter set, and wherein the second target area information is different from the first target area information.

According to a method for analyzing a medical image according to an one embodiment, wherein the first target area information is defined by a first boundary and the second target area information is defined by a second boundary, wherein at least one boundary of the first boundary and the second boundary on the target medical image includes another boundary of the first boundary and the second boundary.

According to a method for analyzing a medical image according to an one embodiment, wherein the target parameter set is used to obtain the area information related to the target area, wherein when the treatment plan information includes the first feature information, the target parameter set is determined as the first parameter set, and the providing the treatment auxiliary information comprises: obtaining a third target area information obtained based on a tumor area information obtained via the artificial neural network which is applied the first parameter set, and wherein when the treatment plan information includes the second feature information, the target parameter set is determined as the second parameter set, and the providing the treatment auxiliary information comprises: obtaining a fourth target area information obtained based on the tumor area information obtained via the artificial neural network which is applied the second parameter set, and wherein the third target area information is substantially the same as the fourth target area information.

According to a method for analyzing a medical image according to an one embodiment, the obtaining the treatment plan information comprises: obtaining a user input selecting at least one of the first feature information or the second feature information, via an input module, the selecting the target parameter set comprises: selecting a parameter set corresponding to the user input among the first parameter set corresponding to the first feature information and the second parameter set corresponding to the second feature information as the target parameter set.

According to a method for analyzing a medical image according to an one embodiment, the method further comprises: providing a second treatment auxiliary information which is related to the target area, obtained based on the target medical image and the artificial neural network which does not include the target parameter set, wherein the second treatment auxiliary information is obtained by the artificial neural network independent of the first feature information or the second feature information.

According to a method for analyzing a medical image according to an one embodiment, the method further comprises: obtaining an user input, via an input module, which selects at least one of a first target area information and a second target area information and instructs to initiate an irradiation of radiation based on the selected target area information; and instructing an initiation of the irradiation of radiation in response to the user input; wherein the first target area information is an information related to the target area obtained based on area information obtained via the artificial neural network applied the first parameter set, and wherein the second target area information is an information related to the target area obtained based on area information obtained via the artificial neural network applied the second parameter set.

A method for analyzing a medical image according to an one embodiment, the method comprises: obtaining a target medical image; obtaining a treatment plan information including a first feature information and second feature information related to parameters which are a basis for determining a target area to be irradiated; obtaining a first area related to a target tumor and a second area adjacent to the first area and related to the target area, by performing a segmentation the target medical image into a plurality of areas bas based on the treatment plan information, using an artificial neural network including a node set having a target parameter set determined based on the treatment plan information; determining a boundary of the second area based on the target parameter set of the node set, wherein when the treatment plan information includes the first feature information, the second area has a first boundary, and when the treatment plan information includes the second feature information, the second area has a second boundary different from the first boundary; and providing the determined boundary of the second area and a boundary of the first area on the medical image.

According to a method for analyzing a medical image according to an one embodiment, the method further comprises: determining the target parameter set based on the treatment plan information, and wherein the target parameter set is determined by selecting at least one among a first parameter set corresponding to the first feature information and a second parameter set corresponding to the second feature information;

According to a method for analyzing a medical image according to an one embodiment, wherein when the treatment plan information includes the first feature information, the second area having the first boundary is determined based on the first parameter set, and when the treatment plan information includes the second feature information, the second area having the second boundary is determined based on the second parameter set.

According to a method for analyzing a medical image according to an one embodiment, wherein when the treatment plan information includes the first feature information, the first area has a third boundary, and when the treatment plan information includes the second feature information, the first area has a fourth boundary, wherein the third boundary and the fourth boundary are substantially the same.

According to a method for analyzing a medical image according to an one embodiment, the obtaining the treatment plan information comprises: obtaining a, user input selecting at least one of the first feature information or the second feature information, via an input module, the selecting the target parameter set comprises: based on the user input, selecting a parameter set corresponding to the user input among the first parameter set corresponding to the first feature information and the second parameter set corresponding to the second feature information as the target parameter set.

According to a method for analyzing a medical image according to an one embodiment, wherein the treatment plan information is related to at least one of an operator information, a patient information, a tumor information and a radiation information, wherein the operator information includes at least one of an identity information and a treatment history information related to the operator who treats a tumor, wherein the tumor information includes at least one of information related to a size, type, and expression lever of the tumor to be treated, wherein the radiation information includes at least one of information related to a type, an intensity, a shape, and a risk of the radiation.

According to a method for analyzing a medical image according to an one embodiment, the method further comprises: obtaining an user input, via an input module, related to an user treatment information defining a plurality of areas including a third area related to the tumor area and a fourth area related to the target area to the target medical image; and outputting the target medical image on which a boundary of the third area and a boundary of the fourth area are displayed.

According to a method for analyzing a medical image according to an one embodiment, providing an auxiliary information which is related to the target area, obtained based on the target medical image and the artificial neural network which does not include the target parameter set, wherein the auxiliary information is obtained by the artificial neural network independent of the first feature information or the second feature information.

According to a method for analyzing a medical image according to an one embodiment, the method further comprises: obtaining an user input, via an input module, which instructs to initiate an irradiation of radiation based on the second area; and instructing an initiation of the irradiation of radiation for the second area in response to the user input.

According to a method for analyzing a medical image according to an one embodiment, wherein the plurality of areas is related to at least one of an area corresponding to an organ in which a tumor is located, an area related to a margin considering a movement of a patient, an area related to a margin considering a movement of organ, an area that should not be irradiated with the radiation, and the tumor area.

According to a method for analyzing a medical image according to an one embodiment, wherein the artificial neural network is configured to obtain the plurality of areas by segmentation for the target medical image based on the one or more labels related to an irradiation.

According to a method for analyzing a medical image according to an one embodiment, wherein the one or more labels include a label related to at least one of an area corresponding to an organ in which a tumor is located, an area related to a margin considering a movement of a patient, an area related to a margin considering a movement of organ, an area that should not be irradiated with the radiation, and the tumor area, wherein the artificial neural network is learned to assign the one or more labels to a cell of the target medical image and to obtain an area information related to the target area, an area information related to the area that should not be irradiated with the radiation, and the area information related to the tumor area, wherein the plurality of areas are obtained based on the label assigned to the cell.

A device for analyzing a medical image according to an one embodiment, the device comprises: an image acquisition unit for obtaining a target medical image; and a controller for providing a treatment auxiliary information based on the target medical image, and wherein the controller configured to: obtain a target medical image; obtain a treatment plan information for determining a target area to be radiated, wherein the treatment plan information includes a first feature information or a second feature information; select a target parameter set, based on the treatment plan information, among a first parameter set corresponding to the first feature information and a second parameter set corresponding to the second feature information; determine parameter values of a feature node set including at least one of a plurality of nodes of an artificial neural network learned to obtain an area information related to the target area as the target parameter set, based on the target medical image; and provide the treatment auxiliary information related to the target area corresponding to the treatment plan information, based on the artificial neural network to which the target parameter set is applied and the target medical image.

According to a device for analyzing a medical image according to an one embodiment, wherein the artificial neural network is configured to obtain a plurality of areas including the target area and a tumor area by performing a segmentation to the target medical image, based on one or more labels related to a radiation irradiation.

According to a device for analyzing a medical image according to an one embodiment, wherein one or more labels include a label related to at least one of an area corresponding to an organ in which a tumor is located, an area related to a margin considering a movement of a patient, an area related to a margin considering a movement of organ, an area that should not be irradiated with the radiation, and the tumor area, wherein the artificial neural network is learned to assign the one or more labels to a cell of the target medical image and to obtain an area information related to the target area, an area information related to the area that should not be irradiated with the radiation, and the area information related to the tumor area, wherein the treatment auxiliary information related to the target area is obtained based on the label assigned to the cell.

According to a device for analyzing a medical image according to an one embodiment, the controller is configured to: determine the target parameter set as the first parameter set when the treatment plan information includes the first feature information, and obtain a first target area information obtained based on area information obtained via the artificial neural network which is applied the first parameter set, determine the target parameter set as the second parameter set when the treatment plan information includes the second feature information, and obtain a second target area information obtained based on area information obtained via the artificial neural network which is applied the second parameter set, wherein the second target area information is different from the first target area information.

According to a device for analyzing a medical image according to an one embodiment, wherein the first target area information is defined by a first boundary and the second target area information is defined by a second boundary, wherein at least one boundary of the first boundary and the second boundary on the target medical image includes another boundary of the first boundary and the second boundary.

According to a device for analyzing a medical image according to an one embodiment, wherein the target parameter set is used to obtain the area information related to the target area, and the controller is configured to: determine the target parameter set as the first parameter set when the treatment plan information includes the first feature information, and provide the treatment auxiliary information by providing a third target area information obtained based on a tumor area information obtained via the artificial neural network which is applied the first parameter set, determine the target parameter set as the second parameter set when the treatment plan information includes the second feature information, and provide the treatment auxiliary information by providing a fourth target area information obtained based on the tumor area information obtained via the artificial neural network which is applied the second parameter set, and wherein the third target area information is substantially the same as the fourth target area information.

According to a device for analyzing a medical image according to an one embodiment, the device further comprises: an input module for receiving an user input related to the treatment plan information; and an output module for outputting the treatment auxiliary information in response to the user input; the controller is configured to: obtain a user input selecting at least one of the first feature information or the second feature information, via an input module; and select a parameter set corresponding to the user input among the first parameter set corresponding to the first feature information and the second parameter set corresponding to the second feature information as the target parameter set.

According to a device for analyzing a medical image according to an one embodiment, wherein the treatment plan information is related to at least one of an operator information, a patient information, a tumor information and a radiation information, wherein the operator information includes at least one of an identity information and a treatment history information related to the operator who treats a tumor, wherein the tumor information includes at least one of information related to a size, type, location and expression lever of the tumor to be treated, wherein the radiation information includes at least one of information related to a type, an intensity, an irradiation period, and a risk of the radiation.

According to a device for analyzing a medical image according to an one embodiment, the device further comprises: an input module for receiving an user input defining a plurality of areas; and an output module for outputting an user treatment information in response to the user input; the controller is configured to: obtain the user input, via an input module, related to the user treatment information defining the plurality of areas including a tumor area information related to a tumor area and a target area information related to a target area to the target medical image; and output the user treatment information and the treatment auxiliary information via output module.

According to a device for analyzing a medical image according to an one embodiment, the controller is configured to: provide a second treatment auxiliary information which is related to the target area, obtained based on the target medical image and the artificial neural network which does not include the target parameter set, wherein the second treatment auxiliary information is obtained by the artificial neural network independent of the first feature information or the second feature information.

According to a device for analyzing a medical image according to an one embodiment, the device further comprises: an input module for receiving an user input instructing an initiation of irradiation; wherein the controller is configured to: obtain an user input, via an input module, which selects at least one of a first target area information and a second target area information and instructs to initiate an irradiation of radiation based on the selected target area information; and instruct an initiation of the irradiation of radiation in response to the user input; wherein the first target area information is an information related to the target area obtained based on area information obtained via the artificial neural network applied the first parameter set, and wherein the second target area information is an information related to the target area obtained based on area information obtained via the artificial neural network applied the second parameter set.

A device for analyzing a medical image according to an one embodiment, the device comprises: an image acquisition unit for obtaining a target medical image; and a controller for providing a treatment auxiliary information based on the target medical image, and wherein the controller configured to: obtain a target medical image; obtain a treatment plan information including a first feature information and second feature information related to parameters which are a basis for determining a target area to be irradiated; obtain a first area related to a target tumor and a second area adjacent to the first area and related to the target area, by performing a segmentation the target medical image into a plurality of areas bas based on the treatment plan information, using an artificial neural network including a node set having a target parameter set determined based on the treatment plan information; determine a boundary of the second area based on the target parameter set of the node set, wherein when the treatment plan information includes the first feature information, the second area has a first boundary, and when the treatment plan information includes the second feature information, the second area has a second boundary different from the first boundary; and provide the determined boundary of the second area and a boundary of the first area on the medical image.

According to a device for analyzing a medical image according to an one embodiment, the controller is configured to: determine the target parameter set based on the treatment plan information, and determine the target parameter set by selecting at least one among a first parameter set corresponding to the first feature information and a second parameter set corresponding to the second feature information.

According to a device for analyzing a medical image according to an one embodiment, the controller is configured to: determine the second area having the first boundary based on the first parameter set when the treatment plan information includes the first feature information; and determine the second area having the second boundary based on the second parameter set when the treatment plan information includes the second feature information.

According to a device for analyzing a medical image according to an one embodiment, wherein when the treatment plan information includes the first feature information, the first area has a third boundary, and when the treatment plan information includes the second feature information, the first area has a fourth boundary, wherein the third boundary and the fourth boundary are substantially the same.

According to a device for analyzing a medical image according to an one embodiment, the device further comprises: an input module for receiving an user input related to the treatment plan information; and wherein the controller is configured to: obtain the user input selecting at least one of the first feature information or the second feature information, via the input module; and based on the user input, select a parameter set corresponding to the user input among the first parameter set corresponding to the first feature information and the second parameter set corresponding to the second feature information as the target parameter set.

According to a device for analyzing a medical image according to an one embodiment, wherein the treatment plan information is related to at least one of an operator information, a patient information, a tumor information and a radiation information, wherein the operator information includes at least one of an identity information and a treatment history information related to the operator who treats a tumor, wherein the tumor information includes at least one of information related to a size, type, and expression lever of the tumor to be treated, wherein the radiation information includes at least one of information related to a type, an intensity, a shape, and a risk of the radiation.

According to a device for analyzing a medical image according to an one embodiment, the device further comprises: an input module for receiving an user input related to the treatment plan information; and an output module for outputting a treatment auxiliary information in response to the user input; wherein the controller is configured to: obtain the user input, via the input module, related to an user treatment information defining a plurality of areas including a third area related to the tumor area and a fourth area related to the target area to the target medical image; and output the target medical image on which a boundary of the third area and a boundary of the fourth area are displayed.

According to a device for analyzing a medical image according to an one embodiment, wherein the controller is configured to: provide an auxiliary information which is related to the target area, obtained based on the target medical image and the artificial neural network which does not include the target parameter set, wherein the auxiliary information is obtained by the artificial neural network independent of the first feature information or the second feature information.

According to a device for analyzing a medical image according to an one embodiment, the device further comprises: an input module for receiving an user input instructing an initiation of irradiation; wherein the controller is configured to: obtain the user input, via the input module, which instructs to initiate the irradiation based on the second area; and instruct the initiation of the irradiation for the second area in response to the user input;

According to a device for analyzing a medical image according to an one embodiment, wherein the plurality of areas is related to at least one of an area corresponding to an organ in which a tumor is located, an area related to a margin considering a movement of a patient, an area related to a margin considering a movement of organ, an area that should not be irradiated with the radiation, and the tumor area.

According to a device for analyzing a medical image according to an one embodiment, wherein the artificial neural network is configured to obtain the plurality of areas by segmentation for the target medical image based on the one or more labels related to an irradiation.

According to a device for analyzing a medical image according to an one embodiment, wherein the one or more labels include a label related to at least one of an area corresponding to an organ in which a tumor is located, an area related to a margin considering a movement of a patient, an area related to a margin considering a movement of organ, an area that should not be irradiated with the radiation, and the tumor area, wherein the artificial neural network is learned to assign the one or more labels to a cell of the target medical image and to obtain an area information related to the target area, an area information related to the area that should not be irradiated with the radiation, and the area information related to the tumor area, wherein the plurality of areas are obtained based on the label assigned to the cell.

### [Modes of the Invention]

The above-described aspects, features and advantages of the present application will be more apparent from the following detailed description with reference to the accompanying drawings. However, various changes may be made in the present application, and various embodiments may be implemented, and thus example embodiments are illustrated in the drawings and described herein.

Throughout the specification, generally, the same reference numerals represent the same elements. In the drawings of embodiments, elements that have the same function and are within the scope of the same idea are described using the same reference numeral and a redundant description thereof will be omitted.

In the following description, well-known functions or constructions related to the present application are not described in detail if it is determined that they would obscure the present disclosure due to unnecessary detail. Numerals (e.g., first, second, etc.) used to describe the present specification are merely identification symbols for distinguishing one element from other elements.

Terms "module" and "unit" used to describe elements of the following embodiments are only intended or interchangeably used to facilitate the making of the specification and should not be understood as having different meanings or functions.

In the following embodiments, the singular expressions are intended to include plural forms as well, unless the context clearly dictates otherwise.

In the following embodiments, terms such as "include" or "have" mean that the features or components described in the specification are present and do not preclude the possibility that one or more other features or components may be added.

In the drawings, elements may be exaggerated or reduced in size for convenience of description. For example, the size and thickness of each element illustrated in the drawings are arbitrarily illustrated for convenience of description and thus the present disclosure is not necessarily limited thereto.

When an embodiment may be implemented differently, an order of a certain process may be performed differently from an order described herein. For example, two processes described in succession may be performed substantially simultaneously or performed in an order reverse to an order described herein.

In the following embodiments, when elements are referred to as being connected to each other, the elements may be understood as being directly connected to each other or indirectly connected to each other while other elements are interposed therebetween.

For example, when elements are referred herein to as being electrically connected to each other, the elements may be directly electrically connected to each other or indirectly electrically connected to each other while other elements are interposed therebetween.

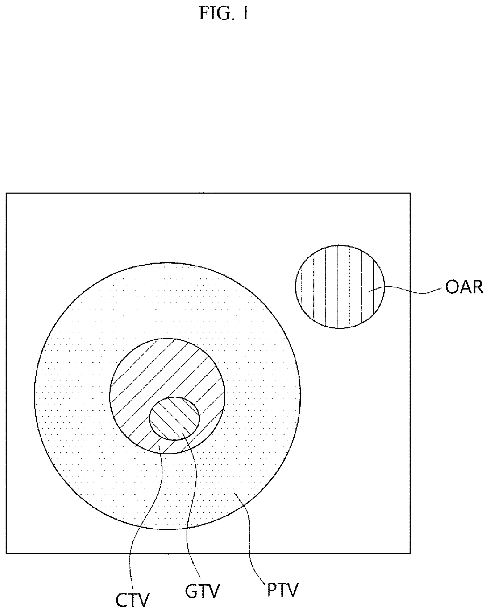

Referring to FIG. 1, in relation to an area to which radiation is to be emitted, treatment assistance information may include information about an area GTV corresponding to an organ in which tumor is located, an area CTV related to a margin when a movement of a patient who will have a radiation treatment is taken into consideration, and a margin PTV in consideration of a fine movement of the organ when tumor treatment is performed a plurality of times. The treatment assistance information may further include information about an area OAR to which radiation should not be emitted, e.g., an area in which organs around the tumor are located.

In this case, the area to which radiation is to be emitted and the area to which radiation should not be emitted may be defined differently according to an operator who will conduct the treatment. The area to which radiation is to be emitted and the area to which radiation should not be emitted may be defined differently according to characteristics of the patient, the type of the tumor, and/or the type of radiation to be used for the tumor treatment.

In a medical image analysis device, a medical image analysis system, and a medical image analysis method according to the present application, an area to which radiation is to be emitted and/or an area to which radiation should not be emitted may be automatically calculated using a technique for segmenting a medical image and treatment assistance information related to the area to which radiation is to be emitted and/or the area to which radiation should not be emitted may be calculated to reflect characteristics of an operator, a patient who will have radiation treatment, and tumor and/or radiation and provided to a user.

The calculating of the treatment assistance information related to tumor treatment will be described below. However, the calculating of the treatment assistance information is only an example and is applicable in all medical fields of generating treatment assistance information to reflect characteristics of an operator and the like on the basis of a medical image analysis, as well as a tumor.

A medical image analysis method, a medical image device, and a medical image analysis system according to an embodiment of the present application will be described below.

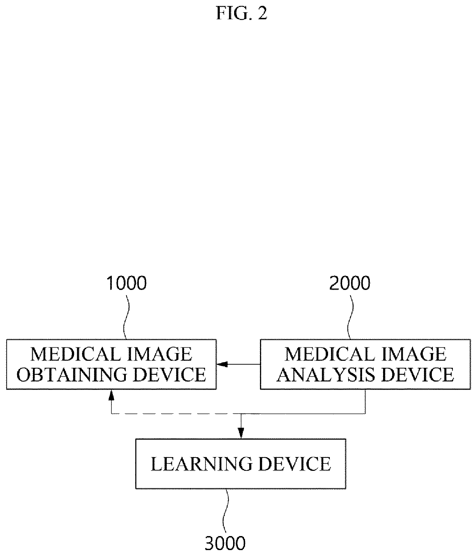

FIG. 2 is a schematic diagram of a medical image analysis system according to an embodiment of the present application. Referring to FIG. 2, the medical image analysis system according to an embodiment of the present application may include a medical image obtaining device 1000, a medical image analysis device 2000, and a learning device 3000.

The medical image obtaining device 1000 may obtain a medical image and transmit the medical image to the medical image analysis device 2000 through a network.

For example, the medical image obtaining device 1000 may be a device for obtaining a magnetic resonance imaging (MRI) image. In this case, the MRI image obtained by the medical image obtaining device 1000 may be transmitted to the medical image analysis device 2000 through a network.

As another example, the medical image obtaining device 1000 may be a device for obtaining a computed tomography (CT) image. In this case, the CT image obtained by the medical image obtaining device 1000 may be transmitted to the medical image analysis device 2000 through the network.

As another example, the medical image obtaining device 1000 may be a device for obtaining an image obtained by radiography. In this case, the image obtained by radiography and the medical image obtaining device 1000 may be transmitted to the medical image analysis device 2000 through the network.

Alternatively, the medical image obtaining device 1000 may be configured with a plurality of medical image obtaining devices 1000

However, the medical image obtaining device 1000 described above is only an example, and thus, the present disclosure is not limited thereto and the medical image obtaining device 1000 should be understood to include any suitable devices or systems used for medical imaging.

An image obtained by the medical image obtaining device 1000 may be a two-dimensional (2D) image. In this case, the medical image may include pixel information associated with coordinates, colors, intensities, etc. of pixels.

The medical image obtained by the medical image obtaining device 1000 may be a three-dimensional (3D) image. In this case, the medical image may include pixel information associated with coordinates, colors, intensities, etc. of voxels.

The medical image obtained by the medical image obtaining device 1000 may include feature information related to the medical image. For example, the medical image may include information about a target, i.e., a patient who will have treatment. In detail, the information about the patient may be understood to mean identification information (e.g., age, gender, name, etc.) of the patient or medical information (e.g., an underlying disease, treatment history, etc.) of an operator who will conduct the treatment. In this case, the information about the patient may be structured as metadata of the medical image.

The medical image obtained by the medical image obtaining device 1000 may include information related to alignment of the medical image. For example, the medical image obtaining device 1000 may also obtain data ijk related to an orientation of a captured medical image by taking into consideration a direction RAS of reference coordinate axes of the object. The medical image obtaining device 1000 may obtain the data ijk related to the orientation of the captured medical image by taking into consideration information xyz about coordinate axes of the medical image obtaining device 1000 and the information RAS about the reference coordinate axes of the target 100.

In this case, the data described above may be structured as metadata about the obtained medical image and transmitted to the medical image analysis device 2000 or may be transmitted to the medical image analysis device 2000 separately from the medical image.

The medical image obtained by the medical image obtaining device 1000 may include information related to an anatomical structure of a certain part of the body. In addition, the part of the body may correspond to any part of the body to which medical imaging may apply. For convenience of description, a medical image associated with a tumor will be described in the specification and the drawings to be described below but is only an example, and embodiments set forth herein may apply to all cases in which a medical image is analyzed to treat a disease occurring in any part of the body (e.g., lung, breast, heart, joints, vessels, etc.), as well as a tumor.

The medical image obtaining device 1000 according to an embodiment of the present application may be embodied as a server. In this case, the server may be configured to store medical images and information related thereto. In addition, the server may be configured to modify or process medical images and information related thereto.

In addition, medical images may be stored in a memory of the medical image analysis device 2000 or a server and used to perform segmentation or output treatment assistance information. This will be described in detail below.

The medical image analysis device 2000 according to an embodiment of the present application may segment a medical image, which is obtained from the medical image obtaining device 1000, using an artificial neural network trained by the learning device 3000 and output treatment assistance information. This will be described in detail below.

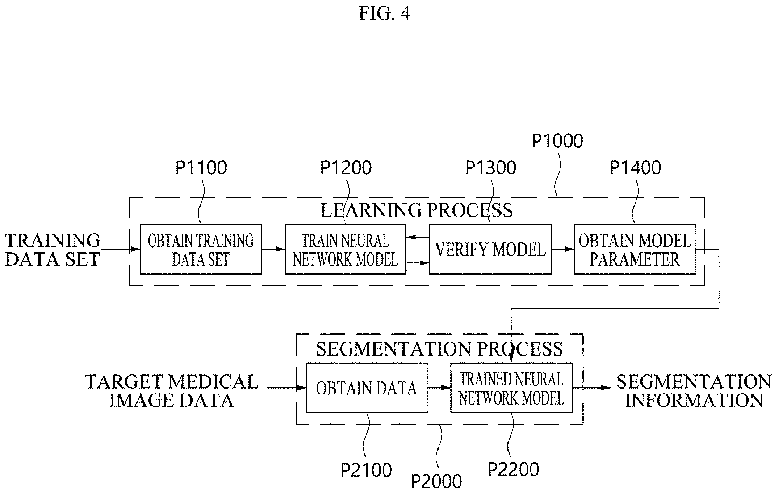

The learning device 3000 according to an embodiment of the present application may renew a parameter set of a neural network, which is for segmentation of a medical image, using a training data set. In addition, the learning device 3000 according to an embodiment of the present application may renew a parameter set of a neural network, for segmentation of a medical image related to feature information, using the training data set. This will be described in detail with reference to FIGS. 4 to 14 below.

FIG. 2 illustrates that the medical image obtaining device 1000, the medical image analysis device 2000, and the learning device 3000 are provided as separate devices. However, this is only an example, and the medical image obtaining device 1000, the medical image analysis device 2000, and the learning device 3000 may be embodied together as one device. Alternatively, some of the medical image obtaining device 1000, the medical image analysis device 2000, and the learning device 3000 may be provided as separate devices and the other may be embodied as one device.

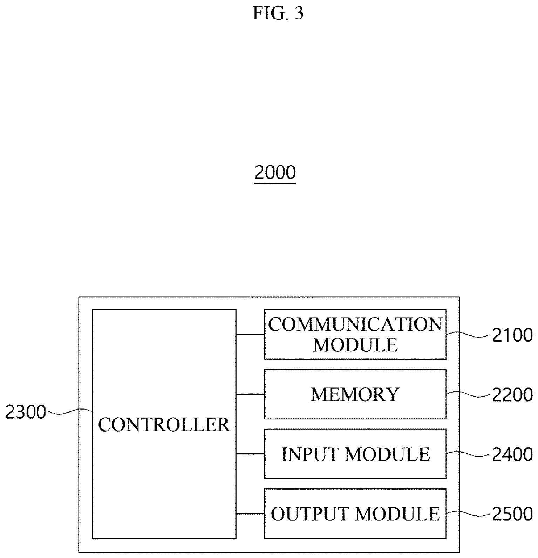

A configuration of the medical image analysis device 2000 according to an embodiment of the present application will be described with reference to FIG. 3 below. FIG. 3 is a block diagram of a medical image analysis device 2000 according to an embodiment of the present application.

The medical image analysis device 2000 according to an embodiment of the present application may include a communication module 2100, a memory 2200, and a controller 2300.

The communication module 2100 may communicate with the medical image obtaining device 1000, the learning device 3000, and an external device. In other words, the medical image analysis device 2000 may transmit a medical image to or receive a medical image from the medical image obtaining device 1000 or transmit data to or receive data from external devices, including a repeater, a server, etc., and the learning device 3000 through the communication module 2100.

For example, the medical image analysis device 2000 may receive a medical image from the medical image obtaining device 1000 and parameter information regarding a trained neural network from the learning device 3000 through the communication module 2100. As another example, the medical image analysis device 2000 may transmit information related to an analysis result to an arbitrary external device via the communication module 2100. As another example, the medical image analysis device 2000 may be connected to the Internet via the communication module 2100 to upload various pieces of data related to the medical image and information related to the analysis result.

The communication module 2100 is largely divided into a wired type and a wireless type. Because the wired type and the wireless type have merits and demerits, both a wired type communication module and a wireless type communication module may be provided for the medical image analysis device 2000.

Here, a representative example of the wired type communication module includes a local area network (LAN) or universal serial bus (USB) communication, or other methods may be used.

Here, the wireless type communication module may generally include a communication method based on a wireless personal area network (WPAN) such as Bluetooth or ZigBee. However, wireless communication protocol is not limited thereto, and the wireless type communication module may also use a communication method based on wireless local area network (WLAN) such as Wi-Fi or other known communication methods.

The memory 2200 may store various pieces of information. The memory 2200 may store various pieces of data temporarily or semi-permanently. Examples of the memory 2200 may include a hard disk drive (HDD), a solid-state drive (SSD), a flash memory, a read-only memory (ROM), a random access memory (RAM), etc.

The memory 2200 may be embedded in or detachably mounted in the medical image analysis device 2000. The memory 2200 may store various pieces of data necessary to operate the medical image analysis device 2000, including an operating system (OS) for driving the medical image analysis device 2000 or a program for operating the components of the medical image analysis device 2000. For example, the memory 2200 may store various pieces of data related to the medical image and the information related to the analysis result.

The controller 2300 may control overall operations of the medical image analysis device 2000. For example, the controller 2300 may load a program for operating the medical image analysis device 2000 from the memory 2200 and execute the program.

The controller 2300 may be embodied as a central processing unit (CPU) or a device similar thereto using hardware, software, or a combination thereof. The controller 2300 may be provided as an electronic circuit, which processes an electrical signal to perform a control function, in a hardware manner or may be provided as a program or code, which drives a hardware circuit, in a software manner.

Referring to FIG. 3, the medical image analysis device 2000 according to an embodiment of the present application may include an input module 2400 and an output module 2500.

In this case, the medical image analysis device 2000 may use the input module 2400 and the output module 2500 to obtain a user input and output information corresponding to the user input. For example, the medical image analysis device 2000 may use the input module 2400 to obtain a user input to request for obtaining data, a user input to request for preprocessing of the medical image, a user input related to image segmentation, and a user input regarding feature information for determining a parameter set of some nodes, and use the output module 2500 to output corresponding information.

For example, a user may input a condition or setting related to an analysis of the medical image analysis device 2000 through the input module 2400.

For example, a user may set target feature information, for segmenting a target medical image, through the input module 2400. In this case, the medical image analysis device 2000 may be implemented to segment the medical image on the basis of the target feature information received from the input module 2400.

The input module 2400 may be embodied in various forms such as a mouse, a keyboard, a touchpad, etc.

The output module 2500 may be provided to output a notification, a result of analyzing a medical image, etc. during a medical image analysis operation of the medical image analysis device 2000.

For example, when the medical image analysis device 2000 performs segmentation of a medical image, a notification window indicating an error in the medical image may be provided through the output module 2500.

As another example, when the medical image analysis device 2000 performs segmentation of the medical image, a segmentation result may be provided through the output module 2500.

As another example, when the medical image analysis device 2000 completes analyzing a metal image, a result of analyzing the medical image (e.g., segmentation information, treatment assistance information, or the like) may be provided to a user through the output module 2500.

The output module 2050 may be implemented in any suitable form, such as a display of a smartphone or a display of a monitor.

In addition, the medical image analysis device 2000 according to an embodiment of the present application may further include a user interface for obtaining a user input through the input module 2400 and outputting information corresponding to the user input through the output module 2500.

Although FIG. 3 illustrates that the medical image analysis device 2000 according to an embodiment of the present application includes the input module 2400 and the output module 2500, the medical image analysis device 2000 is only an example and may be provided without the input module 2400 and the output module 2500.

In this case, an external device separated from the medical image analysis device 2000 may include an input module and an output module as described above. Analysis results obtained by the medical image analysis device 2000 may be transmitted to a separate external device through a communication module and may be provided to a user through the input module and the output module of the separate external device.

The medical image analysis device 2000 according to an embodiment of the present application may be embodied as a server. In this case, the server may be configured to store a medical image and information related to the medical image, which are transmitted from the medical image obtaining device 1000. In addition, the server may be configured to modify or process the medical image and the information related to the medical image.

The server of the medical image analysis device 2000 may be implemented separately from the server of the medical image obtaining device 1000 but embodiments are not limited thereto and the server of the medical image obtaining device 1000 and the medical image analysis device 2000 may be embodied together as one server. In other words, the medical image obtaining device 1000 and the medical image analysis device 2000 may be configured to include a common server.

The medical image analysis device 2000 according to an embodiment of the present application may perform segmentation of a medical image. In this case, segmentation of a medical image according to an embodiment of the present application may be performed using a trained neural network model.

The medical image analysis device 2000 according to an embodiment of the present application may segment a medical image on the basis of target feature information related to the medical image. In this case, the segmentation of the medical image according to an embodiment of the present application may be performed using a neural network model including a parameter set learned based on the target feature information. A neural network model for segmentation of a medical image according to an embodiment of the present application may be configured such that a feature vector reflecting target feature information is output from some layers of the neural network model.

Some operations performed by the medical image analysis device 2000 according to an embodiment will be described in more detail below.

For convenience of description, analyzing a medical image related to a tumor according to an embodiment will be described below. However, embodiments are not limited to a tumor, and various embodiments set forth herein are applicable to all medical fields in which a medical image is analyzed to treat a disease related to any part of the body, as well as a tumor.

The medical image analysis device 2000 according to an embodiment of the present application may obtain a medical image and information related thereto.

Specifically, the medical image analysis device 2000 may obtain a medical image from the medical image obtaining device 1000. More specifically, the medical image analysis device 2000 may obtain a medical image and information related thereto from the medical image obtaining device 1000 through the communication module 2100.

In addition, the medical image analysis device 2000 may obtain information related to the medical image from the medical image obtaining device 1000, according to a user input through the input module 2400 or from any external device (e.g., a server).

Information related to a medical image may be understood to include data contained in the medical image and feature information related to the medical image.

In this case, the data contained in the medical image may be data related to pixels or voxels included in the medical image, data related to orientation of the medical image, or any metadata structured with respect to the medical image.

In particular, information about a subject (i.e., a patient) in a medical image, which will be described below, may be structured as metadata with respect to the medical image.

In this case, the feature information related to the medical image may be information related to at least one of operator information, patient information, tumor information, and radiation information. The feature information may be a basis on which treatment assistance information is calculated. In other words, the feature information may be used to plan treatment assistance information and thus may be referred to as treatment plan information.

Hereinafter, the terms "feature information" and "treatment plan information" will be used interchangeably with each other for convenience of description but embodiments are not limited thereby.

For example, the operator information may include identification information (e.g., age, gender, name, etc.) of an operator who performs treatment on the basis of a medical image.

Alternatively, the operator information may include treatment history information of the operator for setting or defining an area to which radiation is to be emitted with respect to the medical image. For example, a first operator may have defined a first area of a medical image as an area to which radiation is to be emitted and may have performed treatment on the first area. In this case, the feature information related to the medical image may include identification information of the first operator and treatment history information in which the first area of the medical image is selected as an area to which radiation is to be emitted. On the other hand, a second operator may have determined a second area of the medical image as an area to which radiation is to be emitted and may have performed radiation treatment on the second area. In this case, the feature information related to the medical image may include identification information of the second operator and treatment history information in which the second area of the medical image is selected as an area to which radiation is to be emitted.

For example, the patient information may include identification information (e.g., age, gender, name, etc.) of a patient who will have radiation treatment related to a tumor.

Alternatively, the patient information may include medical information (e.g., underlying disease, treatment history, etc.) of the patient. For example, the patient may have an underlying disease or previous treatment history related to radiation treatment. In this case, the feature information related to the medical image may include the identification information of the patient, information as to whether the patient has an underlying disease, information about the underlying disease, and information related to the previous treatment history.

For example, the tumor information may be understood to include information related to the tumor, including the size, shape, grade, type, or position of the tumor, which is related to the medical image. In this case, the information related to the tumor related to the medical image may be feature information. For example, the first patient may have a tumor corresponding to first tumor information (e.g., a first size, a first shape, a first position, etc.). On the other hand, the second patient may have a tumor corresponding to second tumor information (e.g., a second size, a second shape, a second position, etc.). In this case, the first tumor information and the second tumor information may be feature information related to the medical image.

For example, the radiation information may be understood to include information related to radiation, including the type of radiation used to perform a medical procedure on or treat a tumor, the intensity of radiation, a cycle of radiation emission, a spot size of radiation, the manufacturer of a radiation generator, etc. For example, a medical procedure or treatment may be performed on a first tumor by radiation corresponding to first radiation information. On the other hand, a medical procedure or treatment may be performed on a second tumor by radiation corresponding to second radiation information. In this case, the first radiation information and the second radiation information may be feature information related to the medical image.

The learning device 3000 according to an embodiment of the present application may be implemented to obtain feature information as described above and renew a parameter set on the basis of the feature information to output a feature vector specific to the feature information.

In addition, the medical image analysis device 2000 according to an embodiment of the present application may be implemented to segment the medical image to reflect the feature information by replacing a parameter set of a corresponding node of a neural network with the parameter set obtained on the basis of the feature information.

The medical image analysis device 2000 may be implemented to obtain feature information in the form of metadata obtained by structuring the feature information with respect to the medical image. For example, the identification information (e.g., age, gender, name, etc.) and the like of the patient information included in the feature information may be structured as metadata with respect to the medical images to be obtained by the medical image analysis device 2000.

Alternatively, the medical image analysis device 2000 may be implemented to obtain the feature information described above from any external device. For example, the medical image analysis device 2000 may be implemented to obtain the operator information, the tumor information, the radiation information and/or the patient information included in the feature information from an external device, including a server.

Alternatively, the medical image analysis device 2000 may obtain the feature information input by a user through the input module 2400. For example, the user may input identification information or treatment history information of an operator, information related to a tumor, radiation information for treating the tumor, etc. through the input module 2400. In this case, the medical image analysis device 2000 may obtain feature information by receiving user input.

In this case, feature information related to a medical image may be information related to at least one of operator information, patient information, tumor information, and radiation information. The feature information may be a basis on which treatment assistance information is calculated. In other words, the feature information may be used to plan treatment assistance information and thus may be referred to as treatment plan information.

Hereinafter, the terms 'feature information' and 'treatment plan information' will be used interchangeably with each other for convenience of description but embodiments are not limited thereby.

For example, the operator information may include identification information (e.g., age, gender, name, etc.) of an operator who performs treatment on the basis of a medical image.

Alternatively, the operator information may include treatment history information of the operator for setting or defining an area to which radiation is to be emitted with respect to the medical image. For example, a first operator may have defined a first area of a medical image as an area to which radiation is to be emitted and may have performed treatment on the first area. In this case, the feature information related to the medical image may include identification information of the first operator and treatment history information in which the first area of the medical image is selected as an area to which radiation is to be emitted. On the other hand, a second operator may have determined a second area of the medical image as an area to which radiation is to be emitted and may have performed radiation treatment on the second area. In this case, the feature information related to the medical image may include identification information of the second operator and treatment history information in which the second area of the medical image is selected as an area to which radiation is to be emitted.

For example, the patient information may include identification information (e.g., age, gender, name, etc.) of a patient who will have radiation treatment related to a tumor.

Alternatively, the patient information may include medical information (e.g., underlying disease, treatment history, etc.) of the patient. For example, the patient may have an underlying disease or previous treatment history related to radiation treatment. In this case, the feature information related to the medical image may include the identification information of the patient, information as to whether the patient has an underlying disease, information about the underlying disease, and information related to the previous treatment history.

For example, tumor information may be meant to encompass the information related to a tumor associated with a medical image, including the size, shape, expression level of a tumor, type, or location of the tumor, or the like. In this case, the information related to the tumor related to the medical image may be feature information. For example, the first operator may have a tumor corresponding to first tumor information (e.g., a first size, a first shape, a first position, etc.). On the other hand, the second operator may have a tumor corresponding to second tumor information (e.g., a second size, a second shape, a second position, etc.). In this case, the first tumor information and the second tumor information may be feature information related to the medical image.

For example, the radiation information may be understood to include information related to radiation, including the type of radiation used to perform a medical procedure on or treat a tumor, the intensity of radiation, a cycle of radiation emission, a spot size of radiation, the manufacturer of a radiation generator, etc. For example, a medical procedure or treatment may be performed on a first tumor by radiation corresponding to first radiation information. On the other hand, a medical procedure or treatment may be performed on a second tumor by radiation corresponding to second radiation information. In this case, the first radiation information and the second radiation information may be feature information related to the medical image.

The learning device 3000 according to an embodiment of the present application may be implemented to obtain feature information as described above and renew a parameter set on the basis of the feature information to output a feature vector specific to the feature information.

In addition, the medical image analysis device 2000 according to an embodiment of the present application may be implemented to segment the medical image which reflects the feature information by replacing a parameter set of a corresponding node of a neural network with the parameter set obtained on the basis of the feature information.

The medical image analysis device 2000 may be implemented to obtain feature information in the form of metadata obtained by structuring the feature information with respect to the medical image. For example, the identification information (e.g., age, gender, name, etc.) and the like of the patient information included in the feature information may be structured as metadata with respect to the medical images to be obtained by the medical image analysis device 2000.

Alternatively, the medical image analysis device 2000 may be implemented to obtain the feature information described above from any external device. For example, the medical image analysis device 2000 may be implemented to obtain the operator information, the tumor information, the radiation information and/or the patient information included in the feature information from an external device, including a server.

Alternatively, the medical image analysis device 2000 may obtain the feature information input by a user through the input module 2400. For example, the user may input identification information or treatment history information of an operator, information related to a tumor, radiation information for treating the tumor, etc. through the input module 2400. In this case, the medical image analysis device 2000 may obtain feature information by receiving user input.

In addition, the medical image analysis device 2000 according to an embodiment of the present application may obtain information related to an operation related to analyzing a medical image.

Specifically, the medical image analysis device 2000 may obtain, from any external device, information related to a template, related to a medical image, for preprocessing or aligning the medical image.

Data obtained by the medical image analysis device 2000 may be stored in the memory 2200 of the medical image analysis device 2000 or an external device (e.g., a server) outside the medical image analysis device 2000. Alternatively, the data obtained by the medical image analysis device 2000 may be transmitted to the learning device 3000. Alternatively, the data obtained by the medical image analysis device 2000 may be transmitted to an external device (e.g., a server).

The medical image analysis device 2000 according to an embodiment of the present application may preprocess a medical image. For example, the medical image analysis device 2000 may perform preprocessing to improve the accuracy of analyzing a medical image. The medical image analysis device 2000 may be provided to preprocess a medical image so as to derive a more accurate segmentation result before segmenting the medical image.

For example, the medical image analysis device 2000 may be provided to convert a form of a medical image obtained from the medical image obtaining device 1000. Specifically, formats of medical images to be analyzed may be unified to train a neural network model more stably and accurately. More specifically, it is more stable and accurate to perform an analysis using a medical image with the same format as a medical image used to train the neural network model. Therefore, the medical image analysis device 2000 according to an embodiment of the present application may be provided to convert a form of a medical image obtained from the medical image obtaining device 1000.

For example, the medical image analysis device 2000 may be provided to remove noise that may be present in the medical image obtained from the medical image obtaining device 1000 or correct artifacts. For example, a blurring technique and a technique using a median filter may be used to remove noise. The medical image analysis device 2000 may remove noise and correct artifacts to derive a more accurate result of segmenting the medical image and may output treatment assistance information on the basis of the more accurate result of the segmentation, and thus, objective treatment assistance information may be provided to a user.

For example, the medical image analysis device 2000 may be provided to correct the intensity of an image obtained from the medical image obtaining device 1000. By appropriately correcting the intensity of the medical image, noise that may be present in the medical image may be removed and a medical image specialized for an anatomical structure to be analyzed may be obtained.

For example, the medical image analysis device 2000 may be provided to smooth a medical image obtained from the medical image obtaining device 1000. For example, blurring or a technique using a Gaussian filter may be used to smooth the medical image.

For example, the medical image analysis device 2000 may be provided to adjust an aspect ratio of a medical image obtained from the medical image obtaining device 1000 or to cut the medical image. For example, the medical image analysis device 2000 may be implemented to use any appropriate cropping technique to cut the medical image. Alternatively, in order to adjust an aspect ratio of a medical image, the medical image analysis device 2000 may be implemented to use an appropriate image resizing technique, such as on-demand image resizing, Lambda image resizing, a resizing method using a CILanczosScaleTransform filter, or a resizing method using a CI filter.

For example, the medical image analysis device 2000 may be implemented to perform a preprocessing operation corresponding to a preprocessing operation performed on a medical image by the learning device 3000, which will be described below. For example, when the learning device 3000 trains a neural network with a medical image using a first preprocessing technique, the medical image analysis device 2000 may be implemented to preprocess a target medical image using a preprocessing technique corresponding to the first preprocessing technique. Therefore, segmentation of a medical image using a neural network model may be more stably and accurately implemented.

The medical image analysis device 2000 according to an embodiment of the present application may align a medical image.

For example, the medical image analysis device 2000 may be implemented to obtain information related to an orientation of a medical image and align the medical image on the basis of the information related to the orientation of the medical image.

As another example, the medical image analysis device 2000 may be implemented to obtain a template related to a medical image and align the medical image spatially with the template to align the medical image.

However, the method of aligning a medical image is only an example and the medical image analysis device 2000 may be implemented to align a medical image by any appropriate method. For example, the medical image analysis device 2000 may be implemented to align a medical image on the basis of a characteristic area included in the medical image.

The medical image analysis device 2000 according to an embodiment of the present application may perform segmentation of a medical image. In this case, the medical image analysis device 2000 may be implemented to segment the medical image on the basis of feature information related to the medical image.

Operations of the learning device 3000 and the medical image analysis device 2000 to segment a medical image according to the present embodiment will be described in detail with reference to FIGS. 4 to 17 below.

According to an embodiment of the present application, the segmentation of a medical image may be performed using a trained neural network model. However, the segmentation of a medical image according to an embodiment of the present application may be implemented by any appropriate method without using the neural network model.

Training a neural network model to segment a medical image and segmenting the medical image using the trained neural network model will be described below.

FIG. 4 will now be referred to. FIG. 4 is a flowchart of a process for segmenting a medical image according to an embodiment of the present application.

Referring to FIG. 4, a segmentation process of a medical image according to an embodiment of the present application may include a learning process P 1000 of an artificial neural network model for segmentation of a medical image and a segmentation process P2000 of a target medical image using the trained artificial neural network model.

In this case, the learning process P1000 may be implemented by the learning device 3000 according to an embodiment of the present application.

The segmentation process P2000 may be implemented by the medical image analysis device 2000 according to an embodiment of the present application.

In this case, a parameter set of a neural network model obtained by the learning process P1000 implemented by the learning device 3000 may be transmitted to the medical image analysis device 2000 through any appropriate communication module.

In this case, the medical image analysis device 2000 may be implemented to segment a target medical image on the basis of the parameter set of the neural network model obtained by the learning process P1000. In addition, the medical image analysis device 2000 may be implemented to output treatment assistance information reflecting feature information on the basis of segmentation information obtained by the segmentation process P2000.

The learning process P1000 according to an embodiment of the present application may include a process P 1100 of obtaining a training data set, a process P1200 of training a neural network model, a process P1300 of verifying the neural network model, and a process P 1400 of obtaining parameters of the neural network model.

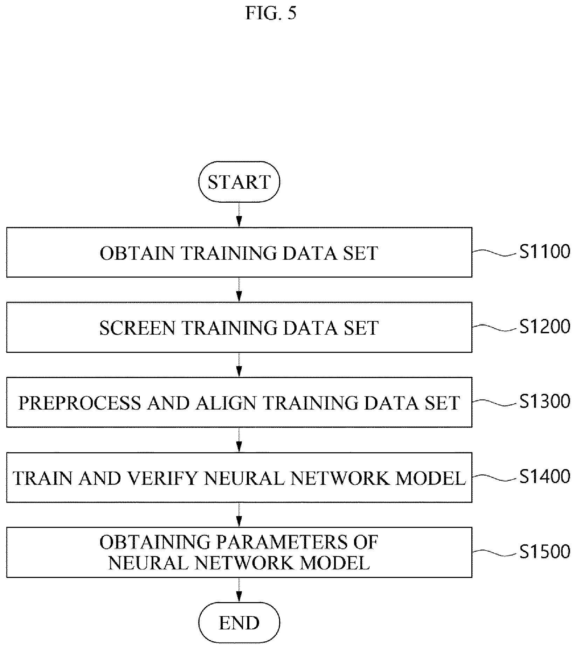

A method of training a neural network model by the learning device 3000 according to an embodiment of the present application will be described with reference to FIG. 5 below. FIG. 5 is a flowchart of a method of training a neural network model by a learning device 3000 according to an embodiment of the present application.

Referring to FIG. 5, the method of training a neural network model by the learning device 3000 according to an embodiment of the present application may include obtaining a training data set (S 1100), screening the training data set (S 1200), preprocessing and aligning the training data set (S1300), training and verifying a neural network model (S 1400), and obtaining parameters of the neural network model (S1500).

In the obtaining of the training data set (51100), the learning device 3000 according to an embodiment of the present application may obtain training data sets related to a medical image from the medical image obtaining device 1000 or external devices such as a server.

In the obtaining of the training data set (51100), the learning device 3000 according to an embodiment of the present application may obtain feature information related to the medical image from the medical image obtaining device 1000, an external device such as a server, and/or a user input. In this case, the obtained feature information may be a basis on which a parameter set of a feature layer that is a part of a hidden layer of the neural network model is learned or renewed.

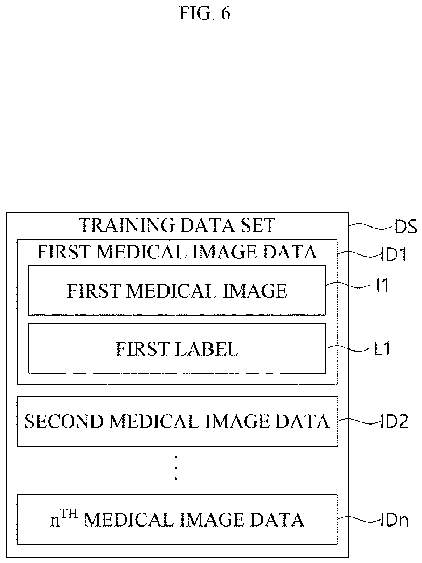

FIG. 6 will now be referred to. FIG. 6 is a diagram of an example of a structure of a training data set related to a medical image according to an embodiment of the present application.

A training data set DS related to a medical image obtained by the learning device 3000 may include at least one piece of medical image data. In other words, the training data set DS obtained by the learning device 3000 may include at least one piece of medical image data, e.g., first medical image data ID1, second medical image data ID2, and n^{th} medical image data IDN.

In addition, the at least one piece of medical image data may include label data of the medical image. For example, the first medical image data ID1 may include first label data L1 of a first medical image I1.

In this case, label-related data included in the training data set illustrated in FIG. 6 may be data labeled manually or automatically by the same operator with respect to the medical image.

However, this is only an example provided for convenience of description, and the label-related data included in the training data set of FIG. 6 may be data labeled manually or automatically by a plurality of operators. In this case, because feature information that may be obtained by the learning device 3000 may include operator information and the like, the training process P 1000 may be sufficiently implemented to achieve the purpose of training a neural network model for segmentation of the medical image by reflecting the feature information.

The medical image data included in the training data set DS obtained by the learning device 3000 may include the medical image and the label-related data.

For example, referring to FIG. 6, the first medical image data ID1 included in the training data set DS may include the first medical image 11 and data related to a first label L1.

Specifically, the first label L1 may be labeled and obtained manually by a clinician who is able to perform a medical procedure on or treat a tumor with respect to the first medical image I1. Alternatively, the first label L1 may be automatically labeled and obtained by an appropriate image segmentation technique.

The label-related data may be a label related to an area in which a tumor medical procedure or treatment is to be performed. For example, in order to perform a medical procedure on or treat a tumor, an area to which radiation is to be emitted and an area to which radiation should not be emitted may be defined. In this case, the label-related data included in the training data set DS of FIG. 6 may be label data defining a plurality of areas including an area corresponding to the tumor in the medical image (hereinafter referred to as a tumor area), an area to which radiation is to be emitted, and an area to which radiation should not be emitted.

Alternatively, the label-related data may include a plurality of independent labels.

For example, the label-related data may include a label related to an area to which radiation is to be emitted and/or an area to which radiation should not be emitted.

For example, the label-related data (e.g., the first labels L1 to an n^{th} label Ln) may include a plurality of independent labels corresponding to an area to which radiation is to be emitted, including an area GTV corresponding to an organ with a tumor, an area related to a margin when a motion of a patient is taken into consideration during radiation treatment, and/or an area PTV related to a margin when a fine motion of the organ is taken into consideration when tumor treatment is performed a plurality of times.

For example, the label-related data (e.g., the first label L1 to the n^{th} label Ln) may include a label related to an area OAR to which radiation should not be emitted.

The medical image and the label-related data included in the medical image data may be a basis on which an artificial neural network model is trained and verified in relation to a training method according to an embodiment of the present application.

The medical image data included in the training data set DS may further include data related to feature information of the medical image.

The data related to the feature information may be related to at least one of operator information, patient information, tumor information, and radiation information as described above.

In this case, the information related to the feature information may be structured as metadata of the medical image. For example, the patient information including identification information (e.g., age, gender, name, etc.) of a patient with respect to the medical image may be structured as metadata of the medical images.

The medical image data included in the training data set DS may further include data related to the orientation of the medical image. In this case, the learning device 2000 may be implemented to align the medical image on the basis of the data related to the orientation of the medical image.

Although FIG. 6 illustrates only the data included in the first medical image data ID1, the data is only an example for convenience of description, and medical image data of a training data set including second medical image data ID2 or n^{th} medical image data IDn may include images and label-related data.

However, the learning device 3000 may be implemented to obtain the data related to the orientation of the medical image and/or the data related to the feature information from an external device, separately from the medical image.

Alternatively, the learning device 3000 may be implemented to receive the data related to the orientation of the medical image and/or the data related to the feature information from user input.

In the screening of the medical image data set (S1200), the learning device 3000 according to an embodiment of the present application may be implemented to screen the training data set obtained in the obtaining of the training data set (S 1000) or select only some medical image data from the medical image data included in the training data set.

For example, some medical image data of the obtained training data set may not be appropriate for training an artificial neural network model for segmentation. For example, some medical image data may include serious artifacts or noise. Such medical image data may not be suitable for training the artificial neural network model.

Thus, the learning device 3000 may be implemented to screen the medical image data included in the obtained training data set or to select medical image data effective to train the artificial neural network model.

In the preprocessing and aligning of the training data set (S 1300), the learning device 3000 according to an embodiment of the present application may be implemented to remove noise or artifacts in the medical image included in the training data set or to perform a preprocess operation for correcting the intensity of the medical image.

In addition, the learning device 3000 according to an embodiment of the present application may be implemented to align the medical image on the basis of the data related to the orientation of the medical image or align the medical image by matching the medical image with a template.

In this regard, the aligning of the medical image may be implemented by the learning device 3000 in relation to the preprocessing operation of the medical image analysis device 2000 described above. Alternatively, the preprocessing and aligning of the medical image may be implemented by the medical image analysis device 2000 through exchange of data between the learning device 3000 and the medical image analysis device 2000, and thereafter, a resultant medical image may be transmitted to the learning device 3000.

In the training and verifying of the neural network model (S1400), the learning device 3000 according to an embodiment of the present application may train the artificial neural network model for segmentation of the medical image.

Specifically, the artificial neural network model may include an input layer for receiving medical image data, an output layer for outputting a labeling result, which is a segmentation result, and a hidden layer including at least one node.

In this case, the learning device 3000 may be implemented to input the input medical image data included in the obtained training data set through the input layer and obtain output data related to a label of the medical image data, which is obtained by the neural network model, through the output layer.

For example, the learning device 3000 may be implemented to train an artificial neural network configured to receive the first medical image data ID1 and output a first prime label L1' through the output layer. The learning device 3000 may receive the second medical image data ID2 through the input layer and obtain a second prime label L2' output through the output layer.

In this case, the learning device 3000 may be implemented to renew the neural network model on the basis of the first label L1 included in the first medical image data ID1, the first prime label L1' obtained through the output layer, a second label L2 included in the second medical image data ID2, the second prime label L2' obtained through the output layer, etc.

In addition, the learning device 3000 may provide a neural network model in which a feature layer is part of the hidden layer to calculate a feature vector for segmentation of the medical image to reflect feature information related to the medical image. In this case, the learning device 3000 may train the neural network model, for segmentation of the medical image to reflect the feature information, through a learning process of renewing a parameter set of at least some nodes of the feature layer included in the hidden layer. That is, the learning device 3000 may train the neural network model to obtain a feature vector, for segmentation of the medical image to reflect the feature information, by renewing a parameter set of some nodes of the feature layer.

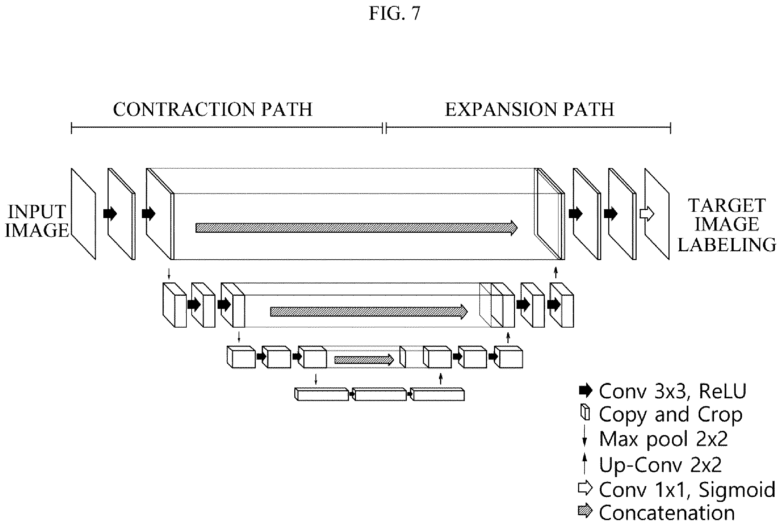

An example of an artificial neural network model that may be used by the learning device 3000 according to an embodiment of the present application will be described with reference to FIG. 7 below.

FIG. 7 illustrates an example of an artificial neural network model that may be used by the learning device 3000 according to an embodiment of the present application.

Referring to FIG. 7, the learning device 3000 according to an embodiment of the present application may use a U-net as an artificial neural network for medical image segmentation.

The U-net used in image segmentation may be configured as an architecture including a contraction path and an expansion path.

Specifically, the contraction path of the U-net can be configured such that two-times convolution and max pooling are consecutively performed. In this case, in the contraction path of the U-net, features related to an image may be extracted.

However, because the size of a feature map reduces in the contraction path, the U-net may further include the expansion path to restore the size of the feature map.

The expansion path of the U-net may be configured such that up-convolution and two-times convolution are consecutively performed. In this case, in the expansion path of the U-NET, the image and the size of the feature map may be extracted.

In addition, an architecture of the U-net may be configured for concatenation of feature maps of the same level to provide location information related to characteristics to the expansion path from the contraction path.

In this case, based on the difference between a label of an input image and a label of an output target image, a parameter set or weight set of at least one node of a layer included in the U-net may be adjusted such that the difference between the label of the input image and the label of the target image is minimal.

Specifically, the learning device 3000 may be implemented to repeatedly adjust the parameter set (or weight set) of the at least one node so as to obtain a parameter or weight of the at least one node for minimizing the difference between the label of the input image and the label of the target image from a neural network model.

In addition, a neural network model that may be used by the learning device 3000 according to an embodiment of the present application may be configured to reflect a label according to feature information related to medical image data of the training data set. Label information of a medical image included in the training data set may vary according to feature information. For example, medical image data may include first label information when the same medical image corresponds to first feature information and include second label information when the same medical image corresponds to second feature information.

More specifically, a result of labeling a medical image by an operator having first operator information may include the first label information and a result of labeling the medical image by an operator having second operator information may include the second label information.

The learning device 3000 according to an embodiment of the present application may train a neural network model for segmentation of a medical image to reflect label information corresponding to feature information related to medical image data. For example, the neural network model may be trained to output the medical image on the basis of the first feature information using the first segmentation information and to output the medical image on the basis of the second feature information using the second segmentation information.

For example, a feature layer for training a label according to feature information related to the medical image data of the training data set may be included in a layer of the neural network model used by the learning device 3000 according to an embodiment of the present application.

For example, when the medical image data of the training data set includes a medical image and label data according to the first feature information, the learning device 3000 according to an embodiment of the present application may be implemented to renew at least one node of the feature layer included in the neural network to have a first parameter set for segmentation of the medical image to reflect the first feature information.

When the medical image data of the training data set includes a medical image and label data according to the second feature information, the learning device 3000 according to an embodiment of the present application may be implemented to train at least one node of the feature layer included in the neural network to have a second parameter set for segmentation of the medical image to reflect the second feature information.

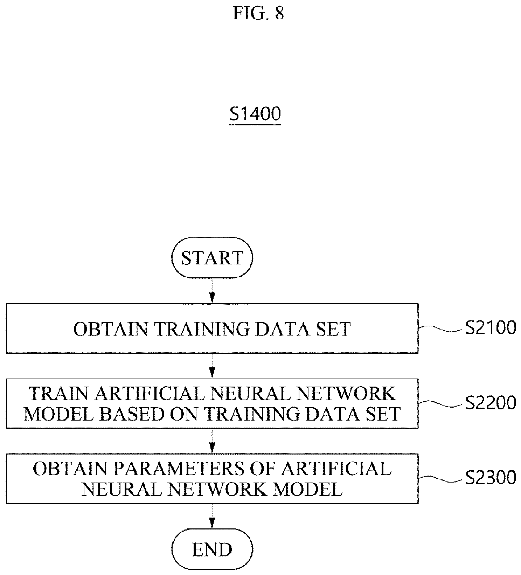

Training a neural network model for segmentation of a medical image to reflect feature information will be described with reference to FIGS. 8 to 14 below.

As described above, the learning device 3000 according to an embodiment of the present application may train an artificial neural network model on the basis of label data included in output data output through the output layer of the neural network model.

Specifically, in the training of the artificial neural network model (S1400), label-related data included in the medical image data obtained in the obtaining of the training data set (S 1100) may be obtained.

In this case, the learning device 3000 may be implemented to train the neural network model on the basis of the medical image data and the label data included in the output data.

More specifically, the learning device 3000 may be implemented to train the neural network model by adjusting a weight set or parameter set of at least one node included in the hidden layer of the neural network model on the basis of the difference between the label data included in the medical image data and the label data included in the output data.

For example, the learning device 3000 may input a first medical image I1 to the input layer of the artificial neural network to obtain label data corresponding to a first-A label L1A included in output data. In this case, the learning device 3000 may train the neural network model on the basis of label data corresponding to the first label L1 included in the first medical image data ID1 and label data related to the first-A label L1A. For example, the learning device 3000 may train the neural network model by adjusting a weight set or parameter set of at least one node included in the hidden layer of the neural network model on the basis of the difference between the first label L1 and the first-A label L1A.

As another example, the learning device 3000 may input an i^{th} image Ii to the input layer of the artificial neural network to obtain label data corresponding to an i^{th}-A label LiA included in output data. In this case, the learning device 3000 may train the neural network model on the basis of label data corresponding to an i^{th} label Li included in the i^{th} image data IDi and label data related to the i^{th}-A label LiA. For example, the learning device 3000 may train the neural network model by adjusting a weight set or parameter set of at least one node included in the hidden layer of the neural network model on the basis of the difference between the i^{th} label Li and the i^{th}-A label LiA. Here, i may be a random number.

In addition, the learning device 3000 according to an embodiment of the present application may be implemented to train or renew the neural network model for segmentation of the medical image to reflect the feature information related to the medical image.

For example, the learning device 3000 may train or renew the neural network model for segmentation of the medical image to reflect the feature information on the basis of the label data included in the training data set and label data included in output data output through the output layer of the neural network model.

For example, the learning device 3000 may input a j^{th} medical image, which is included in the training data set related to the first feature information, to the input layer of the neural network model to obtain label data corresponding to a j^{th}-A label LjA included in output data. In this case, the learning device 3000 may be implemented to train or renew the neural network model to calculate a first feature vector for segmentation of the medical image to reflect the first feature information, based on label data corresponding to the j^{th} label Lj included in the training data set related to the first feature information and the j^{th}-A label data LjA output from the neural network model. Specifically, the learning device 3000 may be implemented to renew a parameter set of at least some nodes of the feature layer included in the neural network model so as to minimize the difference between the label data corresponding to the j^{th} label Lj and the j^{th}-A label LjA output from the neural network model.

For example, the learning device 3000 may input a k^{th} medical image, which is included in a training data set related to the second feature information, to the input layer of the neural network model to obtain label data corresponding to a k^{th}-A label LkA included in output data. In this case, the learning device 3000 may be implemented to train or renew the neural network model to calculate a second feature vector for segmentation of the medical image to reflect the second feature information, based on label data corresponding to the k^{th} label Lk included in the training data set related to the second feature information and the k^{th}-A label data LkA output from the neural network model. Specifically, the learning device 3000 may be implemented to renew a parameter set of at least some nodes of the feature layer included in the neural network model so as to minimize the difference between the label data corresponding to the k^{th} label Lk and the k^{th}-A label LkA output from the neural network model.

However, the above description provides only examples, and the learning device 3000 according to an embodiment of the present application may be implemented to renew a parameter set of any nodes of the neural network model or weight sets of a node so as to train the neural network model for segmentation of the medical image to reflect the feature information.

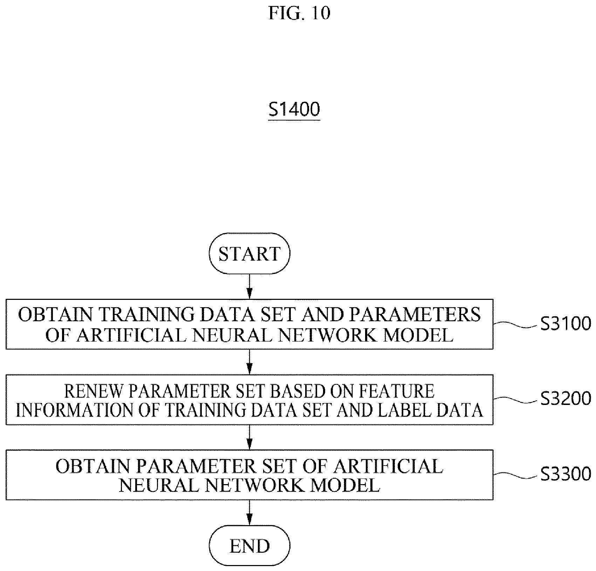

The renewing of the parameter set of at least some nodes of the feature layer constituting part of the hidden layer of the neural network model will be described in detail with reference to FIGS. 10 to 14 below.

In the verifying of the artificial neural network model (S1400), the learning device 3000 according to an embodiment of the present application may verify the trained artificial neural network model.

For example, the learning device 3000 according to an embodiment of the present application may obtain output data including label data output through the trained neural network model, based on at least one piece of medical image data included in the training data set DS. In this case, the learning device 3000 may verify the trained neural network model on the basis of label data related to the at least one piece of medical image data and the label data output through the trained neural network model.

For example, the learning device 3000 may compare a similarity between the label data related to the at least one piece of medical image data and the label data output through the trained neural network model to verify whether a parameter set or weight set of nodes of the hidden layer of the trained neural network model is appropriate.

In addition, in the verifying of the artificial neural network model (S1400), the learning device 3000 according to an embodiment of the present application may verify a parameter set of at least some nodes of the feature layer including a parameter set for calculation of a feature vector.

For example, the learning device 3000 according to an embodiment of the present application may obtain label data output on the basis of a first feature vector calculated on the basis of the trained neural network model with the feature layer and a first parameter set of some nodes of the feature layer, based on at least one piece of medical image data included in the training data set DS related to the first feature information. In this case, the learning device 3000 may verify the trained neural network model on the basis of label data related to at least one piece of medical image data related to the first feature information and label data output through the output layer. For example, the learning device 3000 may compare a similarity between the label data related to the at least one piece of medical image data related to the first feature information and the label data output through the output layer to verify whether the parameter set of at least some nodes of the feature layer is appropriate.

For example, the learning device 3000 according to an embodiment of the present application may obtain label data output on the basis of a second feature vector calculated on the basis of the trained neural network model with the feature layer and a second parameter set of some nodes of the feature layer, based on at least one piece of medical image data included in the training data set DS related to the second feature information. In this case, the learning device 3000 may verify the trained neural network model on the basis of label data related to at least one piece of medical image data related to the second feature information and label data output through the output layer. For example, the learning device 3000 may compare a similarity between the label data related to the at least one piece of medical image data related to the second feature information and the label data output through the output layer to verify whether the parameter set of at least some nodes of the feature layer is appropriate.

In the obtaining of the artificial neural network model (S 1500), the learning device 3000 according to an embodiment of the present application may repeatedly train the artificial neural network model with respect to each piece of medical image data included in the training data set and verify the artificial neural network model to obtain a neural network model with at least one node having a weight set or parameter set for minimizing the difference between label data included in the medical image data and label data output from the artificial neural network model.

In the obtaining of the artificial neural network model (S 1500), the learning device 3000 according to an embodiment of the present application may obtain a parameter set for minimizing the difference between label data included in the medical image data set of the training data set related to the feature information and label data output based on a parameter set of some nodes included in the feature layer.

A parameter set (or weight set) of a node of the obtained neural network model may be used for the artificial neural network model for segmentation of the medical image in the segmentation process P2000.

A parameter set related to some nodes of the obtained feature layer may be used in a medical image segmentation process of the segmentation process P2000.

Although segmentation using the artificial neural network has been described above, the learning device 3000 or the medical image analysis device 2000 set forth herein may employ various image segmentation algorithms, including the image segmentation using the artificial neural network.

For example, an image segmentation algorithm may be provided as a machine learning model. A representative example of the machine learning model includes an artificial neural network. Specifically, a representative example of the artificial neural network includes a deep learning artificial neural network that includes an input layer for receiving data, an output layer for outputting a result, and a hidden layer interposed between the input layer and the output layer to process data. Specifically, examples of the artificial neural network include the convolution neural network, the recurrent neural network, the deep neural network, the generative adversarial network, etc., and as used herein, the artificial neural network should be understood to include the artificial neural network described above, other various types of artificial neural networks, and a combination thereof and is not necessarily limited to the deep learning artificial neural network.

In addition, the machine learning model is not necessarily limited to the artificial neural network model and may further include the K-nearest neighboring algorithm (KNN), RandomForest, the support vector machine (SVM), and the principal component analysis (PCA), etc. and may include an ensemble thereof or a combination of other various methods. It should be understood in embodiments described with respect to the artificial neural network that the artificial neural network may be replaced with other machine learning models unless otherwise specified.

Furthermore, as used herein, the image segmentation algorithm is not necessarily limited to the machine learning model. That is, the image segmentation algorithm may include various judgment/determination algorithms other than the machine learning model.

Therefore, as used herein, the image segmentation algorithm should be understood in a comprehensive sense to include various types of algorithms for performing segmentation using image data.

A method of training a neural network model for segmentation of a medical image by the learning device 3000 according to an embodiment of the present application will be described in detail with reference to FIGS. 8 to 14 below. According to the present embodiment, the training of the neural network model of FIG. 5 (S 1400) may include obtaining a parameter of the neural network model.

According to the present embodiment, the parameter of the neural network model may include a parameter of some nodes of the feature layer which constitutes part of the hidden layer trained to output a feature vector for segmentation of the medical image according to the feature information.

For example, the neural network model for segmentation of the medical image may be primarily trained, and secondly trained by renewing a parameter set (which may be referred to as a feature parameter set) of at least one node (which may be referred to as a feature node) of a feature layer added to a point on the hidden layer of the trained neural network model to reflect the feature information. In this case, according to an embodiment, a parameter set of a node (hereinafter referred to as a common node) included in the hidden layer other than the feature node may be renewed when the neural network model is primarily trained and may be fixed, and feature parameter sets of at least one feature node of the feature layer may be renewed when the neural network model is secondly trained.

For example, the feature layer may be located at a bottleneck layer (a layer between an encoder and a decoder) of the artificial neural network of the primarily trained neural network model. The feature layer may be additionally combined with or added to the bottleneck layer (layer between the encoder and the decoder) after the neural network model is primarily trained. Alternatively, the feature layer may constitute part of the hidden layer of the primarily trained neural network model, and a feature parameter set of a feature node included in the feature layer may be configured not to be renewed when the neural network model is primarily trained and to be renewed when the neural network model is secondly trained.

Various embodiments of training the neural network model to segment a medical image to reflect feature information will be described with reference to FIGS. 8 to 14 below.

FIG. 8 will now be referred to. FIG. 8 is a flowchart of a method of training a neural network model according to an embodiment of the present application. More specifically, FIG. 8 is a detailed flowchart of the training of the neural network model in operation S 1400 of FIG. 5.

The method of training the neural network model according to an embodiment of the present application may include obtaining a training data set (S2100), training an artificial neural network model on the basis of the training data set (S2200), and obtaining a parameter of the artificial neural network model (S2300).

In the obtaining of the training data set (S2100), the learning device 3000 according to an embodiment of the present application may obtain a training data set.

For example, the training data set may include at least one piece of medical image data as described above with reference to FIG. 6.

For example, the learning device 3000 according to an embodiment of the present application may obtain a plurality of training data sets. For example, the learning device 3000 may obtain a plurality of training data sets including a first training data set and a second training data set.

In this case, the first training data set may include at least one piece of medical image data including label data of a medical image according to first feature information. For example, the first training data set may include medical image data related to the first feature information. For example, the first training data set may include medical image data including label data of a medical image obtained by an operator having first operator information.

On the other hand, the second training data set may include at least one piece of medical image data including label data of a medical image related to second feature information different from the first feature information. For example, the second training data set may include medical image data related to the second feature information. For example, the second training data set may include medical image data including label data of a medical image obtained by an operator having second operator information different from the first operator information. Alternatively, the second training data set may include medical image data including label data of an obtained medical image in relation to feature information (e.g., patient information, tumor information, radiation information, etc.) which is different from operator information.

The learning device 3000 according to an embodiment of the present application may obtain feature information. In this case, the feature information may be information related to operator information, patient information, tumor information and/or radiation information as described above.

In this case, the learning device 3000 according to an embodiment of the present application may obtain a user input related to the feature information from the input module 2400.

Alternatively, the learning device 3000 according to an embodiment of the present application may obtain a user input related to the feature information from an external device.

Alternatively, the learning device 3000 according to an embodiment of the present application may obtain the feature information by obtaining metadata structured with respect to the medical image. For example, the feature information, and particularly, the patient information (e.g., personal information of a patient, etc.), may be structured as metadata with respect to the medical image. In this case, the learning device 3000 may be implemented to obtain the feature information by obtaining the metadata structured with respect to the medical image.

In the training of the artificial neural network model on the basis of the training data set (S2200), the learning device 3000 according to an embodiment of the present application may be implemented to train the artificial neural network model on the basis of the obtained training data set.

For example, the learning device 3000 according to an embodiment of the present application may be implemented to train the artificial neural network model on the basis of the obtained training data set.

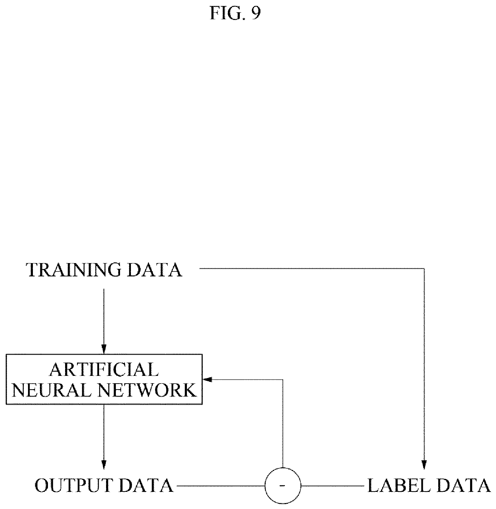

FIG. 9 will now be referred to. FIG. 9 is a schematic diagram illustrating a method of training an artificial neural network model according to an embodiment of the present application.

Referring to FIG. 9, the learning device 3000 according to an embodiment of the present application may input training data to an input layer of an artificial neural network and obtain output data obtained through an output layer of the artificial neural network. In this case, the output data may be data related to a label of a medical image included in the training data set.

In this case, the learning device 3000 according to an embodiment of the present application may train the artificial neural network model on the basis of label data included in the output data and label data related to the medical image included in the training data set.

More specifically, the learning device 3000 according to an embodiment of the present application may adjust a parameter set or weight set of a node included in the artificial neural network model on the basis of the difference between the label data included in the output data and the label data related to the medical image included in the training data set.

The learning device 3000 according to an embodiment of the present application may repeatedly adjust the parameter set or the weight set of each node of the artificial neural network model to obtain a parameter set and/or the weight set of the artificial neural network model for minimizing the difference between the label data included in the output data and the label data related to the medical image included in the training data set.

For example, the learning device 3000 according to an embodiment of the present application may be implemented to train the artificial neural network model on the basis of a training data set, including a first training data set and a second training data set.

In this case, the first training data set may include first label data corresponding to a medical image related to first feature information and the second training data set may include second label data corresponding to a medical image related to second feature information different from the first feature information.

For example, the learning device 3000 according to an embodiment of the present application may train the artificial neural network model on the basis of the training data set, including the first training data set and the second training data set, without considering the feature information. In other words, the method of training the neural network model shown in FIG. 8 may be a method of training a neural network model for segmentation of a medical image regardless of feature information.

As another example, the learning device 3000 according to an embodiment of the present application may be implemented to train the artificial neural network model in units of feature information in consideration of feature information. For example, the learning device 3000 may train the neural network model to include a node having a first parameter set (or first weight set) on the basis of a first training data set including first label data corresponding to the medical image related to the first feature information. On the other hand, the learning device 3000 may train the neural network model to include a node having a second parameter set (or second weight set) on the basis of a second training data set including second label data corresponding to the medical image related to the second feature information.

For example, referring to FIG. 9, the learning device 3000 may input the first training data set related to the first feature information to an input layer of the artificial neural network model and obtain output data obtained through an output layer of the artificial neural network model. In this case, the output data may be data related to a label of the medical image.

In this case, the learning device 3000 according to an embodiment of the present application may train the artificial neural network model on the basis of the output data and first label data related to the medical image included in the first training data set. For example, the first label data included in the first training data set may be data labeled for the medical image according to the first feature information. In this case, the learning device 3000 may train a first artificial neural network model for segmentation of the medical image to reflect the first feature information on the basis of the output data and the first label data.

More specifically, the learning device 3000 according to an embodiment of the present application may adjust a parameter set or weight set of a node included in the artificial neural network model on the basis of the difference between the output data and the first label data related to the medical image included in the first training data set.

In this case, the learning device 3000 may repeatedly adjust a parameter set or a weight set of each node included in the artificial neural network model to train the first artificial neural network model, which includes a node having a first parameter set and/or a first weight set for minimizing the difference between the output data and the first label data related to the medical image included in the first training data set.

As another example, the learning device 3000 may input a second training data set related to the second feature information, which is different from the first feature information, to the input layer of the artificial neural network model and obtain output data obtained through the output layer of the artificial neural network model. In this case, the output data may be data related to a label of the medical image.

In this case, the learning device 3000 according to an embodiment of the present application may train the artificial neural network model on the basis of the output data and second label data related to a medical image included in the second training data set. For example, the second label data included in the second training data set may be data labeled for the medical image according to the second feature information. In this case, the learning device 3000 may train a second artificial neural network model for segmentation of the medical image to reflect the second feature information on the basis of the output data and the second label data.

More specifically, the learning device 3000 according to an embodiment of the present application may adjust a parameter set or weight set of a node included in the artificial neural network model on the basis of the difference between the output data and the second label data related to the medical image included in the second training data set.

In this case, the learning device 3000 may repeatedly adjust a parameter set or weight set of each node included in the artificial neural network model to train the second artificial neural network model, which includes a node having a second parameter set and/or a second weight set for minimizing the difference between the output data and the second label data related to the medical image included in the second training data set.

In this case, the second parameter set and/or the second weight set may be at least partially different from the first parameter set and/or the first weight set related to the first feature information.

Training a neural network model by a method of renewing a parameter set and/or a weight set of a node included in the neural network model for segmentation of a medical image to reflect feature information has been described above. However, this is only an example, and the neural network model may be trained by a method of renewing a parameter set of at least some nodes of a feature layer by combining the feature layer with some layers of an artificial neural network model to calculate a feature vector for segmentation of the medical image in consideration of feature information.

For example, the learning device 3000 according to an embodiment of the present application may renew a parameter set of at least some nodes of a feature layer constituting part of a hidden layer, based on the first training data set including the first label data corresponding to the medical image related to the first feature information. In this case, the renewed parameter set may be a parameter set renewed to output a first feature vector for segmentation of the medical image to correspond to the first label data.

Alternatively, the learning device 3000 according to an embodiment of the present application may renew a parameter set of at least some nodes of the feature layer constituting part of the hidden layer, based on the second training data set including the second label data corresponding to the medical image related to the second feature information different from the first feature information. In this case, the parameter set renewed based on the second training data set may be a parameter set renewed to output a second feature vector for segmentation of the medical image to correspond to the second label data. This will be described in detail with reference to FIGS. 10 to 14 below.

In the obtaining of the parameter of the artificial neural network model (S2300), the learning device 3000 according to an embodiment of the present application may obtain a parameter set and/or a weight set of a node of the artificial neural network model as a result of training the artificial neural network model.

For example, the learning device 3000 according to an embodiment of the present application may obtain the parameter set and/or the weight set of the node of the artificial neural network model by training the neural network model on the basis of the training data set regardless of feature information. In this case, the learning device 3000 according to an embodiment may be implemented to renew a parameter set of at least some nodes of the feature layer to obtain a neural network model reflecting feature information illustrated in FIG. 10 by fixing the parameter set obtained in operation S2300 and combining the feature layer with or adding the feature layer to some layers (a bottleneck layer which is a layer between an encoder and a decoder) of the neural network model.

For example, the learning device 3000 according to an embodiment of the present application may obtain a parameter set and/or a weight set of a node of an artificial neural network model configured to reflect feature information by training the neural network model in consideration of the feature information and on the basis of a training data set. For example, as described above, the learning device 3000 may be implemented to train the first neural network model on the basis of the first training data set related to the first feature information and obtain a first parameter set (or a first weight set) of a node included in the first neural network model.

On the other hand, the learning device 3000 may be implemented to train the second neural network model on the basis of the second training data set related to the second feature information and obtain a second parameter set (or a second weight set) of a node included in the second neural network model.

The obtained parameter sets (or weight sets) may be used to segment a target medical image in the segmentation process P2000 of FIG. 4.

As another example, the learning device 3000 according to an embodiment of the present application may be implemented to learn a parameter set of at least some nodes of the feature layer to calculate a feature vector related to the feature information by combining the feature layer with some layers (e.g., the bottleneck layer which is a layer between the encoder and the decoder) of the artificial neural network model to reflect the feature information in operation S2300. In this case, the learning device 3000 according to an embodiment of the present application may obtain a parameter set of at least some nodes of the feature layer.

The parameter set of the at least some nodes of the feature layer may be added to the node corresponding to the trained neural network model in the segmentation process P2000 of FIG. 4 to be used for segmentation of a target medical image.

Alternatively, the parameter set of the at least some nodes of the feature layer may be additionally renewed according to a training method related to FIG. 10 to be described below.

A method of renewing a parameter set of at least some nodes of a feature layer constituting part of a hidden layer according to an embodiment of the present application will described with reference to FIGS. 10 to 14 below.

FIG. 10 is a flowchart of a method of training an artificial neural network model according to an embodiment of the present application. More specifically, FIG. 10 is a detailed flowchart of the training of the neural network model in operation S 1400 of FIG. 5.

The learning device 3000 according to an embodiment of the present application may further renew a parameter set of at least some nodes of a feature layer constituting a hidden layer in relation to training an artificial neural network model.

The method of training an artificial neural network model according to an embodiment of the present application may include obtaining a training data set and a parameter of the artificial neural network model (S3100), renewing a parameter set on the basis of feature information of the training data set and label data (S3200), and obtaining a parameter set of the artificial neural network model (S3300).

In this case, in the obtaining of the training data set and the parameter of the artificial neural network model (S3100), the learning device 3000 according to an embodiment of the present application may obtain a training data set for segmentation of a medical image.

In this case, the training data set may include a plurality of training data sets classified according to feature information. In other words, the training data set may include a first training data set related to first feature information and a second training data set related to second feature information.

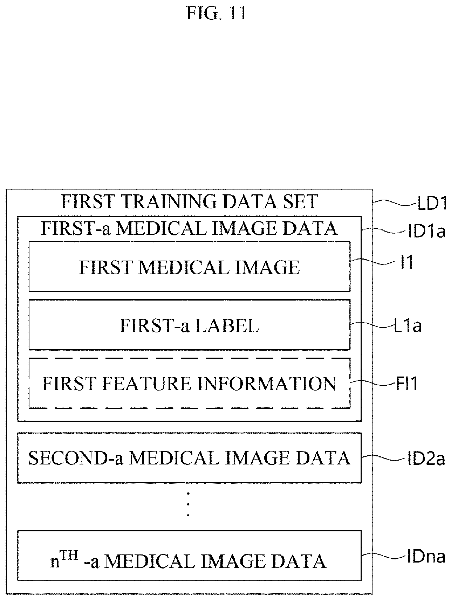

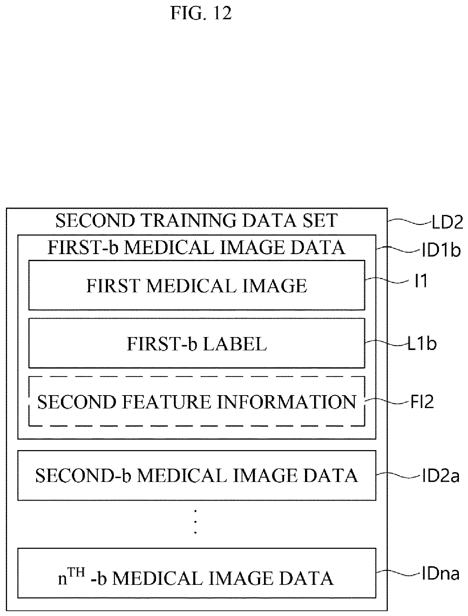

FIGS. 11 and 12 will now be referred to. FIGS. 11 and 12 are diagrams of examples of a structure of a training data set related to a medical image according to an embodiment of the present application. Specifically, the training data sets of FIGS. 11 and 12 may be used to renew a parameter set of at least some nodes of a feature layer added to some layers of an artificial neural network model for segmentation of a medical image to reflect feature information.

Referring to FIG. 11, the first training data set may include at least one piece of medical image and label-related data related to first feature information and corresponding to the at least one medical image. In other words, the first training data set may include at least one piece of medical image data including medical image and label-related data.

In addition, the learning device 3000 may obtain data related to feature information in connection with the first training data set. The data related to the feature information may be obtained from an external device or obtained from a user input through an input module. The data related to the feature information may be structured as metadata with respect to the medical image, and the learning device 3000 may obtain the metadata to obtain the data related to the feature information.

In this case, the first training data set may include label-related data defined according to the first feature information in relation to at least one medical image.

For example, the first training data set may include first-a medical image data, and the first-a medical image data may include a first medical image and data related to a first-a label. In this case, the first-a label may be label data defined with respect to a first medical image in connection with the first feature information.

For example, the first-a label may be a label related to an area to which radiation is to be emitted and/or an area to which radiation should not be emitted, which are defined with respect to the first medical image by a first operator having feature information of first operator information.

As another example, the first training data set may include second-a medical image data, and the second-a medical image data may include a second medical image and data related to a second-a label. In this case, the second-a label may be label data generated with respect to a second medical image in connection with the first feature information.

For example, the second-a label may be a label related to an area to which radiation is to be emitted and/or an area to which radiation should not be emitted, which are defined with respect to the second medical image by the first operator having first feature information of the first operator information.

At least one piece of label-related data included in the first training data set may be data defined with respect to the medical image in relation to the first feature information.

The learning device 3000 according to an embodiment of the present application may renew a parameter set of at least some nodes of a feature layer, for outputting a first feature vector for segmentation of the medical image to reflect the first feature information, using the first training data set.

Referring to FIG. 12, the second training data set may include at least one piece of medical image and label-related data related to second feature information and corresponding to the at least one medical image. In other words, the second training data set may include a least one piece of medical image data including the medical image and label-related data.

In addition, the learning device 3000 may obtain data related to feature information in connection with the second training data set. As described above, the data related to the feature information may be obtained from an external device or obtained from a user input through an input module. In addition, the data related to the feature information may be structured as metadata with respect to the medical image, and the learning device 3000 may obtain the metadata to obtain the data related to the feature information.

In this case, the second training data set may include label-related data defined according to the second feature information, which is different from the first feature information, in relation to at least one medical image.

For example, the second training data set may include first-b medical image data, and the first-b medical image data may include the first medical image and data related to a first-b label. In this case, the first-b label may be label data generated with respect to the first medical image in connection with the second feature information.

For example, the first-b label may be a label related to an area to which radiation is to be emitted and/or an area to which radiation should not be emitted, which are defined with respect to the first medical image by a second operator having second feature information of the second operator information.

As another example, the second training data set may include second-b medical image data, and the second-b medical image data may include a second medical image and data related to a second-b label. In this case, the second-b label may be label data generated with respect to the second medical image in connection with the second feature information.

For example, the second-b label may be a label related to an area to which radiation is to be emitted and/or an area to which radiation should not be emitted, which are defined with respect to the second medical image by the second operator having the second feature information of the second operator information.

At least one piece of label-related data included in the second training data set may be data defined with respect to the medical image in relation to the second feature information. Specifically, all of the at least one piece of label-related data included in the first training data set may be data related to the first feature information, whereas all of the at least one piece of label-related data included in the second training data set may be data related to the second feature information.

In this case, the first-b label may be different from the first-a label described above with reference to FIG. 11. Specifically, with respect to the same first medical image, the area to which radiation is to be emitted (or the area to which radiation should not be emitted) defined by the first operator having the feature information of the first operator information may be different from the area to which radiation is to be emitted (or the area to which radiation should not be emitted) defined by the second operator having the feature information of the second operator information.

In this case, the second-b label may be different from the second-a label described above with reference to FIG. 11. Specifically, with respect to the same first medical image, the area to which radiation is to be emitted (or the area to which radiation should not be emitted) defined by the first operator having the first feature information of the first operator information may be different from the area to which radiation is to be emitted (or the area to which radiation should not be emitted) defined by the second operator having the second feature information of the second operator information.

The learning device 3000 according to an embodiment of the present application may renew a parameter set of at least some nodes of a feature layer, for outputting a second feature vector for segmentation of the medical image to reflect the second feature information, using the second training data set.

FIGS. 11 and 12 are described above focusing on operator information in connection with feature information related to the first training data set and the second training data set.

However, the above description is merely provided for convenience of description, and the first feature information and/or the second feature information may be related to patient information, tumor information, and/or radiation information, as well as the operator information.

In other words, the learning device 3000 according to an embodiment of the present application may train a neural network model to segment an area to which radiation is to be emitted to perform a medical procedure on or treat a tumor according to feature information related to operator information, patient information, tumor information and/or radiation information. To this end, the feature information of the training data sets illustrated in FIGS. 11 and 12 may be configured in association with operator information, patient information, tumor information, radiation information and/or a combination thereof.

For example, the learning device 3000 according to an embodiment of the present application may be implemented to train the neural network model to segment a medical image differently according to the operator information. For example, a first operator may have feature information related to an aggressive treatment history (e.g., a treatment history with a treatment tendency above an average treatment range) to perform a medical procedure on or treat a tumor. That is, the first operator may define an area to which radiation is to be emitted to perform a medical procedure on or treat a tumor as a first area which is a relatively wide area. On the other hand, the second operator may have feature information associated with a conservative treatment history (e.g., a treatment history with a treatment tendency within the average treatment range) to perform a medical procedure on or treat a tumor. That is, the second operator may define an area to which radiation is to be emitted to perform a medical procedure on or treat a tumor as a second area narrower than the first area.

The learning device 3000 according to an embodiment of the present application may renew a parameter set of at least some nodes of a feature layer constituting part of a hidden layer of the neural network model so that a medical image may be segmented in consideration of the operator information. For example, the learning device 3000 may use the first training data set of FIG. 11 to train the neural network model for segmentation of the medical image dependent on first operator information. In this case, the first training data set of FIG. 11 may be configured to include label data of the medical image related to first feature information including the first operator information. In addition, the learning device 3000 may use the second training data set of FIG. 12 to train the neural network model for segmentation of the medical image dependent on the second operator information. In this case, the second training data set of FIG. 12 may be configured to include label data of the medical image related to the second feature information including the second operator information.

For example, the learning device 3000 according to an embodiment of the present application may be implemented to train the neural network model to segment the medical image differently according to patient information (e.g., age, gender, an underlying disease, and a treatment history). For example, a first patient may have age information belonging to a first age section or have an underlying disease and thus may need to have a conservative radiation treatment (e.g., a radiation treatment of a range that is narrower than an average radiation emission range). That is, there may be a need to define an area, to which radiation is to be emitted to perform a medical procedure on or treat the first patient's tumor, as a first area which is a relatively narrow area. On the other hand, a second patient may have age information belonging to a second age section lower than the first age section or have no underlying disease and thus may need to have an aggressive radiation treatment (e.g., a radiation treatment of a range that is wider than the average radiation emission range). That is, there may be a need to define an area, to which radiation is to be emitted to perform a medical procedure on or treat the second patient's tumor, as a second area wider than the first area.

The learning device 3000 according to an embodiment of the present application may be implemented to train the neural network model to segment a medical image in consideration of patient information. For example, the learning device 3000 may use the first training data set of FIG. 11 to train the neural network model to reflect first patient information. In this case, the first training data set of FIG. 11 may be configured to include label data of the medical image related to the first feature information related to the first operator information. In this case, the learning device 3000 may renew a parameter set of at least one node of the feature layer, which constitutes part of the hidden layer of the neural network model, with a first parameter set to output a feature vector dependent on the first patient information, on the basis of the first patient information and the label data.

In addition, the learning device 3000 may use the second training data set of FIG. 12 to train the neural network model to reflect second patient information. In this case, the second training data set of FIG. 12 may be configured to include label data of the medical image related to second feature information related to the second operator information.

In this case, the learning device 3000 may renew a parameter set of at least one node of the feature layer, which constitutes part of the hidden layer of the neural network model, with a second parameter set to output a feature vector dependent on the second patient information, on the basis of the first patient information and the label data.

For example, the learning device 3000 according to an embodiment of the present application may be implemented to train the neural network model for segmentation of the medical image differently according to the tumor information (e.g., a size, a shape, an expression degree, and the position of a tumor (e.g., a distance to a neighboring organ)). For example, a first tumor of first tumor information has a size less than a first size or has a lower expression degree than a first expression degree and thus may need to have a conservative radiation treatment (e.g., a radiation treatment of a range that is narrower than an average radiation emission range). That is, there may be a need to define an area to which radiation is to be emitted with respect to the first tumor as a first area which is a relatively narrow area. On the other hand, a second tumor of second tumor information has a size greater than a second size or has a higher expression degree than a second expression degree and thus may need to have an aggressive radiation treatment (e.g., a radiation treatment of a range that is wider than the average radiation emission range). That is, there may be a need to define an area to which radiation is to be emitted with respect to the second tumor as a second area wider than the first area.

The learning device 3000 according to an embodiment of the present application may be implemented to train the neural network model to segment a medical image in consideration of tumor information. For example, the learning device 3000 may use the first training data set of FIG. 11 to train the neural network model to reflect first tumor information. In this case, the first training data set of FIG. 11 may be configured to include label data of the medical image related to first feature information related to the first tumor information.

In this case, the learning device 3000 may renew a parameter set of at least one node of the feature layer constituting part of the hidden layer of the neural network model to calculate a feature vector dependent on the first tumor information on the basis of the first tumor information and label data related to a radiation emission area defined in relation to the first tumor information.

In addition, the learning device 3000 may use the second training data set of FIG. 12 to train the neural network model to reflect second tumor information. In this case, the second training data set of FIG. 12 may be configured to include label data of the medical image related to second feature information related to the second tumor information.

In this case, the learning device 3000 may renew a parameter set of at least one node of the feature layer constituting part of the hidden layer of the neural network model to calculate a feature vector dependent on the second tumor information on the basis of the second tumor information and label data related to a radiation emission area defined in relation to the second tumor information.

For example, the learning device 3000 according to an embodiment of the present application may be implemented to train the neural network model for segmentation of the medical image differently according to radiation information (e.g., a type of radiation, a radiation emission period (e.g., a long pulse or a short pulse), an intensity of radiation, and a spot size). For example, first radiation of first radiation information (e.g., a first type or a first manufacturer), may be high-intensity radiation that is relatively dangerous to the body, and when a treatment is conducted using the first radiation, there may be a need to conduct a conservative radiation treatment (e.g., a radiation treatment of a range that is narrower than an average radiation emission range). That is, there may be a need to define an area to which radiation is to be emitted as a first area which is a relatively narrow area in the case of a tumor treatment using the first radiation. On the other hand, second radiation of second radiation information (e.g., a second type or a second manufacturer), may be low-intensity radiation that is relatively safe for the body, and there may be a need to conduct an aggressive radiation treatment (e.g., a radiation treatment of a range that is wider than the average radiation emission range) in this case. That is, there may be a need to define an area to which radiation is to be emitted as a second area wider than the first area in the case of a tumor treatment using the second radiation.

The learning device 3000 according to an embodiment of the present application may train the neural network model for segmentation of a medical image in consideration of radiation information used to treat a tumor. For example, the learning device 3000 may use the first training data set of FIG. 11 to train the neural network model to reflect first radiation information. In this case, the first training data set of FIG. 11 may be configured to include label data of the medical image related to first feature information related to the first radiation information.

In this case, the learning device 3000 may renew a parameter set of at least one node of the feature layer constituting part of the hidden layer of the neural network model to calculate a feature vector dependent on the first radiation information on the basis of the first radiation information and label data related to a radiation emission area defined in relation to the first radiation information.

In addition, the learning device 3000 may use the second training data set of FIG. 12 to train the neural network model to reflect second radiation information. In this case, the second training data set of FIG. 12 may be configured to include label data of the medical image related to second feature information related to the second radiation information.

In this case, the learning device 3000 may renew a parameter set of at least one node of the feature layer constituting part of the hidden layer of the neural network model to calculate a feature vector dependent on the second radiation information on the basis of the second radiation information and label data related to a radiation emission area defined in relation to the second radiation information.

However, the training data sets described above are only examples, and the learning device 3000 according to an embodiment of the present application may obtain a training data set appropriate for training the neural network model for segmentation of the medical image dependent on feature information including operator information, patient information, tumor information, and radiation information as described above, and a combination thereof.

Although learning an area to which radiation is to be emitted has been described above, training the neural network model in relation to an area to which radiation should not be emitted may also be applied in a similar manner.

In the obtaining of the training data set and the parameter of the artificial neural network model (S3100), the learning device 3000 according to an embodiment of the present application may be implemented to obtain an artificial neural network model and a parameter set (or a weight set) of a node of the artificial neural network model.

For example, the parameter set obtained by the learning device 3000 may be a parameter set of a neural network model for learning segmentation of a medical image obtained without considering feature information.

In this case, the learning device 3000 may be implemented to renew a parameter set of some nodes of a feature layer additionally for calculation of a feature vector dependent on feature information by adding the feature layer to or combining the feature layer with a hidden layer of the obtained neural network model. Specifically, the learning device 3000 may be implemented to renew a parameter set of some nodes of the feature layer related to the feature information on the basis of the neural network model having the obtained parameter set and the training data set. Therefore, the learning device 3000 according to an embodiment of the present application may obtain a final neural network model including a parameter set for segmentation of the medical image specifically for the feature information.

As another example, the learning device 3000 according to an embodiment of the present application may obtain a first neural network model having a node with a first parameter set and a second neural network model having a node with a second parameter set, which are trained in consideration of the feature information.

The first neural network model may be a neural network model for segmentation of the medical image to reflect the first feature information and may include a node with the first parameter set.

The medical image analysis device 2000 according to an embodiment of the present application may be implemented to obtain first segmentation information using the first neural network model by segmenting a target medical image to reflect the first feature information.

Alternatively, the learning device 3000 according to an embodiment of the present application may be implemented to obtain a neural network model for precise segmentation of a medical image to be more specific to the first feature information by combining a hidden layer of the first neural network model with an additional feature layer and renewing a parameter set of at least one node of the additional feature layer.

The second neural network model may be a neural network model for segmentation of the medical image to reflect the second feature information and may include a node with the second parameter set different from the first parameter set.

The medical image analysis device 2000 according to an embodiment of the present application may be implemented to obtain second segmentation information using the second neural network model by segmenting a target medical image to reflect the second feature information.

Alternatively, the learning device 3000 according to an embodiment of the present application may be implemented to obtain a neural network model for precise segmentation of a medical image to be more specific to the second feature information by combining a hidden layer of the second neural network model with an additional feature layer and renewing a parameter set of at least one node of the additional feature layer.

In the obtaining of the training data set and the parameter of the artificial neural network model (S3100), the learning device 3000 according to an embodiment of the present application may be implemented to obtain a parameter set for calculation of an initial feature vector related to feature information. For example, as described above with reference to FIG. 8, the learning device 3000 according to an embodiment of the present application may include the parameter set of the neural network model and the feature layer constituting part of the hidden layer of the neural network model to be primarily trained with a parameter set of at least one node of the feature layer. The primarily trained neural network model may be implemented to calculate an initial feature vector dependent on the feature information. In this case, in the obtaining of the training data set and the parameter of the artificial neural network model (S3100), the learning device 3000 according to an embodiment of the present application may be implemented to obtain a neural network model including a parameter set for calculation of an initial feature vector related to the feature information.

In the renewing of the parameter set on the basis of the feature information and the label data of the training data set (S3200), the learning device 3000 according to an embodiment of the present application may be implemented to renew or learn the parameter set on the basis of the artificial neural network model including a node with the obtained parameter set and the training data set.

In this case, with the learning device 3000 according to an embodiment, the neural network model obtained in operation S3100 may be trained independently with the feature information, and in operation S3200, a parameter set of a node of the neural network model obtained in operation S3100 may be fixed. In other words, in operation S3200, the parameter sets obtained in operation S3100 may be fixed not to be renewed. In this case, in operation S3200, the learning device 3000 may renew a parameter set of at least one node included in the feature layer for segmentation of the medical image to be specific to the feature information by adding the feature layer to some layers of the hidden layer of the neural network model.

When an architecture of the neural network model is configured as a U-net based on an encoder and a decoder, the feature layer may be combined with or added to a bottleneck layer (a layer between the encoder and the decoder) of the neural network model obtained in operation S3100 to learn a segmentation operation of the medical image to reflect the feature information.

In addition, in the renewing of the parameter set on the basis of the feature information and the label data of the training data set (S3200), the learning device 3000 according to an embodiment of the present application may train with or renew the parameter set differently according to the feature information.

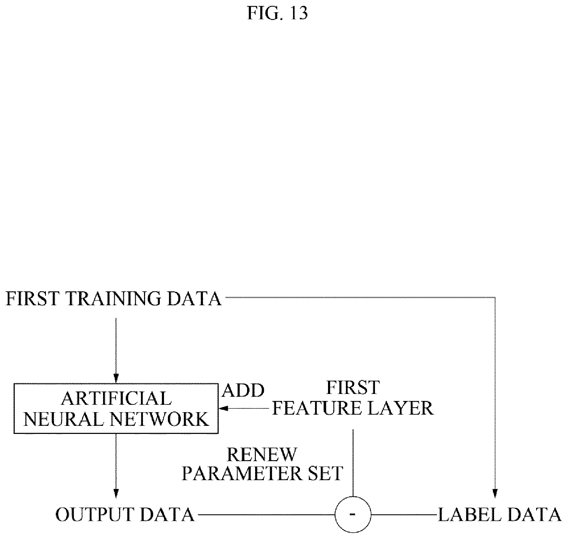

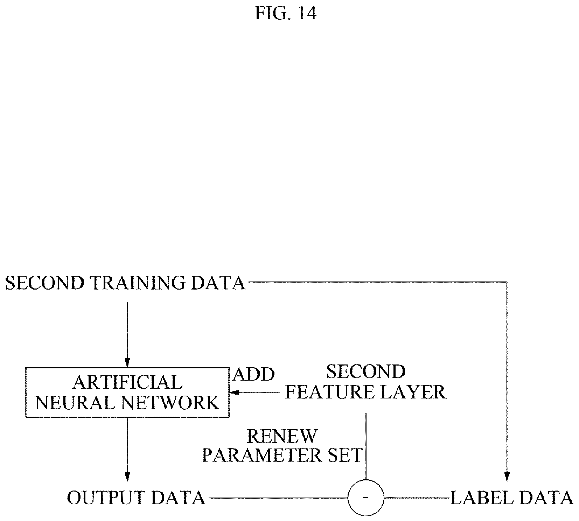

A method of renewing a parameter set according to an embodiment of the present application will be described in detail with reference to FIGS. 13 and 14 below.

FIGS. 13 and 14 are schematic diagrams illustrating a method of renewing a parameter set of a feature layer according to an embodiment of the present application.

Specifically, FIG. 13 is a schematic diagram illustrating a method of renewing a parameter set of a feature layer to calculate a first feature vector on the basis of the first training data set of FIG. 11.

FIG. 14 is a schematic diagram illustrating a method of renewing the parameter set of the feature layer to calculate a second feature vector different from the first feature vector on the basis of the second training data set of FIG. 12.

Referring to FIG. 13, the learning device 3000 according to an embodiment of the present application may be configured to renew or train with a parameter set of at least one node included in a first feature layer on the basis of the first training data set. The first feature layer shown in FIG. 13 may be combined with or added to a hidden layer of a neural network model before renewing the parameter set.

Specifically, the first training data set may include a medical image and label-related data defining a radiation treatment area related to first feature information. In this case, the learning device 3000 may renew the parameter set to obtain a neural network model for calculation of a first feature vector to segment the medical image to be specific to the first feature information.

For example, the learning device 3000 may be implemented to input the medical image included in the first training data set to an input layer of the neural network model and obtain output data through an output layer of the neural network model. In this case, the learning device 3000 may renew a parameter set of at least one node included in a first feature layer constituting part of the hidden layer of the neural network model on the basis of the label data related to the first feature information included in the first training data set and the output data.

For example, the learning device 3000 may input an i^{th} medical image included in the first training data set to the input layer of the neural network model and obtain output data through the output layer of the neural network model.

In this case, the output data may include label data about the i^{th} medical image. For example, the output data may be i^{th}-A label data related to the i^{th} medical image.

In this case, the learning device 3000 according to an embodiment of the present application may be implemented to renew a parameter of at least one node included in the first feature layer on the basis of an i^{th} A label included in the output data and an i^{th}-a label related to the i^{th} medical image included in the first training data set. Specifically, the learning device 3000 may be implemented to renew the parameter set of the at least one node included in the first feature layer such that the difference between the i^{th}-A label and the i^{th}-a label related to the i^{th} medical image included in the first training data set is minimal.

In this case, because the first training data set includes the medical image and label data about a radiation emission area defined in relation to the first feature information, the neural network model trained with the first training data set may be trained to output the medical image as the label data related to the first feature information.

Referring to FIG. 14, the learning device 3000 according to an embodiment of the present application may be configured to renew or train with a parameter set of the feature layer for calculation of a second feature vector based on the second training data set differentiated from the first training data set. The second feature layer shown in FIG. 14 may be combined with or added to the hidden layer of the neural network model before renewing the parameter set.

Specifically, the second training data set may include the medical image and label-related data defining a radiation treatment area related to second feature information. In this case, the learning device 3000 may renew a parameter set to obtain a neural network model for calculation of the second feature vector to segment the medical image to be specific to the second feature information. For example, the learning device 3000 may be implemented to input the medical image included in the second training data set to an input layer of the neural network model and obtain output data through an output layer of the neural network model. In this case, the learning device 3000 may renew a parameter set of at least one node included in a second feature layer constituting part of a hidden layer of the neural network model on the basis of the label data related to the second feature information included in the second training data set and the output data.

For example, the learning device 3000 may input a j^{th} medical image included in the second training data set to the input layer of the neural network model and obtain output data through the output layer of the neural network model.

In this case, the output data may include label-related data about the j^{th} medical image. For example, the output data may include j^{th}-B label data related to the j^{th} medical image.

In this case, the learning device 3000 according to an embodiment of the present application may be implemented to renew a parameter of at least one node included in the second feature layer on the basis of a j^{th}-B label and j^{th}-b label related to the i^{th} medical image included in the second training data set. Specifically, the learning device 3000 may be implemented to renew the parameter set of the at least one node included in the second feature layer such that the difference between the j^{th}-B label and the j^{th}-b label related to the j^{th} medical image included in the second training data set is minimal.

In this case, because the second training data set includes the medical image and label data about a radiation emission area defined in relation to the second feature information, the neural network model trained with the second training data set may be trained to output the medical image as the label data related to the second feature information.

The first feature information and/or the second feature information may be related to operator information, patient information, tumor information and/or radiation information as described above.

In the renewing of the parameter set on the basis of the feature information and the label data of the training data set (S3200), the learning device 3000 according to an embodiment of the present application may be implemented to renew a parameter set on the basis of the operator information, the patient information, the tumor information and/or the radiation information described above. In this case, the learning device 3000 may renew parameters to calculate a feature vector that varies according to a type of feature information (e.g., operator information and patient information), and renew parameters to calculate a feature vector that varies according to the difference between feature information (e.g., first operator information and second operator information) even in a common type of feature information.

For example, the learning device 3000 according to an embodiment of the present application may renew a parameter set on the basis of feature information related to operator information as described above with reference to FIGS. 13 and 14.

For example, the learning device 3000 may renew a parameter set of at least one node of the feature layer included in the neural network model to calculate a first feature vector for segmentation of the medical image to be specific to the operator information on the basis of a training data set related to feature information related to the operator information.

Specifically, the training data set may include label-related data about a radiation emission area for a medical image obtained by a first operator having first operator information. In this case, the learning device 3000 may input the training data set to the input layer of the artificial neural network model and obtain output data through the output layer. In this case, the learning device 3000 may obtain a neural network model having at least one node with a first-a parameter set by renewing a parameter set on the basis of the output data and label data related to the first operator information included in the training data set.

In addition, the training data set may include label-related data about a radiation emission area for a medical image obtained by a second operator having second operator information. In this case, the learning device 3000 may input the training data set to the input layer of the artificial neural network model and obtain output data through the output layer. In this case, the learning device 3000 may obtain a neural network model having at least one node with a first-b parameter set by renewing a parameter set on the basis of the output data and label data related to the second operator information included in the training data set. The learning device 3000 according to an embodiment of the present application may renew the parameter set to obtain a neural network model having a different parameter set according to a difference in operator information. Therefore, the medical image analysis device 2000 may achieve an advantageous effect of automatically dividing the medical image into a plurality of areas using the trained neural network model to be specific to the operator information.

As another example, the learning device 3000 may renew a parameter set of at least one node of the feature layer included in the neural network model to calculate a second feature vector for segmentation of the medical image to be dependent on patient information on the basis of a training data set related to feature information related to the patient information. Specifically, the training data set may include label data for a medical image related to an area to which radiation is to be emitted (or an area to which radiation should not be emitted) to treat a tumor of a first patient having first patient information. In this case, the learning device 3000 may input the training data set to the input layer of the artificial neural network model and obtain output data through the output layer. In this case, the learning device 3000 may obtain a neural network model having at least one node with a second-a parameter set by renewing a parameter set on the basis of the output data and label data related to the first patient information included in the training data set. In addition, the training data set may include label data for a medical image related to an area to which radiation is to be emitted (or an area to which radiation should not be emitted) to treat a tumor of a second patient having second patient information. In this case, the learning device 3000 may input the training data set to the input layer of the artificial neural network model and obtain output data through the output layer. In this case, the learning device 3000 may obtain a neural network model having at least one node with a second-b parameter set by renewing a parameter set on the basis of the output data and label data related to the second patient information included in the training data set. The learning device 3000 according to an embodiment of the present application may renew the parameter set to obtain a neural network model having a different parameter set according to a difference in patient information. Thus, the medical image analysis device 2000 may achieve an advantageous effect of obtaining segmentation information dependent on a patient by using the trained neural network model.

As another example, the learning device 3000 may renew a parameter set of at least one node of the feature layer included in the neural network model to calculate a third feature vector for segmentation of the medical image to be specific to tumor information on the basis of a training data set related to feature information related to the tumor information.

Specifically, the training data set may include label data for a medical image related to an area to which radiation is to be emitted (or an area to which radiation should not be emitted) to treat a first tumor of first tumor information. In this case, the learning device 3000 may input the training data set to the input layer of the artificial neural network model and obtain output data through the output layer. In this case, the learning device 3000 may obtain a neural network model having at least one node with a third-a parameter set by renewing a parameter set on the basis of the output data and the label data related to the first tumor information included in the training data set.

In addition, the training data set may include label data for a medical image related to an area to which radiation is to be emitted (or an area to which radiation should not be emitted) to treat a second tumor of second tumor information. In this case, the learning device 3000 may input the training data set to the input layer of the artificial neural network model and obtain output data through the output layer. In this case, the learning device 3000 may obtain a neural network model having at least one node with a third-b parameter set by renewing the parameter set on the basis of the output data and the label data related to the second tumor information included in the training data set. The learning device 3000 according to an embodiment of the present application may renew the parameter set to obtain a neural network model with a different parameter set according to a difference in tumor information. Therefore, the medical image analysis device 2000 may achieve an advantageous effect of automatically dividing the medical image into a plurality of areas using the trained neural network model to be specific to the tumor information.

As another example, the learning device 3000 may renew a parameter set of at least one node of the feature layer included in the neural network model to calculate a fourth feature vector for segmentation of the medical image to be dependent on the tumor information on the basis of a training data set related to feature information related to radiation information.

Specifically, the training data set may include label data for a medical image related to an area to which radiation is to be emitted (or an area to which radiation should not be emitted) to treat a tumor by first radiation of first radiation information. In this case, the learning device 3000 may input the training data set to the input layer of the artificial neural network model and obtain output data through the output layer. In this case, the learning device 3000 may obtain a neural network model having at least one node with a fourth-a parameter set by renewing a parameter set on the basis of the output data and the label data related to the first radiation information included in the training data set. In addition, the training data set may include label data for a medical image related to an area to which radiation is to be emitted (or an area to which radiation should not be emitted) to treat a tumor by second radiation of second radiation information. In this case, the learning device 3000 may input the training data set to the input layer of the artificial neural network model and obtain output data through the output layer. In this case, the learning device 3000 may obtain a neural network model having at least one node with a fourth-b parameter set by renewing the parameter set on the basis of the output data and the label data related to the second radiation information included in the training data set. The learning device 3000 according to an embodiment of the present application may renew the parameter set to obtain a neural network model having a different parameter set according to a difference in radiation information. Thus, the medical image analysis device 2000 may achieve an advantageous effect of obtaining segmentation information dependent on the radiation information by using the trained neural network model.

On the other hand, as described above, in the obtaining of the training data set and the parameters of the artificial neural network model (S3100), an initial parameter set included in some nodes of the hidden layer for calculation of an initial feature vector, and particularly, at least one node of the feature layer, may be obtained.

In this case, in the renewing of the feature vector of the basis of the feature information of the training data set and the label data (S3200), the learning device 3000 according to an embodiment of the present application may renew the initial parameter set to obtain a neural network model having a final parameter set for segmentation of the medical image to be specific to the feature information on the basis of the obtained initial parameter set.

In the obtaining of the parameter set of the artificial neural network model (S3300), the learning device 3000 according to an embodiment of the present application may repeatedly perform the renewing of the parameter set of the neural network model as described above to obtain a neural network model having a parameter set for minimizing the difference between the label data included in the output data and the label data included in the training data set.

It has been described above in connection with operation S3200 of FIG. 10 that the parameter set or weight sets of the node of the neural network model obtained in operation S3100 may be fixed and the parameter set of the at least one node included in the feature layer may be renewed. However, the above description is merely an example and embodiments are not limited thereto, and the learning device 3000 may be implemented to renew a parameter set and weight sets of a node of the neural network model, which is included in the hidden layer other than the feature layer, together.

Referring back to FIG. 4, the segmentation process P2000 according to an embodiment of the present application may include a data obtaining process P2100 and a segmentation process P2200 using a trained neural network model.

The segmentation process P2000 may be implemented by the medical image analysis device 2000 according to an embodiment of the present application.

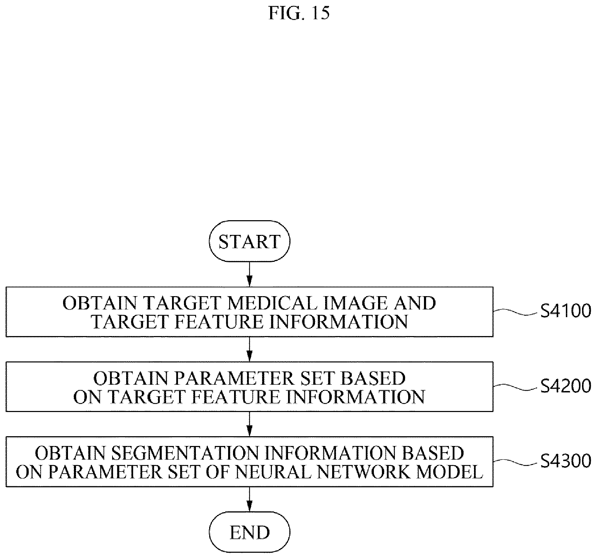

A segmentation operation of a medical image using a neural network model, which is performed by the medical image analysis device 2000 according to an embodiment of the present application, will be described with reference to FIG. 15 below. FIG. 15 is a flowchart of an image segmentation method using a neural network model, which is performed by the medical image analysis device 2000, according to an embodiment of the present application.

Referring to FIG. 15, the image segmentation method using a neural network model, which is performed by the medical image analysis device 2000, according to an embodiment of the present application may include obtaining a target medical image and target feature information (S4100), obtaining a parameter set on the basis of the target feature information (S4200), and obtaining segmentation information on the basis of a parameter set of a neural network model (S4300).

In the obtaining of the target medical image and the target feature information (S4100), the medical image analysis device 2000 may obtain a target medical image from the medical image obtaining device 1000.

In addition, the medical image analysis device 2000 may obtain target feature information to be used for segmentation of the target medical image from the medical image obtaining device 1000, an external device, or a user input.

In this case, the target feature information may be a basis on which a parameter set of a neural network model is obtained and replaced, as will be described below.

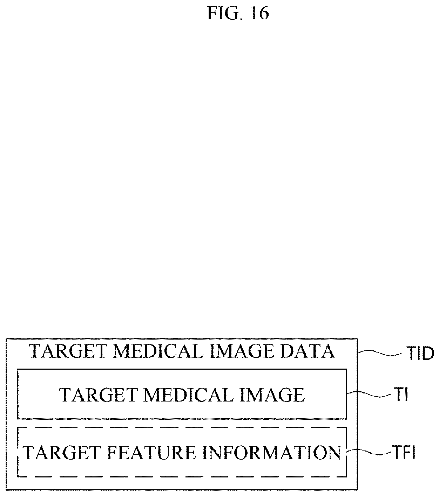

FIG. 16 will now be referred to. FIG. 16 is a diagram illustrating an example of a structure of a target medical image according to an embodiment of the present application.

For example, according to an embodiment of the present application, target medical image data TID obtained by the medical image analysis device 2000 may include information about a target medical image TI. For example, the information about the target medical image TI may be understood to include information related to coordinates, intensity, colors, and the like of pixels.

As another example, the target medical image data TID may include target feature information TFI. In this case, the target feature information TFI may be structured as metadata with respect to the obtained target medical image TI. For example, the target feature information TFI related to information about a patient who may be a subject included in the target medical image TI may be structured as metadata with respect to the target medical image TI.

On the other hand, the target feature information TFI may be obtained from an external device separately from the target medical image TI.

Alternatively, the target feature information TFI may be obtained from a user input through the input module 2400 of the medical image analysis device 2000 as described above. For example, when a user desires to segment the target medical image TI to reflect first operator information related to a first operator, target feature information related to first operator information may be input through the input module 2400, and the medical image analysis device 2000 may obtain an input corresponding to the first operator information to obtain feature information TFI.

Referring back to FIG. 4, the medical image analysis device 2000 may be implemented to input the target medical image data obtained in the data obtaining process P2100 to the input layer of the trained neural network model.

In this case, the medical image analysis device 2000 may obtain an artificial neural network model including a node with a parameter set obtained by the learning process P 1000 performed by the learning device 2000, and the obtained artificial neural network model may be used as a neural network model for segmenting the target medical image TI.

Therefore, in the obtaining of the parameter set of the neural network model on the basis of the target feature information (S4200), the medical image analysis device 2000 according to an embodiment of the present application may obtain a neural network model and/or a parameter set (or a weight set) of the neural network model.

For example, the learning device 3000 according to an embodiment of the present application may be implemented to obtain an artificial neural network model on the basis of the obtained target feature information.

For example, when the medical image analysis device 2000 obtains first target feature information, the medical image analysis device 2000 may be implemented to obtain a first artificial neural network model including a node with a first parameter set learned on the basis of feature information corresponding to the first target feature information. On the other hand, when the medical image analysis device 2000 obtains second target feature information, the medical image analysis device 2000 may be implemented to obtain a second artificial neural network model including a node with a second parameter set learned on the basis of feature information corresponding to the second target feature information.

For example, when the target feature information is related to feature information including first operator information, the medical image analysis device 2000 according to an embodiment of the present application may be implemented to obtain a neural network model including a node with a first-a parameter set obtained by renewing in relation to the first operator information. In this case, the neural network model including the node with the first-a parameter set may calculate a first-a feature vector for segmentation of a medical image to be specific to the first operator information. For example, when the target feature information obtained by the medical image analysis device 2000 corresponds to the first operator information, the medical image analysis device 2000 may obtain a parameter set renewed on the basis of a training data set including label-related data related to the first operator information and the medical image.

On the other hand, when the target feature information is related to feature information including second operator information, the medical image analysis device 2000 according to an embodiment of the present application may be implemented to obtain a neural network model including a node with a first-b parameter set learned in relation to the second operator information. In this case, the neural network model including the node with the first-b parameter set may calculate a first-b feature vector for segmentation of a medical image to be specific to the first operator information. For example, when the target feature information obtained by the medical image analysis device 2000 corresponds to the second operator information, the medical image analysis device 2000 may obtain a parameter set renewed on the basis of a training data set including label-related data related to the second operator information and the medical image.

As another example, when the target feature information is related to feature information including first patient information, the medical image analysis device 2000 according to an embodiment of the present application may be implemented to obtain a neural network model including a node with a second-a parameter set learned in relation to the first patient information. In this case, the neural network model including the node with the second-a parameter set may calculate a second-a feature vector for segmentation of a medical image to be specific to the first patient information. For example, when the first patient information indicating that a patient corresponds to a first age section or has an underlying disease is obtained as target feature information, the medical image analysis device 2000 may obtain a neural network model having a node with a parameter set learned on the basis of a training data set including label-related data about a medical image related to the feature information indicating that the patient corresponds to the first age section or has the underlying disease and the medical image.

On the other hand, when the target feature information is related to feature information including second patient information, the medical image analysis device 2000 according to an embodiment of the present application may be implemented to obtain a neural network model including a node with a second-b parameter set renewed in relation to the second patient information. In this case, the neural network model including the node with the second-b parameter set may calculate a second-b feature vector for segmentation of the medical image to be specific to the second patient information. For example, when the second patient information indicating that a patient corresponds to a second age section or does not have an underlying disease is obtained as target feature information, the medical image analysis device 2000 may obtain a neural network model having a node with a parameter set learned on the basis of a training data set including label-related data about a medical image related to the feature information indicating that the patient corresponds to the second age section or does not have the underlying disease and the medical image.

As another example, when the target feature information is related to feature information including first tumor information, the medical image analysis device 2000 according to an embodiment of the present application may be implemented to obtain a neural network model including a node with a third-a parameter set learned in relation to the first tumor information. In this case, the neural network model including the node with the third-a parameter set may calculate a third-a feature vector for segmentation of a medical image to be specific to the first tumor information. For example, when a first type of tumor information is obtained as target feature information, the medical image analysis device 2000 may obtain a neural network model including a node with a parameter set learned on the basis of a training data set including label-related data about medical image related feature information corresponding to the first type and the medical image.

On the other hand, when the target feature information is related to feature information including second tumor information, the medical image analysis device 2000 according to an embodiment of the present application may be implemented to obtain a neural network model including a node with a third-b parameter set renewed in relation to the second tumor information. In this case, the neural network model including the node with the third-b parameter set may calculate a third-b feature vector for segmentation of the medical image to be specific to the second tumor information. For example, when a second type of tumor information is obtained as target feature information, the medical image analysis device 2000 may obtain a neural network model including a node with a parameter set learned on the basis of a training data set including label-related data about medical image related feature information corresponding to the second type and the medical image.

In this case, the first type and the second type may be understood to include a size of a tumor, a degree of expression of the tumor, a shape of the tumor, or a position of the tumor.

As another example, when the target feature information is related to feature information including first radiation information, the medical image analysis device 2000 according to an embodiment of the present application may be implemented to obtain a neural network model including a node with a fourth-a parameter set learned in relation to the first radiation information. In this case, the neural network model including the node with the fourth-a parameter set may calculate a fourth-a feature vector for segmentation of the medical image to be specific to the first radiation information. For example, when information about a first type of radiation for treating a tumor at a first intensity or for a first period is obtained as target feature information, the medical image analysis device 2000 may obtain a neural network model including a node with a parameter set learned on the basis of a training data set including label data of a medical image divided into areas to which radiation is to be emitted to treat a tumor using the first type of radiation and the medical image.

On the other hand, when the target feature information is related to feature information including second radiation information, the medical image analysis device 2000 according to an embodiment of the present application may be implemented to obtain a neural network model including a node with a fourth-b parameter set renewed in relation to the second radiation information. In this case, the neural network model including the node with the fourth-b parameter set may calculate a fourth-b feature vector for segmentation of the medical image to be specific to the first radiation information. For example, when information about a second type of radiation for treating a tumor at a second intensity or for a second period is obtained as target feature information, the medical image analysis device 2000 may obtain a neural network model including a node with a parameter set learned on the basis of a training data set including label data of a medical image divided into areas to which radiation is to be emitted to treat a tumor using the second type of radiation and the medical image.

In other words, the medical image analysis device 2000 according to an embodiment of the present application may obtain the neural network model renewed in consideration of the target feature information and/or a parameter set of at least one node of the neural network model to segment a target medical image in consideration of the target feature information related to the operator information, the patient information, the tumor information, and/or the radiation information.

However, the above description is only an example and the target feature information may be configured to obtain at least one parameter set according to a combination of various feature information. For example, when the target feature information is related to the first operator information and the second tumor information, the medical image analysis device 2000 may be implemented to obtain the first-a parameter set and the second-b parameter set or obtain a new parameter set.

Referring back to FIG. 15, in the obtaining of the segmentation information on the basis of the parameter set of the neural network model (S4300), the medical image analysis device 2000 according to an embodiment of the present application may be implemented to segment the target medical image on the basis of the trained neural network model and a parameter set of some nodes of the neural network model.

The medical image analysis device 2000 according to an embodiment of the present application may be implemented to perform segmentation of the target medical image on the basis of the parameter set of the trained neural network model. Specifically, the medical image analysis device 2000 may be implemented to input the target medical image to an input layer of the trained neural network model and output a result of segmenting the target medical image through an output layer.

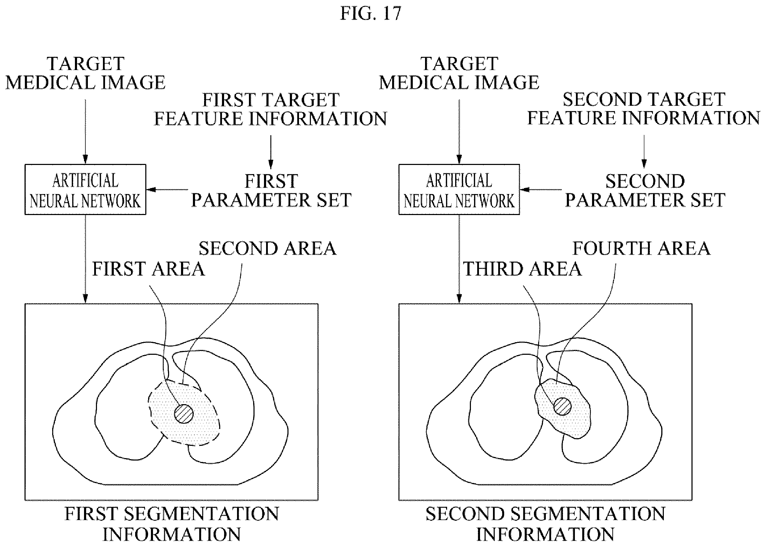

FIG. 17 will now be referred to. FIG. 17 is a schematic diagram illustrating segmenting a target medical image by the medical image analysis device 2000 according to an embodiment of the present application.

Referring to FIG. 17, the medical image analysis device 2000 according to an embodiment of the present application may obtain a target medical image, a trained artificial neural network, and target feature information.

For example, when the target feature information is related to first target feature information, the medical image analysis device 2000 according to an embodiment of the present application may obtain a first parameter set on the basis of the first object feature information.

In this case, the obtained first parameter set may correspond to a node of a feature layer constituting part of a hidden layer of the trained artificial neural network model.

The medical image analysis device 2000 may input the target medical image into an input layer of the artificial neural network model and obtain first segmentation information output through an output layer on the basis of the trained artificial neural network model including the first parameter set included in at least one node of the feature layer.

In this case, with the first segmentation information, a medical image may be divided into a plurality of areas to be specific to the first target feature information. For example, the first segmentation information may include a first area corresponding to a tumor and a second area related to a target area to which radiation is to be emitted while reflecting the first target feature information.

As another example, when the target feature information is related to second target feature information, the medical image analysis device 2000 according to an embodiment of the present application may obtain a second parameter set on the basis of the second object feature information.

In this case, the obtained second parameter set may correspond to a node of the feature layer constituting part of the hidden layer of the trained artificial neural network model.

The medical image analysis device 2000 may input the target medical image into the input layer of the artificial neural network model and obtain second segmentation information output through the output layer on the basis of the trained artificial neural network model including the second parameter set included in at least one node of the feature layer.

In this case, with the second segmentation information, a medical image may be divided into a plurality of areas to be specific to the second target feature information. For example, the second segmentation information may include a third area corresponding to a tumor and a fourth area related to a target area to which radiation is to be emitted while reflecting the second target feature information.

In this case, the second area included in the first segmentation information and the fourth area included in the second segmentation information may be obtained differently due to different target feature information. For example, the second area may be defined by a first boundary, but the fourth area may be defined by a second boundary different from the first boundary.

In other words, the medical image analysis device 2000 according to an embodiment of the present application may be implemented to divide an area to which radiation is to be emitted to be dependent on feature information. Because an area to which radiation is to be emitted may be segmented differently according to feature information, the medical image analysis device 2000 may achieve an advantageous effect of providing a user with treatment assistance information specific to the feature information on the basis of segmentation information.

Meanwhile, the medical image analysis device 2000 according to an embodiment of the present application may be implemented to use at least two trained neural network models in parallel to segment the target medical image on the basis of a combination of various target feature information.

The first neural network model and the second neural network model may be provided independently. The first neural network model and the second neural network model may share at least one layer. The first neural network model and the second neural network model may be provided to obtain different outputs on the basis of the target medical image.

For example, the medical image analysis device 2000 may include a first neural network model and a second neural network model that are provided in parallel with each other. The first neural network model may be a neural network model for segmenting the target medical image to obtain a tumor area. On the other hand, the second neural network model may be a model for segmenting the target medical image to obtain an area to which radiation is to be emitted or an area to which radiation should not be emitted.

For example, a plurality of areas of the target medical image, which are finally obtained by the first neural network model and the second neural network model provided in parallel, may include a tumor area, areas (e.g., areas GTV, CTV, and PTV) to which radiation is to be emitted, and/or an area OAR to which radiation should not be emitted.

In an embodiment, the first neural network model may obtain the tumor area. The first neural network model may obtain the tumor area independent of the feature information. The tumor area is an area of the medical image corresponding to a tumor and thus should be obtained in substantially the same manner regardless of the feature information. In this case, according to an embodiment of the present application, the tumor area is obtained by the first neural network model that does not include a parameter set related to the target feature information and thus may be segmented while not being substantially affected by a change in the target feature information. Accordingly, the tumor area may be obtained without being affected by the target feature information. To this end, the medical image analysis device 2000 may obtain, as the first neural network model, a trained neural network model that does not include a feature vector related to the target feature information.

Alternatively, the first neural network model may obtain a feature vector on the basis of a parameter set determined according to tumor type information and obtain a tumor area on the basis of the obtained feature vector to be dependent on at least a portion of the tumor type information. For example, when the tumor type information includes information indicating that the size of a tumor is greater than an average size, a parameter set renewed to calculate a feature vector reflecting a tendency of defining an area by an operator with respect to the tumor type information may be obtained and a tumor area may be obtained on the basis of the feature vector.

The second neural network model may obtain an area related to a treatment plan. The second neural network model may obtain an area related to a treatment plan to be dependent on at least a portion of the feature information. Areas obtained in relation to a treatment plan by the second neural network model (e.g., the areas GTV, CTV, and PTV to which radiation is to be emitted or the area OAR to which radiation should not be emitted) may be divided to be specific to the target feature information. To this end, the medical image analysis device 2000 may obtain, as the second neural network model, a neural network model including a feature vector with at least one node having a parameter set for calculation of a feature vector related to the target feature information. The areas GTV, CTV, and PTV to which radiation is to be emitted and/or the area OAR to which radiation should not be emitted are obtained by the second neural network model including at least one node having a parameter set renewed to calculate a feature vector and thus may be obtained to reflect a change in the target feature information.

However, the above description is only an example, and the medical image analysis device 2000 may be implemented by providing at least two trained neural network models in parallel but may be provided to achieve the above-described purposes by driving at least two trained neural network models.

The medical image analysis device 2000 may be implemented to overlay visual graphics on a plurality of areas, including a tumor area, an area to which radiation is to be emitted and/or an area to which radiation should not be emitted, and display resultant areas to a user through the output module 2500.

The segmentation information obtained by the medical image analysis device 2000 according to an embodiment of the present application may be a form of labeling corresponding to a plurality of areas, including a tumor area obtained from the target medical image and an area to which radiation is to be emitted. For example, segmentation information output through the output layer of the neural network model may be in the form of labeling data including a first label defining a first areas obtained from the target medical image and a second label defining a second area obtained from the target medical image.

Specifically, the medical image includes information related to the coordinates of a cell (e.g., a pixel or a voxel). The medical image analysis device 2000 may obtain probability information representing a possibility of corresponding to a plurality of labels assigned to each cell included in a medical image through a neural network. For example, with respect to a first cell, probability information indicating that a possibility of corresponding to a first label among the plurality of labels is a first probability and a possibility of corresponding to a second label among the plurality of labels is a second probability may be obtained.

In this case, the medical image analysis device 2000 may assign, as a label of each cell, a label with a highest probability among the plurality of labels for each cell. For example, when it is most probable that the first cell corresponds to the first label, the first cell may be assigned the first label.

Furthermore, the medical image analysis device 2000 may obtain area information on the basis of cells assigned the same label. For example, first area information may be obtained on the basis of information about a plurality of cells to which the first label is assigned. On the other hand, second area information may be obtained on the basis of information about a plurality of cells to which the second label different from the first label is assigned.

The medical image analysis device 2000 according to an embodiment of the present application may perform visual graphical processing appropriate to provide the segmentation information to a user.

For example, the segmentation information may be processed such that a first color is overlaid on a first area of a target image and a second color is overlaid on a second area of the target image on the basis of the first label.

As another example, the medical image analysis device 2000 may process the segmentation information such that a first boundary related to a first area obtained based on the first label and a second boundary related to a second area obtained based on the second label are displayed on a target image.

Therefore, the first area and the second area may be more easily distinguished from each other. However, the above description is only an example and an output result may be configured as any form for distinguishing between the first area and the second area.

Alternatively, the segmentation information obtained by the medical image analysis device 2000 according to an embodiment of the present application may include information related to the medical image a plurality of areas including a tumor area and an area to which radiation is to be emitted.

Alternatively, the segmentation information may further include information related to an area to which radiation should not be emitted.

For example, the segmentation information may include information related to a plurality of areas, including an area GTV corresponding to an organ in which a tumor is located, an area CTV related to a margin when a patient's movement is taken into consideration during a radiation treatment, an area PTV related to a margin when fine motions of the organ are taken into consideration when a tumor treatment is conducted a plurality of times, and/or an area OAR to which radiation should not be emitted. For example, the area GTV corresponding to an organ in which a tumor is located, the area CTV related to a margin when a patient's movement is taken into consideration during a radiation treatment, an area PTV related to a margin when fine motions of the organ are taken into consideration when a tumor treatment is conducted a plurality of times, and/or an area OAR to which radiation should not be emitted may be obtained differently according to the target feature information. Specifically, the medical image analysis device 2000 according to an embodiment of the present application may segment the target medical image using a neural network model trained to automatically divide the medical image into a plurality of areas in consideration of feature information. Thus, the medical image analysis device 2000 according to an embodiment of the present application may be implemented to automatically divide a plurality of areas in consideration of the target feature information. In this case, the plurality of areas may be defined differently according to the target feature information.

For example, when the target feature information is related to first operator information including history information of a tumor treatment conducted by defining an area to which radiation is to be emitted to be wider than an average area definition range, the medical image analysis device 2000 may be implemented to divide areas (e.g., the areas GTV, CTV, or PTV) to which radiation is to be emitted to be wider than the average area definition range using a neural network model trained to reflect the first operator information. On the other hand, when the target feature information is related to second operator information including history information of a tumor treatment conducted by defining an area to which radiation is to be emitted to be narrower than the average area definition range, the medical image analysis device 2000 may be implemented to divide areas (e.g., the areas GTV, CTV, and PTV) to which radiation is to be emitted to be narrower than the average area definition range using a neural network model trained to reflect the second operator information.

For example, when the target feature information is related to first operator information including history information of a tumor treatment conducted by defining an area to which radiation should not be emitted to be wider than the average area definition range, the medical image analysis device 2000 may be implemented to divide an area (e.g., the area OAR) to which radiation should not be emitted to be wider than the average area definition range using a neural network model trained to be specific to the first operator information. For example, when the target feature information is related to second operator information including history information of a tumor treatment conducted by defining an area to which radiation should not be emitted to be narrower than the average area definition range, the medical image analysis device 2000 may be implemented to divide an area (e.g., the area OAR) to which radiation should not be emitted to be narrower than the average area definition range using a neural network model trained to be specific to the second operator information.

However, the above description is only an example, and the medical image analysis device 2000 may be implemented to divide an area to which radiation is to be emitted or an area to which radiation should not be emitted on the basis of a neural network model and/or a parameter set trained in relation to patient information, tumor information, and/or radiation information other than the operator information.

Thus, the segmentation information may be used as treatment assistance information in relation to a medical procedure on or treatment of a tumor.

In addition, the medical image analysis device 2000 according to an embodiment of the present application may obtain the segmentation information by segmenting the target medical image on the basis of the neural network model obtained based on the target feature information. Therefore, the medical image analysis device 2000 according to an embodiment of the present application may provide a user with treatment assistance information reflecting the target feature information.

For example, when the medical image analysis device 2000 obtains target feature information corresponding to first operator information, the medical image analysis device 2000 may obtain segmentation information on the basis of a neural network model including a node with a first-a parameter set related to the first operator information. For example, when the first operator information includes information related to an aggressive medical procedure history (which shows, for example, a tendency of treatment equal to or greater than an average treatment range) to treat a tumor, the segmentation information obtained by the medical image analysis device 2000 may include a plurality of areas, including a tumor area and an area to which radiation is to be emitted, and the area to which radiation is to be emitted may be defined by a first-a boundary.

As another example, when the medical image analysis device 2000 obtains target feature information corresponding to second operator information, the medical image analysis device 2000 may obtain segmentation information on the basis of a neural network model including a node with a first-b parameter set related to the second operator information. For example, when the second operator information includes information related to a conservative medical procedure history (which shows, for example, a tendency of treatment less than the average treatment range) to treat a tumor, the segmentation information obtained by the medical image analysis device 2000 may include a plurality of areas, including a tumor area and an area to which radiation is to be emitted, and the area to which radiation is to be emitted may be defined by a first-b boundary.

In this case, the first-a boundary and the first-b boundary may be external boundaries of the area to which radiation is to be emitted.

In this case, the first-a boundary reflecting the first operator information may be different from the first-b boundary reflecting the second operator information. Thus, a size of the area defined by the first-a boundary reflecting the first operator information may be different from that of the area defined by the first-b boundary reflecting the second operator information. In this case, the size of the area defined by the first-a boundary is calculated by a neural network model trained to be specific to the aggressive medical procedure history (which shows, for example, the tendency of treatment equal to or greater than the average treatment range) to treat a tumor and thus may be greater than that of the area defined by the second-a boundary.

For example, when the medical image analysis device 2000 obtains target feature information corresponding to first patient information, the medical image analysis device 2000 may obtain segmentation information on the basis of a neural network model including a node with a second-a parameter set related to the first patient information. For example, when the first patient information includes information indicating that a patient's age belongs to a first age section or that a patient does not have an underlying disease, the segmentation information obtained by the medical image analysis device 2000 may include a plurality of areas, including a tumor area and an area to which radiation is to be emitted, and the area to which radiation is to be emitted may be defined by a second-a boundary.

As another example, when the medical image analysis device 2000 obtains target feature information corresponding to second patient information, the medical image analysis device 2000 may obtain segmentation information on the basis of a neural network model including a node with a second-b parameter set related to the second patient information. For example, when the second patient information includes information indicating that a patient's age belongs to a second age section including age values greater than those of the first age section or that a patient has an underlying disease, the segmentation information obtained by the medical image analysis device 2000 may include a plurality of areas, including a tumor area and an area to which radiation is to be emitted, and the area to which radiation is to be emitted may be defined by a second-b boundary.

In this case, the second-a boundary and the second-b boundary may be external boundaries of the area to which radiation is to be emitted.

In this case, the second-a boundary reflecting the first patient information may be different from the second-b boundary reflecting the second patient information. Thus, a size of the area defined by the second-a boundary reflecting the first patient information may be different from that of the area defined by the second-b boundary reflecting the second patient information. In this case, the size of the area defined by the second-a boundary is calculated by a neural network model trained to obtain an area to which radiation is to be emitted within a range wider than an average treatment range in consideration of information about the patient and thus may be greater than the area of the area defined by the second-b boundary.

For example, when the medical image analysis device 2000 obtains target feature information corresponding to first tumor information, the medical image analysis device 2000 may obtain segmentation information on the basis of a neural network model including a node with a third-a parameter set related to the first tumor information. For example, when the first tumor information includes information indicating a tumor having a relatively high degree of expression (e.g., Grade 3 or higher) or having a size greater than an average size, the segmentation information obtained by the medical image analysis device 2000 may include a plurality of areas, including a tumor area and an area to which radiation is to be emitted, and the area to which radiation is to be emitted may be defined by a third-a boundary.

As another example, when the medical image analysis device 2000 obtains target feature information corresponding to second tumor information, the medical image analysis device 2000 may obtain segmentation information on the basis of a neural network model including a node with a third-b parameter set related to the second tumor information. For example, when the second tumor information includes information indicating a tumor having a relatively low degree of expression (e.g., less than Grade 3) or having a size less than the average size, the segmentation information obtained by the medical image analysis device 2000 may include a plurality of areas, including a tumor area and an area to which radiation is to be emitted, and the area to which radiation is to be emitted may be defined by a third-b boundary.

In this case, the third-a boundary and the third-b boundary may be external boundaries of the area to which radiation is to be emitted.

In this case, the third-a boundary obtained on the basis of the first tumor information may be different from the third-b boundary obtained on the basis of the second tumor information. Thus, a size of the area defined by the third-a boundary obtained on the basis of the first tumor information may be different from that of the area defined by the third-b boundary obtained on the basis of the second tumor information. In this case, the size of the area defined by the third-a boundary may be calculated by a neural network model trained to obtain an area to which radiation is to be emitted within a range wider than an average treatment range in consideration of the tumor information and thus may be greater than the size of the area defined by the third-b boundary.

For example, when the medical image analysis device 2000 obtains target feature information corresponding to first radiation information, the medical image analysis device 2000 may obtain segmentation information on the basis of a neural network model including a node with a fourth-a parameter set related to the first radiation information. For example, when the first radiation information indicates that radiation to be emitted is of a first type (e.g., a first manufacturer, radiation of a first wavelength band, etc.), the segmentation information obtained by the medical image analysis device 2000 may include a plurality of areas, including a tumor area and an area to which radiation is to be emitted, and the area to which radiation is to be emitted may be defined by a fourth-a boundary.

As another example, when the medical image analysis device 2000 obtains target feature information corresponding to second radiation information, the medical image analysis device 2000 may obtain segmentation information on the basis of a neural network model including a node with a fourth-b parameter set related to the second radiation information. For example, when the second radiation information indicates that radiation to be emitted is of a second type (e.g., a second manufacturer, radiation of a second wavelength band, etc.), the segmentation information obtained by the medical image analysis device 2000 may include a plurality of areas, including a tumor area and an area to which radiation is to be emitted, and the area to which radiation is to be emitted may be defined by a fourth-b boundary.

In this case, the fourth-a boundary and the fourth-b boundary may be external boundaries of the area to which radiation is to be emitted.

In this case, the fourth-a boundary obtained on the basis of the first radiation information may be different from the fourth-b boundary obtained on the basis of the second radiation information. Thus, a size of the area defined by the fourth-a boundary obtained on the basis of the first radiation information may be different from that of the area defined by the fourth-b boundary obtained on the basis of the second radiation information.

As described above, the medical image analysis device 2000 according to an embodiment of the present application may be implemented to obtain an area to which radiation is to be emitted to be specific to the target feature information.

Thus, the segmentation information obtained by the medical image analysis device 2000 according to an embodiment of the present application may be used as treatment assistance information for obtaining an area to which radiation is to be emitted in relation to a tumor. In particular, because an area to which radiation is to be emitted may be automatically defined in consideration of feature information, the medical image analysis device 2000 may provide a user with accurate treatment assistance information while reflecting the feature information in relation to an elaborate tumor treatment.

In addition, a user may input feature information through an input module to obtain treatment assistance information while reflecting the feature information, and the medical image analysis device 2000 may obtain the treatment assistance information to reflect the feature information on the basis of a user input. Thus, the treatment assistance information may be obtained on the basis of the feature information and thus the feature information may be referred to as treatment plan information.

Meanwhile, the medical image analysis device 2000 according to an embodiment of the present application may receive a user input instructing to emit radiation on the basis of the segmentation information. For example, the medical image analysis device 2000 may obtain a user input instructing to start to emit radiation for treating tumor through the input module 2400, based on the segmentation information.

Alternatively, the medical image analysis device 2000 according to an embodiment of the present application may start to emit radiation according to the segmentation information, based on a user input instructing to start to emit radiation. For example, the medical image analysis device 2000 may transmit, to an external radiation emission device, an instruction to start emitting radiation on the basis of the segmentation information and a user input.

Alternatively, the medical image analysis device 2000 according to an embodiment of the present application may further include a radiation outputter. In this case, the medical image analysis device 2000 may start to emit radiation on the basis of the segmentation information and the user input.

Meanwhile, in the segmentation process P2000, the medical image analysis device 2000 according to an embodiment of the present application may be implemented to renew or update the parameter set of the trained artificial neural network model. For example, in the segmentation process P2000, the medical image analysis device 2000 may renew or update a parameter set included in at least one node of the feature layer constituting a layer of the hidden layer. Alternatively, in the segmentation process P2000, the medical image analysis device 2000 may renew or update a parameter set included in at least one node included in the hidden layer other than the feature layer.

For example, the medical image analysis device 2000 may be implemented to modify segmentation information, which is obtained by segmenting the target medical image, manually or using software through the segmentation process P2200 using the trained neural network model. In this case, the medical image analysis device 2000 may be implemented to renew or update the artificial neural network model by modifying a weight set or a parameter set of at least one node of the neural network model on the basis of the modified segmentation information.

For example, the medical image analysis device 2000 according to an embodiment of the present application may receive a user input related to modifying at least part of the obtained segmentation information and receive a user input instructing to start to emit radiation on the basis of the modified segmentation information. For example, a user may input an instruction to modify the area to which radiation is to be emitted or the area to which radiation should not be emitted, which is indicated in the segmentation information.

In this case, the medical image analysis device 2000 may be implemented to renew or update the parameter set of the neural network model on the basis of the difference between the segmentation information obtained through the output layer of the neural network model and the modified segmentation information.

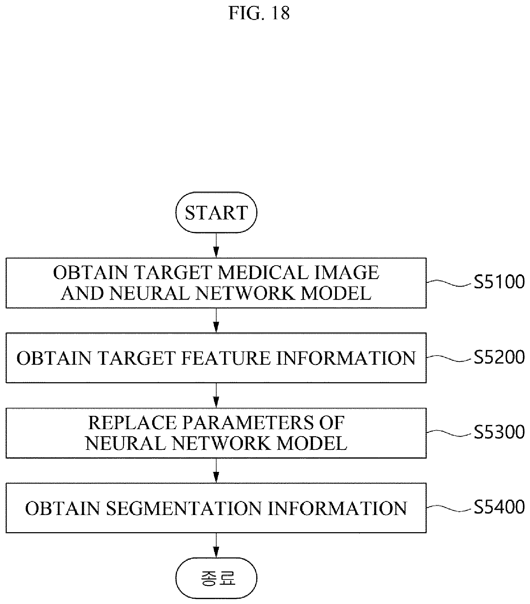

Segmenting a target medical image according to an embodiment of the present application will be described with reference to FIG. 18 below. The operations of the learning device 3000 and the medical image analysis device 2000 for segmentation described above with reference to FIGS. 4 to 17 may also apply and redundant parts will be briefly described below.

FIG. 18 is a flowchart of a segmentation method of a target medical image according to an embodiment of the present application.

Referring to FIG. 18, a segmentation operation of a target medical image according to an embodiment of the present application may include obtaining a target medical image and a neural network model (S5100), obtaining target feature information (S5200), replacing parameters of a node of the neural network model (S5300), and obtaining segmentation information (S5400).

In the obtaining of the target medical image and the neural network model (S5100), the medical image analysis device 2000 according to an embodiment of the present application may obtain a target medical image. In this case, the medical image analysis device 2000 may obtain a target medical image to be analyzed from the medical image obtaining device 1000 or an external device (e.g., a server).

In addition, in the obtaining of the target medical image and the neural network model (S5100), the medical image analysis device 2000 according to an embodiment of the present application may obtain a trained neural network model. In this case, the trained neural network model may include at least one node including a parameter set obtained to calculate a feature vector related to feature information.

Alternatively, in the obtaining of the target medical image and the neural network model (S5100), the medical image analysis device 2000 according to an embodiment of the present application may be implemented to obtain a parameter set of at least one neural network model related to feature information. Specifically, various feature information may be provided. For example, the feature information may be related to operator information, patient information, tumor information, radiation information, or a combination thereof, and the medical image analysis device 2000 according to an embodiment of the present application may obtain a plurality of parameter sets related to various feature information.

For example, in the obtaining of the target medical image and the neural network model (S5100), the medical image analysis device 2000 according to an embodiment of the present application may obtain a parameter set of at least one neural network model related to the operator information.

For example, the medical image analysis device 2000 according to an embodiment of the present application may obtain a parameter set of at least one neural network model related to the patient information.

For example, the medical image analysis device 2000 according to an embodiment of the present application may obtain a parameter set of at least one neural network model related to the tumor information.

For example, the medical image analysis device 2000 according to an embodiment of the present application may obtain a parameter set of at least one neural network model related to the radiation information.

In obtaining of the target feature information (S5200), the medical image analysis device 2000 according to an embodiment of the present application may obtain target feature information related to the target medical image. In this case, the target feature information may be treatment plan information on the basis of which target medical image may be segmented to obtain treatment assistance information.

In this case, the medical image analysis device 2000 according to an embodiment of the present application may obtain target feature information from a user input through the input module 2400.

Alternatively, the medical image analysis device 2000 according to an embodiment of the present application may obtain the target feature information from an external device (e.g., a server).

Alternatively, the medical image analysis device 2000 according to an embodiment of the present application may obtain the target feature information by obtaining metadata with respect to the target medical image.

The medical image analysis device 2000 according to an embodiment of the present application may be implemented to segment the target medical image on the basis of the obtained target feature information.

Specifically, the medical image analysis device 2000 may be implemented to replace the parameter set of the trained neural network model on the basis of the target feature information (S5300).

More specifically, the medical image analysis device 2000 may be implemented to select a parameter set related to the target feature information from among a plurality of parameter sets related to the feature information.

For example, the medical image analysis device 2000 may obtain a neural network model including at least one parameter set related to the feature information. In this case, the medical image analysis device 2000 may be implemented to select a parameter set related to the target feature information from among the at least one parameter set.

For example, when the medical image analysis device 2000 obtains the operator information as the target feature information, the medical image analysis device 2000 may be implemented to select a first parameter set related to the operator information from among at least one parameter set.

In this case, the first parameter set may be obtained by learning on the basis of feature information related to operator information included in a training data set corresponding to the target feature information, and a neural network model including a node having the first parameter set may segment the target medical image to be specific to the operator information. Accordingly, segmentation information or treatment assistance information reflecting an operator's area definition tendency may be obtained.

For example, when the first parameter set is obtained on the basis of operator information including treatment history information defining an area to which radiation is to be emitted to be wider than an average treatment range, the neural network model including at least one node having the first parameter set may divide an area of the target medical image to which radiation is to be emitted to be wider than the average treatment range.

For example, when the medical image analysis device 2000 obtains the patient information as the target feature information, the medical image analysis device 2000 may be implemented to select a second parameter set related to the patient information from among at least one parameter set.

In this case, the second parameter set may be obtained by learning on the basis of feature information related to patient information included in the training data set corresponding to the target feature information, and a neural network model including a node having the second parameter set may segment the target medical image to be specific to the patient information. Accordingly, segmentation information or treatment assistance information reflecting an operator's area definition tendency that varies according to information about a patient may be obtained.

For example, when the second parameter set is obtained by learning to define an area to which radiation is to be emitted to be wider than an average treatment range on the basis of patient information indicating that the patient belongs to a first age section or does not have an underlying disease, a neural network model including at least one node having the second parameter set may divide an area of the target medical image to which radiation is to be emitted to be wider than the average treatment range.

For example, when the medical image analysis device 2000 obtains the tumor information as the target feature information, the medical image analysis device 2000 may be implemented to select a third parameter set related to the tumor information from among at least one parameter set.

In this case, the third parameter set may be obtained on the basis of feature information related to tumor information included in the training data set corresponding to the target feature information, and a neural network model including a node having the third parameter set may segment the target medical image to be specific to the tumor information. Accordingly, segmentation information or treatment assistance information reflecting an operator's area definition tendency that varies according to the tumor information (e.g., a type of tumor, etc.) may be obtained.

For example, when the third parameter set is obtained by learning to define an area to which radiation is to be emitted to be narrower than an average treatment range on the basis of tumor information indicating a first type (e.g., a tumor having a size less than an average size or having a degree of expression less than Grade 3), a neural network model including at least one node having the third parameter set may divide an area of the target medical image to which radiation is to be emitted to be narrower than the average treatment range.

For example, when the medical image analysis device 2000 obtains the radiation information as the target feature information, the medical image analysis device 2000 may be implemented to select a fourth parameter set related to the radiation information from among at least one parameter set.

In this case, the fourth parameter set may be obtained on the basis of feature information related to radiation information included in the training data set corresponding to the target feature information, and a neural network model including a node having the fourth parameter set may segment the target medical image to be specific to the radiation information. Accordingly, segmentation information or treatment assistance information reflecting an operator's area definition tendency that varies according to the radiation information (e.g., the manufacturer, type, wavelength band, etc. of radiation) may be obtained.

For example, when the fourth parameter set is obtained by learning to define an area to which radiation is to be emitted to be narrower than an average treatment range on the basis of radiation information indicating that radiation to be used to conduct a medical procedure to treat a tumor has a first wavelength band or is generated by a radiation generator manufactured by a first manufacturer, a neural network model including at least one node having the fourth parameter set may divide an area of the target medical image to which radiation is to be emitted to be narrower than the average treatment range.

In addition, the medical image analysis device 2000 may be implemented to replace the parameter set of the trained neural network model with a selected parameter set.

For example, the medical image analysis device 2000 may be implemented to replace a parameter set (or a weight set) of some nodes of the trained neural network model with a parameter set obtained on the basis of the target feature information.

Specifically, the medical image analysis device 2000 may be implemented to replace some parameters of a node of the neural network model obtained in operation S5100 with a parameter set selected on the basis of the target feature information.

In this case, the resultant parameter set may be a feature parameter set included in at least one feature node included in a feature layer. For example, in the learning process P1000, for segmentation of a medical image to be specific to feature information, a feature parameter set related to a feature layer located in a bottleneck layer (layer between an encoder and a decoder) may be renewed. In this case, in the segmentation process P2000, the medical image analysis device 2000 may be implemented to replace a parameter set of a node corresponding to a feature node by selecting a parameter set corresponding to the feature parameter set of the feature node renewed in the learning process P1000.

However, the above description is only an example, and the medical image analysis device 2000 may be implemented to replace a parameter set of a node corresponding to a common node by appropriately selecting a parameter set of the common node other than the feature node so as to segment a medical image to reflect the feature information.

For example, when the target feature information is related to first feature information, the medical image analysis device 2000 may select a first parameter set related to the first feature information from among a plurality of parameter sets and replace the first parameter set with a parameter set of some nodes (e.g., at least one node included in a feature layer) of the neural network model, thereby configuring a neural network model for segmentation of the target medical image to be specific to the first feature information.

As described above, the target feature information (or the first feature information) may be related to operator information, patient information, tumor information and/or radiation information, and the medical image analysis device 2000 according to an embodiment of the present application may select and obtain a different parameter set according to the target feature information and replace a parameter set of some nodes of the neural network model with a parameter set when the target feature information is taken into consideration.

Thus, the medical image analysis device 2000 according to an embodiment of the present application may segment the target medical image into a plurality of areas to be specific to the target feature information. In other words, segmentation information or treatment assistance information reflecting an operator's area definition tendency may be obtained. Alternatively, segmentation information or treatment assistance information reflecting an operator's area definition tendency that varies according to patient information, tumor information and/or radiation information may be obtained.

However, the above description is only an example and a parameter set of a trained neural network model may be replaced according to various combinations of the operator information, the patient information, the tumor information and/or the radiation information.

Alternatively, the parameter set of the trained neural network model may be replaced on the basis of appropriate information other than the operator information, the patient information, the tumor information and/or the radiation information.

In the obtaining of the segmentation information (S5400), the medical image analysis device 2000 according to one embodiment of the present application may obtain segmentation information related to the target medical image using the neural network model including the resultant parameter set.

For example, the medical image analysis device 2000 may obtain segmentation information related to the target medical image on the basis of the neural network model including a node having the resultant parameter set reflecting the operator information.

For example, when the target feature information is related to first operator information, the medical image analysis device 2000 may select a first-1 parameter set learned in consideration of the first operator information from among a plurality of parameter sets and replace a parameter set of at least one node (e.g., at least one node included in the feature layer) of the neural network model with the first-1 parameter set.

In this case, the medical image analysis device 2000 may segment the target medical image on the basis of the neural network model including the node in which the parameter set is replaced with the first-1 parameter set, thereby obtaining first segmentation information specific to the first operator information.

On the other hand, when the target feature information is related to second operator information, the medical image analysis device 2000 may select a first-2 parameter set learned in consideration of the second operator information from among the plurality of parameter sets and replace a parameter set of at least one node (e.g., at least one node included in the feature layer) of the neural network model with the first-2 parameter set.

In this case, the medical image analysis device 2000 may segment the target medical image on the basis of the neural network model including the node having the first-2 parameter set, thereby obtaining second segmentation information specific to the second operator information.

As another example, when the target feature information is related to first patient information, the medical image analysis device 2000 may select a second-1 parameter set learned in consideration of the first patient information from among the plurality of parameter sets and replace a parameter set of at least one node (e.g., at least one node included in the feature layer) of the neural network model with the second-1 parameter set.

In this case, the medical image analysis device 2000 may segment the target medical image on the basis of the neural network model including the node having the second-1 parameter set, thereby obtaining first segmentation information reflecting an operator's area definition tendency with respect to the first patient information.

On the other hand, when the target feature information is related to second patient information, the medical image analysis device 2000 may select a second-2 parameter set learned in consideration of the second patient information from among the plurality of parameter sets and replace a parameter set of some nodes (e.g., at least one node included in the feature layer) of the neural network model with the second-2 parameter set.

In this case, the medical image analysis device 2000 may segment the target medical image on the basis of the neural network model including the node having the second-2 parameter set, thereby obtaining second segmentation information reflecting an operator's area definition tendency with respect to the second patient information.

As another example, when the target feature information is related to first tumor information, the medical image analysis device 2000 may select a third-1 parameter set learned in consideration of the first tumor information from among the plurality of parameter sets and replace a parameter set of some nodes (e.g., at least one node included in the feature layer) of the neural network model with the third-1 parameter set.

In this case, the medical image analysis device 2000 may segment the target medical image on the basis of the neural network model including the node having the third-1 parameter set, thereby obtaining first segmentation information reflecting an operator's area definition tendency with respect to the first tumor information.

On the other hand, when the target feature information is related to second tumor information, the medical image analysis device 2000 may select a third-2 parameter set learned in consideration of the second tumor information from among the plurality of parameter sets and replace a parameter set of some nodes (e.g., at least one node included in the feature layer) of the neural network model with the third-2 parameter set.

In this case, the medical image analysis device 2000 may segment the target medical image on the basis of the neural network model including the node having the third-2 parameter set, thereby obtaining second segmentation information reflecting an operator's area definition tendency with respect to the second tumor information.

As another example, when the target feature information is related to first radiation information, the medical image analysis device 2000 may select a fourth-1 parameter set learned in consideration of the first radiation information from among the plurality of parameter sets and replace a parameter set of some nodes (e.g., at least one node included in the feature layer) of the neural network model with the fourth-1 parameter set.

In this case, the medical image analysis device 2000 may segment the target medical image on the basis of the neural network model including the node having the fourth-1 parameter set, thereby obtaining first segmentation information reflecting an operator's area definition tendency with respect to the first radiation information.

On the other hand, when the target feature information is related to second radiation information, the medical image analysis device 2000 may select a fourth-2 parameter set learned in consideration of the second radiation information from among the plurality of parameter sets and replace a parameter set of some nodes (e.g., at least one node included in the feature layer) of the neural network model with the fourth-2 parameter set.

In this case, the medical image analysis device 2000 may segment the target medical image on the basis of the neural network model including the node having the fourth-2 parameter set, thereby obtaining second segmentation information reflecting an operator's area definition tendency with respect to the second radiation information.

In this case, at least some information included in the first segmentation information and at least some information included in the second segmentation information may be different from each other.

Specifically, both the first segmentation information and the second segmentation information may include a first area related to a tumor area and a second area to which radiation is to be emitted (e.g., areas GTV, CTV, and PTV).

In this case, the second area of the first segmentation information may be obtained by a neural network model including the first-1 parameter set obtained from the training data set including the first operator information indicating a tendency of defining an area to which radiation is to be emitted (e.g., the areas GTV, CTV, and PTV) to be relatively wide. In this case, the second area of the first segmentation information may be defined by a first boundary which is relatively wide. On the other hand, the second area of the second segmentation information may be obtained by a neural network model including the first-2 parameter set obtained from the training data set including the second operator information indicating a tendency of defining an area to which radiation is to be emitted (e.g., the areas GTV, CTV, and PTV) to be relatively narrow and may be defined by a second boundary, which is narrower than the first boundary, in this case.

Alternatively, the second area of the first segmentation information may be obtained by a neural network model including the second-1 parameter set obtained from the training data set including label data defining an area to which radiation is to be emitted (e.g., the areas GTV, CTV, and PTV) to be relatively wide on the basis of the first patient information including age information belonging to a relatively low age section. In this case, because the neural network model including the second-1 parameter set may segment the target medical image to reflect the age information belonging to the relatively low age section, the second area of the first segmentation information may be defined by the first boundary which is relatively wide. Alternatively, the second area of the second segmentation information may be obtained by a neural network model including the second-2 parameter set obtained from the training data set including label data defining an area to which radiation is to be emitted (e.g., the areas GTV, CTV, and PTV) to be relatively narrow on the basis of the second patient information indicating an underlying disease. In this case, because the neural network model including the second-2 parameter set may segment the target medical image to reflect the information indicating the underlying disease, the second area of the second segmentation information may be defined by the second boundary which is narrower than the first boundary.

Alternatively, the second area of the first segmentation information may be obtained by a neural network model including the third-1 parameter set obtained from the training data set including label data defining an area to which radiation is to be emitted (e.g., the areas GTV, CTV, and PTV) to be relatively wide on the basis of the first tumor information including information related to a type of tumor, which indicates that a size of the tumor is greater than or equal to a certain size, or a degree of expression of the tumor is greater than or equal to a certain degree. In this case, because the neural network model including the third-1 parameter set may segment the target medical image in consideration of the type of the tumor, the second area of the first segmentation information may be defined by the first boundary which is relatively wide. Alternatively, the second area of the second segmentation information may be obtained by a neural network model including the third-2 parameter set obtained from the training data set including label data defining an area to which radiation is to be emitted (e.g., the areas GTV, CTV, and PTV) to be relatively narrow on the basis of the second tumor information indicating that the tumor is located adjacent to a neighboring organ. In this case, the neural network model including the third-2 parameter set may segment the target medical image by reflecting positional information of the tumor indicating that the tumor is located adjacent to the neighboring organ. Accordingly, the second area of the second segmentation information may be defined by the second boundary which is narrower than the first boundary.

Alternatively, the second area of the first segmentation information may be obtained by a neural network model including the third-1 parameter set obtained from the training data set including label data defining an area to which radiation is to be emitted (e.g., the areas GTV, CTV, and PTV) to be relatively wide on the basis of the first tumor information including information related to a type of tumor, which indicates that a size of the tumor is greater than or equal to a certain size or a degree of expression of the tumor is greater than or equal to a certain degree. In this case, because the neural network model including the third-1 parameter set may segment the target medical image in consideration of the type of the tumor, the second area of the first segmentation information may be defined by the first boundary which is relatively wide. Alternatively, the second area of the second segmentation information may be obtained by a neural network model including the third-2 parameter set obtained from the training data set including label data defining an area to which radiation is to be emitted (e.g., the areas GTV, CTV, and PTV) to be relatively narrow on the basis of the second tumor information indicating that the tumor is located adjacent to a neighboring organ. In this case, the neural network model including the third-2 parameter set may segment the target medical image by reflecting positional information of the tumor indicating that the tumor is located adjacent to the neighboring organ. Accordingly, the second area of the second segmentation information may be defined by the second boundary which is narrower than the first boundary.

Alternatively, the second area of the first segmentation information may be obtained from a neural network model including the fourth-1 parameter set obtained from the training data set. In this case, the fourth-1 parameter set may be obtained from a renewed parameter set of the training data set including label data defining an area to which radiation is to be emitted (e.g., the areas GTV, CTV, and PTV) to be relatively wide on the basis of the first radiation information including information indicating that the intensity of radiation is lower than a certain intensity. In this case, because the neural network model including the fourth-1 parameter set may segment the target medical image in consideration of the type, intensity, etc. of the radiation, the second area of the first segmentation information may be defined by the first boundary which is relatively wide. On the other hand, the second area of the second segmentation information may be obtained from a neural network model including the fourth-2 parameter set obtained from the training data set. In this case, the fourth-2 parameter set may be obtained from a renewed parameter set of the training data set including label data defining an area to which radiation is to be emitted (e.g., the areas GTV, CTV, and PTV) to be relatively narrow on the basis of the second radiation information indicating that the intensity of radiation is higher than a certain intensity or the radiation is relatively dangerous to the body. In this case, because the neural network model including the fourth-2 parameter set may segment the target medical image in consideration of the intensity, degree of risk, etc. of the radiation, the second area of the second segmentation information may be defined by the second boundary which is relatively narrow.

Therefore, the segmentation information of the target medical image, which is generated by the medical image analysis device 2000 according to an embodiment of the present application, may provide an area, to which radiation is to be emitted, differently according to the operator information, the patient information, the tumor information and/or the radiation information and thus may be used as treatment assistance information when the tumor is treated.

On the other hand, a boundary of the first area related to the tumor area may be calculated differently according to a replaced parameter set. However, according to an embodiment, a boundary of the first area of the first segmentation information and a boundary of the first area of the second segmentation information may be substantially the same regardless of the replaced parameter set.

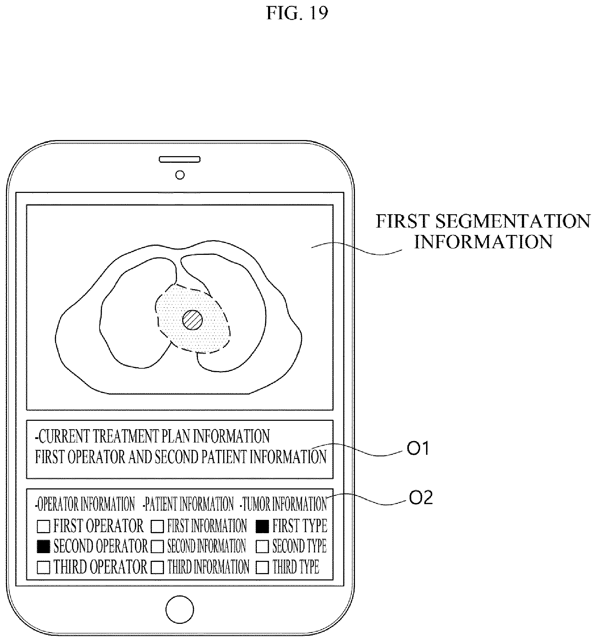

A user interface related to a result of analyzing a target medical image according to an embodiment of the present application will be described with reference to FIGS. 19 to 24 below.

FIG. 19 illustrates an example of a user interface related to a result of analyzing a target medical image according to an embodiment of the present application.

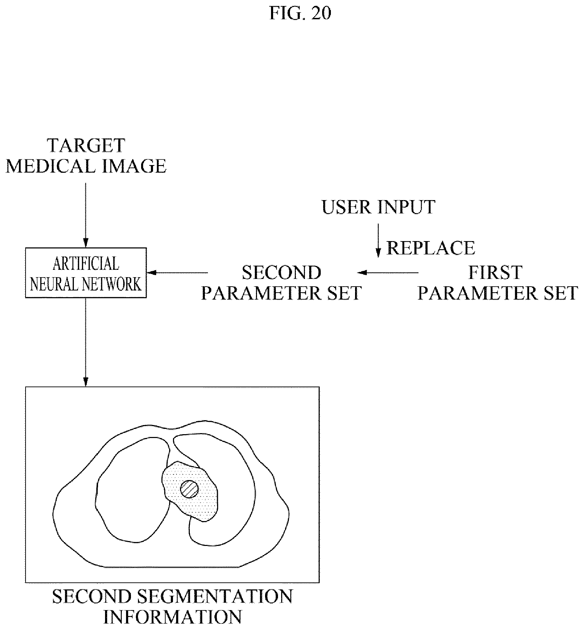

FIG. 20 is a schematic diagram illustrating segmenting a target medical image according to an embodiment of the present application.

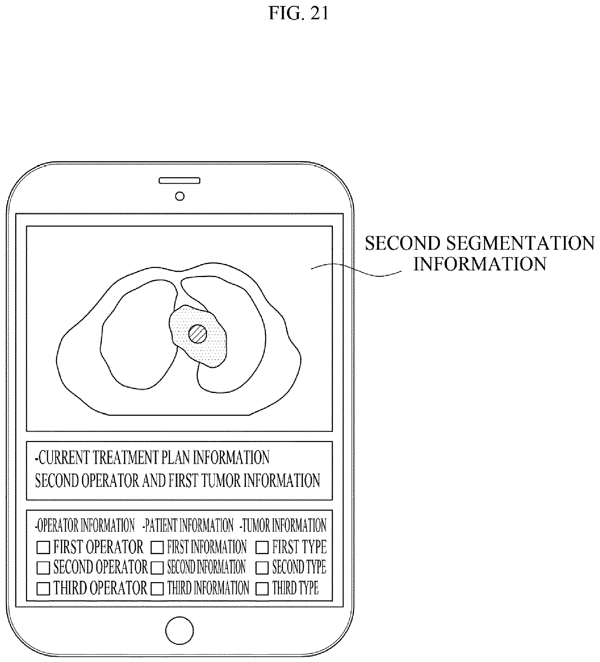

FIG. 21 illustrates an example of a user interface related to a result of analyzing a target medical image analysis according to an embodiment of the present application.

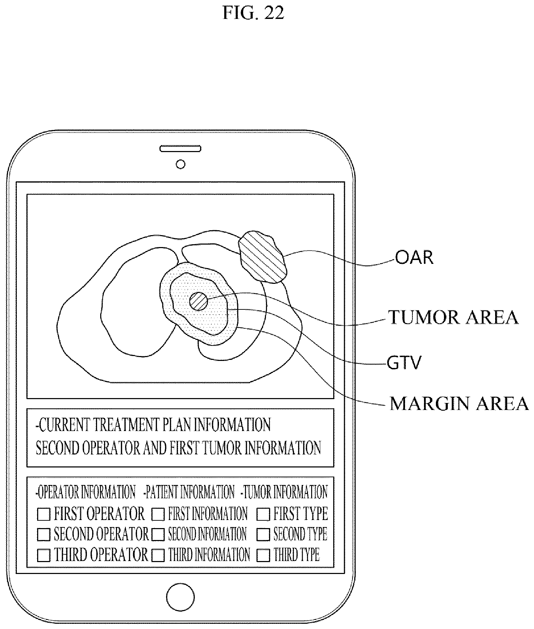

FIGS. 22 to 24 illustrate examples of a user interface related to a result of analyzing a target medical image according to an embodiment of the present application.

The medical image analysis device 2000 according to an embodiment of the present application may be implemented to output segmentation information that is a result of analyzing a medical image through the output module 2500. In this case, the medical image analysis device 2000 may also output checkboxes for setting target feature information through the output module 2500 together, and a user may select the target feature information through the input module 2400.

In this case, the medical image analysis device 2000 according to an embodiment of the present application may be implemented to output corresponding segmentation information of a target medical image through the output module 2500 on the basis of the target feature information obtained through the input module 2400.

For example, FIG. 19 is referred to. FIG. 19 illustrates outputting, through the output module 2500, a result of segmenting a target medical image on the basis of a neural network model including a parameter set related to target feature information including first operator information and second operator information. In other words, the first segmentation information, which is the result of segmenting the target medical image, may include areas (e.g., a tumor area, an area to which radiation is to be emitted, an area to which radiation should not be emitted, etc.) of the target medical image divided on the basis of a neural network model including a parameter set related to the first operator information and the second operator information.

In this case, the medical image analysis device 2000 may provide a user with treatment assistance information related to an area to which radiation is to be emitted and/or an area to which radiation should not be emitted to treat a tumor when target feature information corresponding to the first operator information and the second operator information is obtained on the basis of first segmentation information.

In this case, the medical image analysis device 2000 according to an embodiment of the present application may be implemented to output current feature information (or current treatment plan information) and information O1 related thereto together. In addition, the medical image analysis device 2000 may be implemented to further output treatment plan information 02 configured to allow a user to set feature information.

For example, referring to FIG. 19, a user may input a user input instructing to select certain information from among operator information, patient information, tumor information and/or radiation information (not shown in FIG. 19) included in the treatment plan information 02 through the input module 2400.

In this case, the medical image analysis device 2000 may be implemented to output segmentation information corresponding to the user input.

For example, referring to FIG. 20, the medical image analysis device 2000 may be implemented to obtain a user input for setting target feature information (e.g., a second operator and first tumor) and perform replacement with a parameter set corresponding to selected target feature information (e.g., replacement of a first parameter set with a second parameter set) on the basis of the obtained user input, as described above with reference to FIG. 19. In other words, the medical image analysis device 2000 may be implemented to select a parameter set corresponding to the target characteristic information on the basis of the obtained user input and replace a parameter set of an existing neural network model with the selected parameter set.

Accordingly, the medical image analysis device 2000 according to an embodiment of the present application may segment the target medical image on the basis of the selected target feature information and convert the first segmentation information into second segmentation information and output the second segmentation information through the output module 2500.

In this case, a boundary of an area to which radiation is to be emitted, which is included in the second segmentation information, and a boundary of an area to which radiation is to be emitted, which is included in the first segmentation information, may be different from each other when a difference in the target feature information is reflected.

FIG. 21 will now be referred to. FIG. 21 illustrates outputting through the output module 2500 a result of segmenting a target medical image on the basis of a neural network model including a parameter set related to target feature information including second operator information and first tumor information.

In other words, the second segmentation information, which is the result of segmenting the target medical image, may include areas (e.g., a tumor area, an area to which radiation is to be emitted, an area to which radiation should not be emitted, etc.) of the target medical image divided on the basis of a neural network model including a parameter set related to the second operator information and the first tumor information.

In this case, a boundary of an area to which radiation is to be emitted, which is included in the second segmentation information, may be different from the boundary of the area to which radiation is to be emitted, which is included in the first segmentation information of FIG. 19.

For example, the first segmentation information of FIG. 19 may be obtained on the basis of a neural network model including a node having a first parameter set learned based on operator information, which is related to a first operator having treatment history information defining an area to which radiation is to be emitted to be wider than an average treatment range, and patient information, which is learned to define an area to which radiation is to be emitted to be wider than an average treatment range. On the other hand, the second segmentation information of FIG. 21 may be obtained on the basis of a neural network model including a node having a second parameter set learned based on operator information, which is related to a second operator having treatment history information defining an area to which radiation is to be emitted to be narrower than an average treatment range, and tumor information, which is learned to define an area to which radiation is to be emitted to be narrower than an average treatment range.

Therefore, the boundary of the area to which radiation is to be emitted (e.g., the areas GTV, CTV, and PTV), which is included in the second segmentation information, may be different from the boundary of the area to which radiation is to be emitted (e.g., the areas GTV, CTV, and PTV), which is included in the first segmentation information of FIG. 19, and an area thereof may be smaller than an area of the boundary of the area to which radiation is to be emitted (e.g., the areas GTV, CTV, and PTV), which is included in the first segmentation information of FIG. 19.

As described above, the medical image analysis device 2000 may provide a user with treatment assistance information related to an area to which radiation is to be emitted to treat a tumor when target feature information corresponding to the second operator information and the first tumor information is obtained on the basis of the second segmentation information.

On the other hand, the first segmentation information shown in FIG. 19 and the second segmentation information shown in FIG. 21 may include information corresponding to the tumor area. In this case, similarly, a boundary of the tumor area included in the first segmentation information and a boundary of the tumor area included in the second segmentation information may be different from each other according to the target feature information.

However, according to an embodiment, the boundary of the tumor area included in the first segmentation information and the boundary of the tumor area included in the second segmentation information may be substantially the same.

For example, the tumor area included in the first segmentation information and the tumor area included in the second segmentation information may be obtained on the basis of a neural network model trained according to the target feature information (e.g., operator information, patient information, tumor information and/or radiation information) but may be obtained in substantially the same manner regardless of the parameter set related to the target feature information.

In addition, the medical image analysis device 2000 according to an embodiment of the present application may obtain treatment assistance information reflecting a tendency of defining an area to which radiation is to be emitted (or an area to which radiation should not be emitted) according to various operator information.

For example, the first segmentation information of FIG. 19 may be information reflecting a first operator's tendency of defining an area to which radiation is to be emitted with respect to a medical image related to a tumor. On the other hand, the second segmentation information of FIG. 21 may be information reflecting a second operator's tendency of defining an area to which radiation is to be emitted with respect to a medical image related to a tumor.

The medical image analysis device 2000 according to an embodiment may output segmentation information according to the operator information and provide a user with information about an operator's area definition tendency. Thus, the user may be provided with other operators' treatment assistance information related to the tumor. Therefore, the user may establish a treatment plan for the tumor on the basis of the other operators' treatment assistance information and thus obtain optimum treatment assistance information according to a situation to establish an efficient tumor treatment plan.

However, the above descriptions with reference to FIGS. 19 to 21 are only examples for convenience of description, and the medical image analysis device 2000 may be implemented to obtain a user input regarding the target feature information using an appropriate user interface and output segmentation information corresponding to the user input.

In addition, the medical image analysis device 2000 may be implemented to provide a user with segmentation information by obtaining a parameter set using a combination of the operator information, the patient information, the tumor information and/or the radiation information and replace a parameter set of an existing neural network model with the obtained parameter set.

Although not shown in FIGS. 19 to 21, segmentation information may include an area to which radiation should not be emitted (e.g., the area OAR), and the area to which radiation should not be emitted (e.g., the area OAR) may be obtained differently on the basis of a user input related to the target feature information. In other words, a boundary of the area to which radiation should not be emitted may vary according to the target feature information. This will be described in detail with reference to FIG. 22 below.

FIG. 22 will now be referred to. FIG. 22 illustrates an example of a user interface related to a result of analyzing a target medical image analysis according to an embodiment of the present application.

The medical image analysis device 2000 according to an embodiment of the present application may be implemented to output segmentation information through the output module 2500. In this case, the segmentation information may include information related to a tumor area, areas to which radiation is to be emitted and/or an area OAR to which radiation should not be emitted.

For example, the tumor area may be an area in a medical image corresponding to a tumor, and the areas to which radiation is to be emitted may include an area GTV corresponding to an organ in which the tumor is located, and a margin area (an area CTV related to a margin when movement of a patient during a radiation treatment is considered, an area PTV related to a margin when fine motion of the organ when a tumor treatment is performed a plurality of times is considered, and the like).

In this case, the areas to which radiation is to be emitted (e.g., the area GTV, the margin area (e.g., the areas CTV and PTV) and/or the area OAR to which radiation should not be emitted) output by the medical image analysis device 2000 may be output differently according to the target feature information.

For example, the medical image analysis device 2000 may segment the target medical image to obtain the areas to which radiation is to be emitted and/or the area to which radiation should not be emitted. In this case, the medical image analysis device 2000 may obtain a neural network model including a parameter set of renewed parameters on the basis of the target feature information, and the target medical image may be segmented on the basis of the neural network model to obtain and output the areas to which radiation is to be emitted (e.g., the tumor area, the area GTV, the margin area (e.g., the areas CTV and PTV)), and the area OAR to which radiation should not be emitted. In this case, the areas to which radiation is to be emitted (e.g., the tumor area, the area GTV, the margin area (e.g., the areas CTV and PTV)) and the area OAR to which radiation should not be emitted may be obtained and output differently according to the target feature information.

For example, when the medical image analysis device 2000 obtains target feature information related to defining areas to which radiation is to be emitted to be wider than an average treatment range, the medical image analysis device 2000 may obtain the areas, to which radiation is to be emitted (e.g., the tumor area, the area GTV, and the margin area (e.g., the areas CTV and PTV)) and which have a first boundary, from a neural network model including a node having a parameter set selected on the basis of the target feature information. On the other hand, when the medical image analysis device 2000 obtains target feature information related to defining areas to which radiation is to be emitted to be narrower than an average treatment range, the medical image analysis device 2000 may obtain the areas, to which radiation is to be emitted (e.g., the tumor area, the area GTV, and the margin area (e.g., the areas CTV and PTV)) and which have a second boundary, from a neural network model including a node having a parameter set selected on the basis of the target feature information.

In this case, the first boundary and the second boundary may be different from each other, and preferably, an area defined by the first boundary may be larger than an area defined by the second boundary. However, as described above, the first boundary and the second boundary may be substantially the same in relation to the tumor area among the areas to which radiation is to be emitted.

As another example, when the medical image analysis device 2000 obtains target feature information (e.g., operator information including treatment history information indicating an aggressive tumor treatment, tumor information indicating that tumor has a large size or has a high degree of expression, and the like) related to defining the area OAR to which radiation should not be emitted to be narrower than the average treatment range, the medical image analysis device 2000 may obtain an area OAR, to which radiation should not be emitted and which has a third boundary, from a neural network model including a node having a parameter set selected on the basis of the target feature information. On the other hand, when the medical image analysis device 2000 obtains target feature information (e.g., operator information including treatment history information indicating a conservative tumor treatment, tumor information indicating that a tumor has a small size or has a low degree of expression, patient information indicating that a patient is younger or has an underlying disease, and the like) related to defining the area OAR, to which radiation should not be emitted, to be wider than the average treatment range, the medical image analysis device 2000 may obtain an area OAR, to which radiation should not be emitted and which has a fourth boundary, from a neural network model including a node having a parameter set selected on the basis of the target feature information.

In this case, the third boundary and the fourth boundary may be different from each other, and preferably, an area defined by the third boundary may be smaller an area defined by the second boundary.

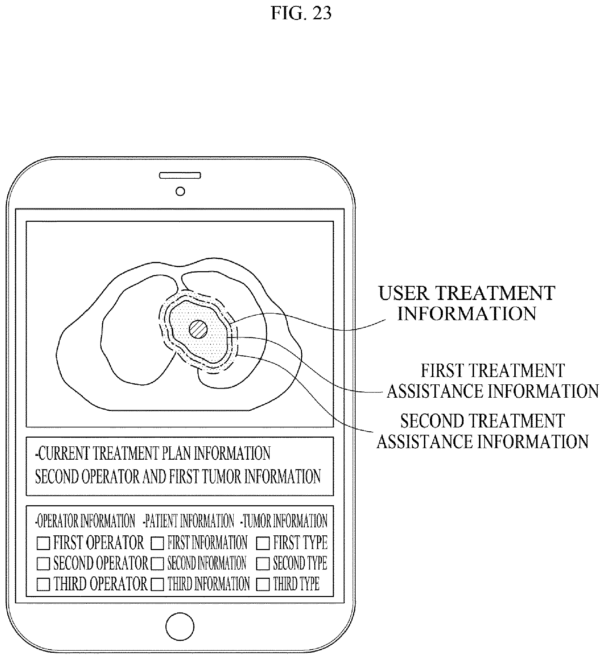

FIG. 23 will now be referred to. FIG. 23 illustrates an example of a user interface related to a result of analyzing a target medical image analysis according to an embodiment of the present application.

The medical image analysis device 2000 according to an embodiment of the present application may be implemented to output user treatment information and at least one piece of treatment assistance information.

Here, the user treatment information may be understood to mean information related to areas to which radiation is to be emitted (e.g., the areas GTV, CTV, and PTV and the like) and/or an area to which radiation should not be emitted (e.g., the area OAR), which is defined by a user in relation to a target medical image. The medical image analysis device 2000 may obtain the user treatment information by receiving a user input defining a boundary of an area corresponding to the target medical image through the input module 2400.

The at least one piece of treatment assistance information may include first treatment assistance information obtained by reflecting target feature information and second treatment assistance information obtained without reflecting target feature information.

For example, the first treatment assistance information may be information output by a neural network model including a node having a parameter set obtained on the basis of second operator information and first tumor information. The first treatment assistance information is obtained by a neural network model trained to reflect an operator's area definition tendency related to the second operator information and an operator's area definition tendency related to the second operator information with respect to the first tumor information and thus may include information related to areas to which radiation is to be emitted and an area to which radiation should not be emitted, which are dependent on the second operator information and the first tumor information.

For example, the second treatment assistance information may be obtained by segmenting the target medical image without reflecting the target feature information, i.e., without considering the second operator and the first tumor information of FIG. 23. For example, referring to FIGS. 8 and 9, the medical image analysis device 2000 according to an embodiment of the present application may obtain the second treatment assistance information by segmenting the target medical image on the basis of a neural network model trained without considering feature information. Similar to the first treatment assistance information, the second treatment assistance information may include information related to areas to which radiation is to be emitted and/or an area to which radiation should not be emitted. However, the second treatment assistance information may be obtained by a neural network model trained without considering the target feature information (e.g., the second procedure information and first tumor information). For example, the second treatment auxiliary information may be obtained by a neural network model including a node (e.g., at least one node included in a feature layer) having a parameter set related to the target feature information. Therefore, the information related to the areas to which radiation is to be emitted and/or the area to which radiation should not be emitted, which are included in the second treatment assistance information, may be different from the information related to the areas to which radiation is to be emitted and/or the area to which radiation should not be emitted, which are included in the first treatment assistance information.

In this case, the medical image analysis device 2000 according to an embodiment of the present application may overlay the user treatment information on the target medical image together with the at least one piece of treatment assistance information and display the user treatment information to a user through the output module 2500. Thus, with the medical image analysis device 2000 according to an embodiment of the present application, a user may compare his or her own treatment information with at least one piece of treatment assistance information output through the neural network model to check his or her own treatment tendency.

In addition, the user may make an optimum treatment plan by effectively modifying at least part of a boundary included in his or her treatment information on the basis of the at least one piece of treatment assistance information.

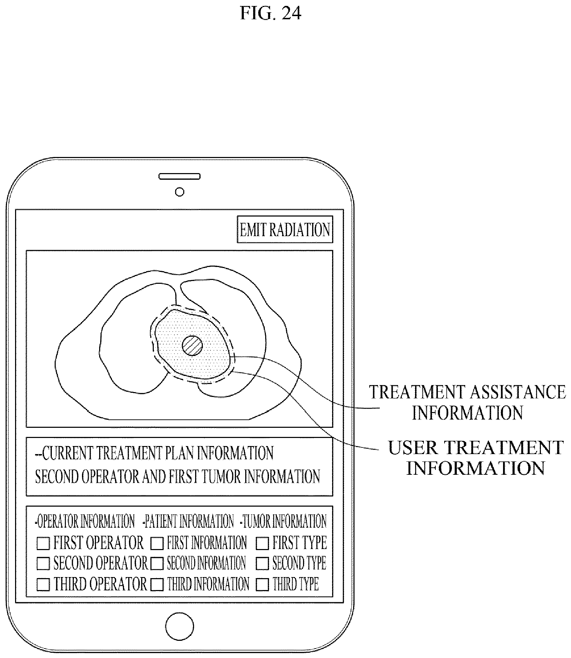

FIG. 24 will now be referred to. FIG. 24 illustrates an example of a user interface related to a result of analyzing a target medical image analysis according to an embodiment of the present application.

The medical image analysis device 2000 according to an embodiment of the present application may be implemented to output user treatment information and at least one piece of treatment assistance information as described above with reference to FIG. 23. That is, treatment assistance information illustrated in FIG. 24 may include the first treatment assistance information or the second treatment assistance information of FIG. 23.

The medical image analysis device 2000 according to an embodiment of the present application may be implemented to emit radiation on the basis of at least one of the user treatment information and the at least one piece of treatment assistance information.

For example, the medical image analysis device 2000 according to an embodiment of the present application may further include a radiation outputter or may be implemented to communicate with an external radiation emission device.

In this case, the medical image analysis device 2000 may be implemented to obtain an input instructing to emit radiation from a user through the input module 2400 and transmit an instruction to emit radiation to the radiation outputter or an external radiation emission device in response to the input from the user.

In this case, the radiation outputter or the external radiation emission device may emit radiation on the basis of at least one of the user treatment information and the at least one piece of treatment assistance information.

Meanwhile, the medical image analysis device 2000 according to an embodiment of the present application may be implemented to provide a notification window for requesting a user to provide an input for selecting at least one of the user treatment information and the at least one piece of treatment assistance information or a notification window for requesting a user to provide an input for modifying at least one of the user treatment information and the at least one piece of treatment assistance information through the output module 2500, in response to a user input instructing to emit radiation.

However, the above descriptions with reference to FIGS. 22 to 24 are only examples for convenience of description, and the medical image analysis device 2000 may use an appropriate user interface to output treatment assistance information (or segmentation information) and/or user treatment information and to receive a user input instructing to emit radiation.

The medical image analysis method, the medical image analysis device and the medical image analysis system set forth herein may be used to analyze a medical image.

In particular, the medical image analysis method, the medical image analysis device and the medical image analysis system set forth herein are applicable to a field of providing treatment assistance information to treat a tumor using radiation. For example, the medical image analysis method, the medical image analysis device and the medical image analysis system set forth herein are applicable to a field of providing treatment assistance information to obtain an area to which radiation is to be emitted on the basis of treatment plan information to treat a tumor.

However, the medical image analysis method, the medical image analysis device, and the medical image analysis system set forth herein are applicable to all fields using a medical image to perform a medical procedure or treatment using radiation, as well as tumor treatment.

The features, structures, effects and the like described above in the embodiments are included in at least one embodiment of the present disclosure and are not necessarily limited only to an embodiment. Furthermore, the features, structures, effects and the like provided as examples in the embodiments may be implemented in combination or in different forms in other embodiments by those of ordinary skill in the field to which embodiments pertain. Therefore, such a combination and different forms should be understood as being included in the scope of the present disclosure.

Although embodiments have been described above, these embodiments are merely examples and are not intended to restrict the present disclosure, and it will be apparent to those of ordinary skill in the art that various modifications and applications may be made without departing from essential features of embodiments. That is, modification may be made in each component specifically shown in the embodiments. In addition, differences related to such modifications and applications should be construed as being included within the scope of the present disclosure defined in the appended claims.

## Claims

1. A method for analyzing a medical image using a device which obtains the medical image and provides a treatment auxiliary information based on the medical image, the method comprising:
obtaining a target medical image;
obtaining a treatment plan information for determining a target area to be radiated, wherein the treatment plan information includes a first feature information or a second feature information;
selecting a target parameter set, based on the treatment plan information, among a first parameter set corresponding to the first feature information and a second parameter set corresponding to the second feature information;
determining parameter values of a feature node set including at least one of a plurality of nodes of an artificial neural network learned to obtain an area information related to the target area as the target parameter set, based on the target medical image; and
providing the treatment auxiliary information related to the target area corresponding to the treatment plan information, based on the artificial neural network to which the target parameter set is applied and the target medical image.

2. The method of claim 1, wherein the artificial neural network is configured to obtain a plurality of areas including the target area and a tumor area by performing a segmentation to the target medical image, based on one or more labels related to a radiation irradiation.

3. The method of claim 2, wherein one or more labels include a label related to at least one of an area corresponding to an organ in which a tumor is located, an area related to a margin considering a movement of a patient, an area related to a margin considering a movement of organ, an area that should not be irradiated with the radiation, and the tumor area, wherein the artificial neural network is learned to assign the one or more labels to a cell of the target medical image and to obtain an area information related to the target area, an area information related to the area that should not be irradiated with the radiation, and the area information related to the tumor area, wherein the treatment auxiliary information related to the target area is obtained based on the label assigned to the cell.

4. The method of claim 1,
wherein when the treatment plan information includes the first feature information, the target parameter set is determined as the first parameter set, and the providing the treatment auxiliary information comprises: obtaining a first target area information obtained based on area information obtained via the artificial neural network which is applied the first parameter set, and
wherein when the treatment plan information includes the second feature information, the target parameter set is determined as the second parameter set, and the providing the treatment auxiliary information comprises: obtaining a second target area information obtained based on area information obtained via the artificial neural network which is applied the second parameter set, and
wherein the second target area information is different from the first target area information.

5. The method of claim 4, wherein the first target area information is defined by a first boundary and the second target area information is defined by a second boundary,
wherein at least one boundary of the first boundary and the second boundary on the target medical image includes another boundary of the first boundary and the second boundary.

6. The method of claim 3, wherein the target parameter set is used to obtain the area information related to the target area,
wherein when the treatment plan information includes the first feature information, the target parameter set is determined as the first parameter set, and the providing the treatment auxiliary information comprises: obtaining a third target area information obtained based on a tumor area information obtained via the artificial neural network which is applied the first parameter set, and
wherein when the treatment plan information includes the second feature information, the target parameter set is determined as the second parameter set, and the providing the treatment auxiliary information comprises: obtaining a fourth target area information obtained based on the tumor area information obtained via the artificial neural network which is applied the second parameter set, and
wherein the third target area information is substantially the same as the fourth target area information.

7. The method of claim 1,
the obtaining the treatment plan information comprising: obtaining a user input selecting at least one of the first feature information or the second feature information, via an input module,
the selecting the target parameter set comprising: selecting a parameter set corresponding to the user input among the first parameter set corresponding to the first feature information and the second parameter set corresponding to the second feature information as the target parameter set.

8. The method of claim 1, wherein the treatment plan information is related to at least one of an operator information, a patient information, a tumor information and a radiation information,
wherein the operator information includes at least one of an identity information and a treatment history information related to the operator who treats a tumor,
wherein the tumor information includes at least one of information related to a size, type, location and expression lever of the tumor to be treated,
wherein the radiation information includes at least one of information related to a type, an intensity, an irradiation period, and a risk of the radiation.

9. The method of claim 1, the method further comprising:
obtaining an user input, via an input module, related to an user treatment information defining a plurality of areas including a tumor area information related to a tumor area and a target area information related to a target area to the target medical image; and
outputting the user treatment information and the treatment auxiliary information via output module.

10. The method of claim 1, the method further comprising:
providing a second treatment auxiliary information which is related to the target area, obtained based on the target medical image and the artificial neural network which does not include the target parameter set,
wherein the second treatment auxiliary information is obtained by the artificial neural network independent of the first feature information or the second feature information.

11. The method of claim 1, the method further comprising:
obtaining an user input, via an input module, which selects at least one of a first target area information and a second target area information and instructs to initiate an irradiation of radiation based on the selected target area information; and
instructing an initiation of the irradiation of radiation in response to the user input;
wherein the first target area information is an information related to the target area obtained based on area information obtained via the artificial neural network applied the first parameter set, and
wherein the second target area information is an information related to the target area obtained based on area information obtained via the artificial neural network applied the second parameter set.

12. A method for analyzing a medical image using a device which obtains the medical image and provides a treatment auxiliary information based on the medical image, the method comprising:
obtaining a target medical image;
obtaining a treatment plan information including a first feature information and second feature information related to parameters which are a basis for determining a target area to be irradiated;
obtaining a first area related to a target tumor and a second area adjacent to the first area and related to the target area, by performing a segmentation the target medical image into a plurality of areas bas based on the treatment plan information, using an artificial neural network including a node set having a target parameter set determined based on the treatment plan information;
determining a boundary of the second area based on the target parameter set of the node set, wherein when the treatment plan information includes the first feature information, the second area has a first boundary, and when the treatment plan information includes the second feature information, the second area has a second boundary different from the first boundary; and
providing the determined boundary of the second area and a boundary of the first area on the medical image.

13. The method of claim 12, the method further comprising:
determining the target parameter set based on the treatment plan information, and wherein the target parameter set is determined by selecting at least one among a first parameter set corresponding to the first feature information and a second parameter set corresponding to the second feature information;

14. The method of claim 13,
wherein when the treatment plan information includes the first feature information, the second area having the first boundary is determined based on the first parameter set, and when the treatment plan information includes the second feature information, the second area having the second boundary is determined based on the second parameter set.

15. The method of claim 13,
wherein when the treatment plan information includes the first feature information, the first area has a third boundary, and when the treatment plan information includes the second feature information, the first area has a fourth boundary,
wherein the third boundary and the fourth boundary are substantially the same.

16. The method of claim 13,
the obtaining the treatment plan information comprising: obtaining a, user input selecting at least one of the first feature information or the second feature information, via an input module,
the selecting the target parameter set comprising: based on the user input, selecting a parameter set corresponding to the user input among the first parameter set corresponding to the first feature information and the second parameter set corresponding to the second feature information as the target parameter set.

17. The method of claim 12, wherein the treatment plan information is related to at least one of an operator information, a patient information, a tumor information and a radiation information,
wherein the operator information includes at least one of an identity information and a treatment history information related to the operator who treats a tumor,
wherein the tumor information includes at least one of information related to a size, type, and expression lever of the tumor to be treated,
wherein the radiation information includes at least one of information related to a type, an intensity, a shape, and a risk of the radiation.

18. The method of claim 12, the method further comprising:
obtaining an user input, via an input module, related to an user treatment information defining a plurality of areas including a third area related to the tumor area and a fourth area related to the target area to the target medical image; and
outputting the target medical image on which a boundary of the third area and a boundary of the fourth area are displayed.

19. The method of claim 12, the method further comprising:
providing an auxiliary information which is related to the target area, obtained based on the target medical image and the artificial neural network which does not include the target parameter set,
wherein the auxiliary information is obtained by the artificial neural network independent of the first feature information or the second feature information.

20. The method of claim 12, the method further comprising:
obtaining an user input, via an input module, which instructs to initiate an irradiation of radiation based on the second area; and
instructing an initiation of the irradiation of radiation for the second area in response to the user input.

21. The method of claim 12, wherein the plurality of areas is related to at least one of an area corresponding to an organ in which a tumor is located, an area related to a margin considering a movement of a patient, an area related to a margin considering a movement of organ, an area that should not be irradiated with the radiation, and the tumor area.

22. The method of claim 21, wherein the artificial neural network is configured to obtain the plurality of areas by segmentation for the target medical image based on the one or more labels related to an irradiation.

23. The method of claim 22, wherein the one or more labels include a label related to at least one of an area corresponding to an organ in which a tumor is located, an area related to a margin considering a movement of a patient, an area related to a margin considering a movement of organ, an area that should not be irradiated with the radiation, and the tumor area, wherein the artificial neural network is learned to assign the one or more labels to a cell of the target medical image and to obtain an area information related to the target area, an area information related to the area that should not be irradiated with the radiation, and the area information related to the tumor area, wherein the plurality of areas are obtained based on the label assigned to the cell.

24. A device for analyzing a medical image and providing a treatment auxiliary information related to a tumor, the device comprising:
an image acquisition unit for obtaining a target medical image; and
a controller for providing a treatment auxiliary information based on the target medical image, and wherein the controller configured to:
obtain a target medical image;
obtain a treatment plan information for determining a target area to be radiated, wherein the treatment plan information includes a first feature information or a second feature information;
select a target parameter set, based on the treatment plan information, among a first parameter set corresponding to the first feature information and a second parameter set corresponding to the second feature information;
determine parameter values of a feature node set including at least one of a plurality of nodes of an artificial neural network learned to obtain an area information related to the target area as the target parameter set, based on the target medical image; and
provide the treatment auxiliary information related to the target area corresponding to the treatment plan information, based on the artificial neural network to which the target parameter set is applied and the target medical image.

25. The device of claim 24, wherein the artificial neural network is configured to obtain a plurality of areas including the target area and a tumor area by performing a segmentation to the target medical image, based on one or more labels related to a radiation irradiation.

26. The device of claim 25, wherein one or more labels include a label related to at least one of an area corresponding to an organ in which a tumor is located, an area related to a margin considering a movement of a patient, an area related to a margin considering a movement of organ, an area that should not be irradiated with the radiation, and the tumor area, wherein the artificial neural network is learned to assign the one or more labels to a cell of the target medical image and to obtain an area information related to the target area, an area information related to the area that should not be irradiated with the radiation, and the area information related to the tumor area, wherein the treatment auxiliary information related to the target area is obtained based on the label assigned to the cell.

27. The device of claim 24, the controller is configured to:
determine the target parameter set as the first parameter set when the treatment plan information includes the first feature information, and obtain a first target area information obtained based on area information obtained via the artificial neural network which is applied the first parameter set,
determine the target parameter set as the second parameter set when the treatment plan information includes the second feature information, and obtain a second target area information obtained based on area information obtained via the artificial neural network which is applied the second parameter set, wherein the second target area information is different from the first target area information.

28. The device of claim 27, wherein the first target area information is defined by a first boundary and the second target area information is defined by a second boundary,
wherein at least one boundary of the first boundary and the second boundary on the target medical image includes another boundary of the first boundary and the second boundary.

29. The device of claim 24, wherein the target parameter set is used to obtain the area information related to the target area, and
the controller is configured to:
determine the target parameter set as the first parameter set when the treatment plan information includes the first feature information, and provide the treatment auxiliary information by providing a third target area information obtained based on a tumor area information obtained via the artificial neural network which is applied the first parameter set,
determine the target parameter set as the second parameter set when the treatment plan information includes the second feature information, and provide the treatment auxiliary information by providing a fourth target area information obtained based on the tumor area information obtained via the artificial neural network which is applied the second parameter set, and
wherein the third target area information is substantially the same as the fourth target area information.

30. The device of claim 24, the device further comprising:
an input module for receiving an user input related to the treatment plan information; and
an output module for outputting the treatment auxiliary information in response to the user input;
the controller is configured to:
obtain a user input selecting at least one of the first feature information or the second feature information, via an input module; and
select a parameter set corresponding to the user input among the first parameter set corresponding to the first feature information and the second parameter set corresponding to the second feature information as the target parameter set.

31. The device of claim 24, wherein the treatment plan information is related to at least one of an operator information, a patient information, a tumor information and a radiation information,
wherein the operator information includes at least one of an identity information and a treatment history information related to the operator who treats a tumor,
wherein the tumor information includes at least one of information related to a size, type, location and expression lever of the tumor to be treated,
wherein the radiation information includes at least one of information related to a type, an intensity, an irradiation period, and a risk of the radiation.

32. The device of claim 24, the device further comprising:
an input module for receiving an user input defining a plurality of areas; and
an output module for outputting an user treatment information in response to the user input;
the controller is configured to:
obtain the user input, via an input module, related to the user treatment information defining the plurality of areas including a tumor area information related to a tumor area and a target area information related to a target area to the target medical image; and
output the user treatment information and the treatment auxiliary information via output module.

33. The device of claim 24, the controller is configured to:
provide a second treatment auxiliary information which is related to the target area, obtained based on the target medical image and the artificial neural network which does not include the target parameter set,
wherein the second treatment auxiliary information is obtained by the artificial neural network independent of the first feature information or the second feature information.

34. The device of claim 22, the device further comprising:
an input module for receiving an user input instructing an initiation of irradiation;
wherein the controller is configured to:
obtain an user input, via an input module, which selects at least one of a first target area information and a second target area information and instructs to initiate an irradiation of radiation based on the selected target area information; and
instruct an initiation of the irradiation of radiation in response to the user input;
wherein the first target area information is an information related to the target area obtained based on area information obtained via the artificial neural network applied the first parameter set, and
wherein the second target area information is an information related to the target area obtained based on area information obtained via the artificial neural network applied the second parameter set.

35. A device for analyzing a medical image and providing a treatment auxiliary information related to a tumor, the device comprising:
an image acquisition unit for obtaining a target medical image; and
a controller for providing a treatment auxiliary information based on the target medical image, and wherein the controller configured to:
obtain a target medical image;
obtain a treatment plan information including a first feature information and second feature information related to parameters which are a basis for determining a target area to be irradiated;
obtain a first area related to a target tumor and a second area adjacent to the first area and related to the target area, by performing a segmentation the target medical image into a plurality of areas bas based on the treatment plan information, using an artificial neural network including a node set having a target parameter set determined based on the treatment plan information;
determine a boundary of the second area based on the target parameter set of the node set, wherein when the treatment plan information includes the first feature information, the second area has a first boundary, and when the treatment plan information includes the second feature information, the second area has a second boundary different from the first boundary; and
provide the determined boundary of the second area and a boundary of the first area on the medical image.

36. The device of claim 35, the controller is configured to:
determine the target parameter set based on the treatment plan information, and
determine the target parameter set by selecting at least one among a first parameter set corresponding to the first feature information and a second parameter set corresponding to the second feature information.

37. The device of claim 36, the controller is configured to:
determine the second area having the first boundary based on the first parameter set when the treatment plan information includes the first feature information; and
determine the second area having the second boundary based on the second parameter set when the treatment plan information includes the second feature information.

38. The device of claim 35,
wherein when the treatment plan information includes the first feature information, the first area has a third boundary, and when the treatment plan information includes the second feature information, the first area has a fourth boundary,
wherein the third boundary and the fourth boundary are substantially the same.

39. The device of claim 36, the device further comprising:
an input module for receiving an user input related to the treatment plan information; and wherein the controller is configured to:
obtain the user input selecting at least one of the first feature information or the second feature information, via the input module; and
based on the user input, select a parameter set corresponding to the user input among the first parameter set corresponding to the first feature information and the second parameter set corresponding to the second feature information as the target parameter set.

40. The device of claim 35, wherein the treatment plan information is related to at least one of an operator information, a patient information, a tumor information and a radiation information,
wherein the operator information includes at least one of an identity information and a treatment history information related to the operator who treats a tumor,
wherein the tumor information includes at least one of information related to a size, type, and expression lever of the tumor to be treated,
wherein the radiation information includes at least one of information related to a type, an intensity, a shape, and a risk of the radiation.

41. The device of claim 35, the device further comprising:
an input module for receiving an user input related to the treatment plan information; and
an output module for outputting a treatment auxiliary information in response to the user input;
wherein the controller is configured to:
obtain the user input, via the input module, related to an user treatment information defining a plurality of areas including a third area related to the tumor area and a fourth area related to the target area to the target medical image; and
output the target medical image on which a boundary of the third area and a boundary of the fourth area are displayed.

42. The device of claim 35, wherein the controller is configured to:
provide an auxiliary information which is related to the target area, obtained based on the target medical image and the artificial neural network which does not include the target parameter set,
wherein the auxiliary information is obtained by the artificial neural network independent of the first feature information or the second feature information.

43. The device of claim 35, the device further comprising:
an input module for receiving an user input instructing an initiation of irradiation;
wherein the controller is configured to:
obtain the user input, via the input module, which instructs to initiate the irradiation based on the second area; and
instruct the initiation of the irradiation for the second area in response to the user input.

44. The device of claim 35, wherein the plurality of areas is related to at least one of an area corresponding to an organ in which a tumor is located, an area related to a margin considering a movement of a patient, an area related to a margin considering a movement of organ, an area that should not be irradiated with the radiation, and the tumor area.

45. The device of claim 44, wherein the artificial neural network is configured to obtain the plurality of areas by segmentation for the target medical image based on the one or more labels related to an irradiation.

46. The device of claim 45, wherein the one or more labels include a label related to at least one of an area corresponding to an organ in which a tumor is located, an area related to a margin considering a movement of a patient, an area related to a margin considering a movement of organ, an area that should not be irradiated with the radiation, and the tumor area, wherein the artificial neural network is learned to assign the one or more labels to a cell of the target medical image and to obtain an area information related to the target area, an area information related to the area that should not be irradiated with the radiation, and the area information related to the tumor area, wherein the plurality of areas are obtained based on the label assigned to the cell.
